# EUROPEAN PATENT APPLICATION

(11) **EP 3 000 483 A1**
(43) Date of publication of application: **30.03.2016**
(21) Application number: 14003356.4
(22) Date of filing: 29.09.2014
(51) Int. Cl.: A61K 51/12, A01K 67/027, C12N 5/0789

(54) **Means and methods for the diagnosis and treatment of neurodegenerative diseases**

(71) Applicant: Charité - Universitätsmedizin Berlin, 10117 Berlin (DE)
(72) Inventor: PRILLER, Josef, 10435 Berlin (DE); BÖTTCHER, Chotima, 10115 Berlin (DE)
(74) Representative: Bohmann, Armin K.

(57) **Abstract**

The present invention is related to a bone marrow-derived myeloid progenitor cell (MPC), preferably an isolated bone marrow-derived myeloid progenitor cell (MPC), wherein the bone marrow-derived myeloid progenitor cell (MPC) is expressing CD34 and c-Kit (CD117).

## Description

The present invention is related to a bone marrow-derived myeloid progenitor cell (MPC); the bone marrow-derived myeloid progenitor cell (MPC) for use in a method for treating a subject suffering from a disease or being at risk of suffering from a disease; the bone marrow-derived myeloid progenitor cell (MPC) for use in a method for treating a subject suffering from a disease or being at risk of suffering from a disease, wherein the bone marrow-derived myeloid progenitor cell (MPC) is a recombinant bone marrow-derived myeloid progenitor cell (MPC) delivering a therapeutically active agent into the subject; the bone marrow-derived myeloid progenitor cell (MPC) for use in a method for diagnosing a disease in a subject; a pharmaceutical composition comprising the bone marrow-derived myeloid progenitor cell (MPC); use of the bone marrow-derived myeloid progenitor cell (MPC) for the manufacture of a medicament; use of the bone marrow-derived myeloid progenitor cell (MPC) for the manufacture of a diagnostic agent; a method for the treatment of a disease, wherein the method comprises administering to a subject the bone marrow-derived myeloid progenitor cell (MPC); and a method for the diagnosis of a disease, wherein the method comprises administering to a subject the bone marrow-derived myeloid progenitor cell (MPC).

Neurodegenerative diseases are on the rise affecting the life of an increasing number of people. The greatest risk factor for neurodegenerative diseases is aging. Many neurodegenerative diseases are late-onset, meaning there are some factors that change as a person ages for each disease. One constant factor is that in each disease, neurons gradually lose function as the disease progresses with age. The process of neurodegeneration is not well understood so the diseases that stem from it have, as yet, no cures. However, clinical trials are performed pursuing a plurality of approaches in the treatment of neurodegenerative diseases.

The problem underlying the present invention is thus the provision of means and methods for the treatment of neuronal diseases and neurodegenerative diseases in particular. A further problem underlying the present invention is the provision of means and methods for the diagnosis of neuronal diseases and neurodegenerative diseases in particular.

These and other problems underlying the present invention are solved by the subject matter of the attached independent claims. Preferred embodiments may be taken from the attached dependent claims.

The present invention is based on the surprising finding that bone marrow-derived myeloid progenitor cells (MPCs) have a therapeutic effect, more specifically a therapeutic effect in neurodegenerative disorders. Based on the findings and experimental evidence provided herein but without wishing to be bound by any theory, the present inventors conclude that bone marrow-derived myeloid progenitor cells (MPCs) give rise to myeloid cells that engraft in the central nervous system (CNS) after bone marrow/hematopoietic stem cell (HSC) transplantation. In light thereof bone marrow-derived myeloid progenitor cells (MPCs) are a suitable means for cell-based gene therapy in the central nervous system (CNS). Furthermore, bone marrow-derived myeloid progenitor cells (MPCs) are superior to hematopoietic stem cells (HSC) as they have a longer lifespan in blood and are present in higher quantity in the bone marrow.

Murine bone marrow-derived myeloid progenitor cells (MPCs) have been described in the prior art, for example, in Auffray et al. (Auffray et al.; J Exp Med. 2009, 206:595-606). Protocols for the identification of bone marrow-derived myeloid progenitor cells (MPCs) are described in Waskow et al. (Waskow et al., Nat. Immunol. 2008, 9:676 - 683) and Liu et al. (Liu et al., Science. 2009, 324:392-397). The identification protocols as described in Waskow et al. and Liu et al. are preferably used in connection with the instant application, whereby the surface antigens and, respectively, surface markers as disclosed herein are used for the characterization of bone marrow-derived myeloid progenitor cells (MPCs) and human bone marrow-derived myeloid progenitor cells (MPCs) in particular. Such surface antigens and, respectively, surface markers are also collectively referred to herein as such surface antigens, and such surface antigen and, respectively, surface marker is also collectively referred to herein as such surface antigen.

The terms bone marrow-derived myeloid progenitor cells and MPCs are interchangeably used herein.

As preferably used herein, bone marrow-derived myeloid progenitor cells (MPCs) are bone marrow-derived myeloid progenitor cells.

In an embodiment the bone marrow-derived myeloid progenitor cells (MPCs) are the ones described in the example part of the instant application and/or the ones which can be identified using the methods disclosed herein, including and in particular the methods described in the example part of the instant application.

The surface antigens used herein for the characterization of a bone marrow-derived myeloid progenitor cell (MPC) and more specifically of a bone marrow-derived myeloid progenitor cell (MPC) are preferably known in the art. Such surface antigen is preferably detected by an antibody, preferably a monoclonal antibody. In other words, a bone marrow-derived myeloid progenitor cell (MPC) of the invention is detected and identified by an antibody binding to such surface antigen or by a plurality or multitude of antibodies binding to different such surface antigens.

In an embodiment the antibody binding to such surface antigen is one that is specifically binding to such surface antigen. An antibody specifically binding to such surface antigen is preferably one the binding of which is such that it does not cross-react with one or several other surface antigens existing on cells of the immune system, preferably of the immune system of a mammal and more preferably of the immune system of a human being. In a preferred embodiment the one or several other surface antigens existing on cells of the immune system are antigens that are used in the characterization of the various types and/or lineages of cells of the immune system.

Antibodies to such surface antigens are commercially available.

In an embodiment the antibody binding to such surface antigen and preferably used in the identification and/or detection of a bone marrow-derived myeloid progenitor cell (MPC) and a bone marrow-derived myeloid progenitor cell (MPC) of the invention in particular, comprises or bears a moiety which allows detection and/or identification of the antibody. Preferably, such detection and/or identification of the antibody allow detection and/or identification of a bone marrow-derived myeloid progenitor cell (MPC) and, in particular, a bone marrow-derived myeloid progenitor cell (MPC) of the invention.

In an embodiment, the moiety that allows detection and/or identification of the antibody, is or comprises a label. Preferably such label allows cell sorting and more preferably fluorescence-activated cell sorting. In a preferred embodiment the moiety that allows detection and/or identification of the antibody is a fluorescent label.

In an embodiment where a bone marrow-derived myeloid progenitor cell (MPC) and, in particular, a bone marrow-derived myeloid progenitor cell (MPC) of the invention is identified by a plurality or multitude of antibodies binding to different such surface antigens, it is preferred that the antibodies of the plurality of antibodies differ from each other as to their moieties that allow detection and/or identification of the antibody. Preferably the antibodies of the plurality of antibodies differ from each other as to their label, which is preferably a fluorescent label.

A bone marrow-derived myeloid progenitor cell (MPC) and, in particular, a bone marrow-derived myeloid progenitor cell (MPC) of the invention is preferably identified and/or detected using an antibody binding to such surface antigen, or by a plurality or multitude of antibodies binding to different such surface antigens together with flow cytometry. In a preferred embodiment flow cytometry is fluorescence-activated cell sorting which is also referred to as FACS. In an alternative embodiment flow cytometry is magnetic-activated cell sorting which is also referred to as MACS. It is within the present invention that when flow cytometry is used in the identification and/or detection of a bone marrow-derived myeloid progenitor cell (MPC) and, in particular, a bone marrow-derived myeloid progenitor cell (MPC) of the invention that the antibody, either individually or as a member of the plurality or multitude of antibodies, bears or comprises a moiety that allows detection and/or identification of the MPC.

It is within the instant invention that a bone marrow-derived myeloid progenitor cell (MPC) and, in particular, a bone marrow-derived myeloid progenitor cell (MPC) of the invention is identified and/or detected by means of a compound which is specifically interaction with such surface antigen but which is different from an antibody. Such compound is preferably one selected from the group comprising an aptamer, a spiegelmer and an anticaline. Methods for the generation and use of such compounds are known in the art. It will be acknowledged by a person skilled in the art that such surface antigen is a target molecule for such compound.

Aptamers are D-nucleic acids that are either single stranded or double stranded and which specifically interact with a target molecule. The generation and selection of aptamers is, e. g., described in European patent EP 0 533 838. Spiegelmers are L- nucleic acids that are either single stranded or double stranded and which specifically interact with a target molecule. The generation and selection of Spiegelmers is, e. g., described in WO 98/08856. Anticalines are target-binding polylpeptides. Anticalines and methods for their generation and selection are, for example, described in German patent application DE 197 42 706.

It is within the present invention that such compounds which specifically interact with such surface antigen are used in the same way as described above for antibodies, and that such compounds which specifically interact with such surface antigen, are, as said antibodies, comprising or bearing a moiety which allows detection and/or identification of such compound.

According to the present invention, a bone marrow-derived myeloid progenitor cell (MPC) is expressing certain surface antigens. In an embodiment expressing a surface marker means that the surface marker is present in a cell or on a cell surface. Preferably, the surface marker is present on the cell. It will be acknowledged by a person skilled in the art that the degree of expression of a surface antigen may vary. The surface antigen may be expressed at a high level, a medium level or a low level. In a preferred embodiment of the instant invention the surface antigen is expressed at a high level. The degree of expression may be indicated in connection with such surface antigen. For example and for the purpose of illustration, CD34^{hi} indicates high expression of CD34, whereas CD34^{lo} indicates low expression of CD34. In an embodiment, the degree of expression can be determined in flow cytometry and FACS in particular, so that only those cells are identified, detected and/or sorted, i.e. removed from the remainder of the cells, which display a predefined expression of a given surface antigen.

In an embodiment, a bone marrow-derived myeloid progenitor cell (MPC) and, in particular, a bone marrow-derived myeloid progenitor cell (MPC) of the invention can also be characterized by certain surface antigens that are not been expressed by such cell. In a preferred embodiment such certain surface antigen is not expressed by such cell on the surface of such cell. The absence of such surface antigen can be detected by an antibody binding to such surface antigen and a compound binding to surface antigen, whereby the absence of such surface antigen is given in case such antibody and compound, respectively, is not binding to such cell.

A bone marrow-derived myeloid progenitor cell (MPC) of the invention is expressing CD34 and c-Kit (CD117).

In an embodiment, a bone marrow-derived myeloid progenitor cell (MPC) of the invention is expressing CD34, c-Kit (CD117) and CD115.

In an embodiment, a bone marrow-derived myeloid progenitor cell (MPC) of the invention is expressing CD34, c-Kit (CD117) and CD135.

In an embodiment, a bone marrow-derived myeloid progenitor cell (MPC) of the invention is expressing CD34, c-Kit (CD 117), CD115 and CD135.

In an embodiment, a bone marrow-derived myeloid progenitor cell (MPC) of the invention is expressing CD34, c-Kit (CD117) and CX3CR1.

In an embodiment, a bone marrow-derived myeloid progenitor cell (MPC) of the invention is expressing CD34, c-Kit (CD117), CD115 and CX3CR1.

In an embodiment, a bone marrow-derived myeloid progenitor cell (MPC) of the invention is expressing CD34, c-Kit (CD117), CD135 and CX3CR1.

In an embodiment, a bone marrow-derived myeloid progenitor cell (MPC) of the invention is expressing CD34, c-Kit (CD117), CD 115, CD135 and CX3CR1.

In an embodiment a bone marrow-derived myeloid progenitor cell (MPC) of the invention is expressing CD34 and c-Kit (CD117), but is IL-17Ra-negative.

In an embodiment, a bone marrow-derived myeloid progenitor cell (MPC) of the invention is expressing CD34, c-Kit (CD117) and CD115, but is IL-17Ra-negative.

In an embodiment, a bone marrow-derived myeloid progenitor cell (MPC) of the invention is expressing CD34, c-Kit (CD117) and CD135, but is IL-17Ra-negative.

In an embodiment, a bone marrow-derived myeloid progenitor cell (MPC) of the invention is expressing CD34, c-Kit (CD 117), CD115 and CD135, but is IL-17Ra-negative.

In an embodiment, a bone marrow-derived myeloid progenitor cell (MPC) of the invention is expressing CD34 c-Kit (CD117) and CX3CR1, but is IL-17Ra-negative.

In an embodiment, a bone marrow-derived myeloid progenitor cell (MPC) of the invention is expressing CD34, c-Kit (CD117), CD115 and CX3CR1, but is IL-17Ra-negative.

In an embodiment, a bone marrow-derived myeloid progenitor cell (MPC) of the invention is expressing CD34, c-Kit (CD117), CD135 and CX3CR1, but is IL-17Ra-negative.

In an embodiment, a bone marrow-derived myeloid progenitor cell (MPC) of the invention is expressing CD34, c-Kit (CD117), CD115, CD135 and CX3CR1, but is IL-17Ra-negative.

In an embodiment, a bone marrow-derived myeloid progenitor cell (MPC) of the invention is expressing CD34 and c-Kit (CD 117), but is negative for at least one B-cell marker.

In an embodiment, a bone marrow-derived myeloid progenitor cell (MPC) of the invention is expressing CD34, c-Kit (CD117) and CD115, but is negative for at least one B-cell marker.

In an embodiment, a bone marrow-derived myeloid progenitor cell (MPC) of the invention is expressing CD34, c-Kit (CD117) and CD 135, but is negative for at least one B-cell marker.

In an embodiment, a bone marrow-derived myeloid progenitor cell (MPC) of the invention is expressing CD34, c-Kit (CD117), CD115 and CD135, but is negative for at least one B-cell marker.

In an embodiment, a bone marrow-derived myeloid progenitor cell (MPC) of the invention is expressing CD34 c-Kit (CD117) and CX3CR1, but is negative for at least one B-cell marker. In an embodiment, a bone marrow-derived myeloid progenitor cell (MPC) of the invention is expressing CD34, c-Kit (CD117), CD115 and CX3CR1, but is negative for at least one B-cell marker.

In an embodiment, a bone marrow-derived myeloid progenitor cell (MPC) of the invention is expressing CD34, c-Kit (CD117), CD 135 and CX3CR1, but is negative for at least one B-cell marker.

In an embodiment, a bone marrow-derived myeloid progenitor cell (MPC) of the invention is expressing CD34, c-Kit (CD117), CD115, CD135 and CX3CR1, but is negative for at least one B-cell marker.

In an embodiment a bone marrow-derived myeloid progenitor cell (MPC) of the invention is expressing CD34 and c-Kit (CD117), but is IL-17Ra-negative and negative for at least one B-cell marker.

In an embodiment, a bone marrow-derived myeloid progenitor cell (MPC) of the invention is expressing CD34, c-Kit (CD 117) and CD115, but is IL-17Ra-negative and negative for at least one B-cell marker.

In an embodiment, a bone marrow-derived myeloid progenitor cell (MPC) of the invention is expressing CD34, c-Kit (CD117) and CD135, but is IL-17Ra-negative and negative for at least one B-cell marker.

In an embodiment, a bone marrow-derived myeloid progenitor cell (MPC) of the invention is expressing CD34, c-Kit (CD117), CD115 and CD135, but is IL-17Ra-negative and negative for at least one B-cell marker.

In an embodiment, a bone marrow-derived myeloid progenitor cell (MPC) of the invention is expressing CD34 c-Kit (CD117) and CX3CR1, but is IL-17Ra-negative and negative for at least one B-cell marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker.

In an embodiment, a bone marrow-derived myeloid progenitor cell (MPC) of the invention is expressing CD34, c-Kit (CD117), CD135 and CX3CR1, but is IL-17Ra-negative and negative for at least one B-cell marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one B-cell marker.

In an embodiment, a bone marrow-derived myeloid progenitor cell (MPC) of the invention is expressing CD34 and c-Kit (CD117), but is negative for at least one T-cell marker.

In an embodiment, a bone marrow-derived myeloid progenitor cell (MPC) of the invention is expressing CD34, c-Kit (CD 117) and CD 115, but is negative for at least one T-cell marker.

In an embodiment, a bone marrow-derived myeloid progenitor cell (MPC) of the invention is expressing CD34, c-Kit (CD117) and CD135, but is negative for at least one T-cell marker.

In an embodiment, a bone marrow-derived myeloid progenitor cell (MPC) of the invention is expressing CD34, c-Kit (CD 117), CD 115 and CD 135, but is negative for at least one T-cell marker.

In an embodiment, a bone marrow-derived myeloid progenitor cell (MPC) of the invention is expressing CD34 c-Kit (CD117) and CX3CR1, but is negative for at least one T-cell marker.

In an embodiment, a bone marrow-derived myeloid progenitor cell (MPC) of the invention is expressing CD34, c-Kit (CD117), CD115 and CX3CR1, but is negative for at least one T-cell marker.

In an embodiment, a bone marrow-derived myeloid progenitor cell (MPC) of the invention is expressing CD34, c-Kit (CD117), CD135 and CX3CR1, but is negative for at least one T-cell marker.

In an embodiment, a bone marrow-derived myeloid progenitor cell (MPC) of the invention is expressing CD34, c-Kit (CD117), CD115, CD135 and CX3CR1, but is negative for at least one T-cell marker.

In an embodiment a bone marrow-derived myeloid progenitor cell (MPC) of the invention is expressing CD34 and c-Kit (CD117), but is IL-17Ra-negative and negative for at least one T-cell marker.

In an embodiment, a bone marrow-derived myeloid progenitor cell (MPC) of the invention is expressing CD34, c-Kit (CD 117) and CD 115, but is IL-17Ra-negative and negative for at least one T-cell marker.

In an embodiment, a bone marrow-derived myeloid progenitor cell (MPC) of the invention is expressing CD34, c-Kit (CD117) and CD135, but is IL-17Ra-negative and negative for at least one T-cell marker.

In an embodiment, a bone marrow-derived myeloid progenitor cell (MPC) of the invention is expressing CD34, c-Kit (CD 117), CD115 and CD135, but is IL-17Ra-negative and negative for at least one T-cell marker.

In an embodiment, a bone marrow-derived myeloid progenitor cell (MPC) of the invention is expressing CD34 c-Kit (CD117) and CX3CR1, but is IL-17Ra-negative and negative for at least one T-cell marker.

In an embodiment, a bone marrow-derived myeloid progenitor cell (MPC) of the invention is expressing CD34, c-Kit (CD117), CD115 and CX3CR1, but is IL-17Ra-negative and negative for at least one T-cell marker.

In an embodiment, a bone marrow-derived myeloid progenitor cell (MPC) of the invention is expressing CD34, c-Kit (CD117), CD135 and CX3CR1, but is IL-17Ra-negative and negative for at least one T-cell marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one T-cell marker.

In an embodiment, a bone marrow-derived myeloid progenitor cell (MPC) of the invention is expressing CD34 and c-Kit (CD 117), but is negative for at least one B-cell marker and negative for at least one T-cell marker.

In an embodiment, a bone marrow-derived myeloid progenitor cell (MPC) of the invention is expressing CD34, c-Kit (CD 117) and CD 115, but is negative for at least one B-cell marker and negative for at least one T-cell marker.

In an embodiment, a bone marrow-derived myeloid progenitor cell (MPC) of the invention is expressing CD34, c-Kit (CD 117) and CD 135, but is negative for at least one B-cell marker and negative for at least one T-cell marker.

In an embodiment, a bone marrow-derived myeloid progenitor cell (MPC) of the invention is expressing CD34, c-Kit (CD117), CD115 and CD135, but is negative for at least one B-cell marker and negative for at least one T-cell marker.

In an embodiment, a bone marrow-derived myeloid progenitor cell (MPC) of the invention is expressing CD34 c-Kit (CD117) and CX3CR1, but is negative for at least one B-cell marker and negative for at least one T-cell marker.

In an embodiment, a bone marrow-derived myeloid progenitor cell (MPC) of the invention is expressing CD34, c-Kit (CD117), CD115 and CX3CR1, but is negative for at least one B-cell marker and negative for at least one T-cell marker.

In an embodiment, a bone marrow-derived myeloid progenitor cell (MPC) of the invention is expressing CD34, c-Kit (CD117), CD135 and CX3CR1, but is negative for at least one B-cell marker and negative for at least one T-cell marker.

In an embodiment, a bone marrow-derived myeloid progenitor cell (MPC) of the invention is expressing CD34, c-Kit (CD117), CD115, CD135 and CX3CR1, but is negative for at least one B-cell marker and negative for at least one T-cell marker.

In an embodiment a bone marrow-derived myeloid progenitor cell (MPC) of the invention is expressing CD34 and c-Kit (CD117), but is IL-17Ra-negative and negative for at least one B-cell marker and negative for at least one T-cell marker.

In an embodiment, a bone marrow-derived myeloid progenitor cell (MPC) of the invention is expressing CD34, c-Kit (CD 117) and CD115, but is IL-17Ra-negative and negative for at least one B-cell marker and negative for at least one T-cell marker.

In an embodiment, a bone marrow-derived myeloid progenitor cell (MPC) of the invention is expressing CD34, c-Kit (CD117) and CD135, but is IL-17Ra-negative and negative for at least one B-cell marker and negative for at least one T-cell marker.

In an embodiment, a bone marrow-derived myeloid progenitor cell (MPC) of the invention is expressing CD34, c-Kit (CD 117), CD 115 and CD 135, but is IL-17Ra-negative and negative for at least one B-cell marker and negative for at least one T-cell marker.

In an embodiment, a bone marrow-derived myeloid progenitor cell (MPC) of the invention is expressing CD34 c-Kit (CD117) and CX3CR1, but is IL-17Ra-negative and negative for at least one B-cell marker and negative for at least one T-cell marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker and negative for at least one T-cell marker.

In an embodiment, a bone marrow-derived myeloid progenitor cell (MPC) of the invention is expressing CD34, c-Kit (CD117), CD135 and CX3CR1, but is IL-17Ra-negative and negative for at least one B-cell marker and negative for at least one T-cell marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one B-cell marker and negative for at least one T-cell marker.

In an embodiment a bone marrow-derived myeloid progenitor cell (MPC) of the invention is expressing CD34 and c-Kit (CD 117), but is negative for at least one mature myeloid cell marker.

In an embodiment, a bone marrow-derived myeloid progenitor cell (MPC) of the invention is expressing CD34, c-Kit (CD117) and CD115, but is negative for at least one mature myeloid cell marker.

In an embodiment, a bone marrow-derived myeloid progenitor cell (MPC) of the invention is expressing CD34, c-Kit (CD117) and CD135, but is negative for at least one mature myeloid cell marker.

In an embodiment, a bone marrow-derived myeloid progenitor cell (MPC) of the invention is expressing CD34, c-Kit (CD 117), CD115 and CD 135, but is negative for at least one mature myeloid cell marker.

In an embodiment, a bone marrow-derived myeloid progenitor cell (MPC) of the invention is expressing CD34 c-Kit (CD117) and CX3CR1, but is negative for at least one mature myeloid cell marker.

In an embodiment, a bone marrow-derived myeloid progenitor cell (MPC) of the invention is expressing CD34, c-Kit (CD117), CD115 and CX3CR1, but is negative for at least one mature myeloid cell marker.

In an embodiment, a bone marrow-derived myeloid progenitor cell (MPC) of the invention is expressing CD34, c-Kit (CD117), CD135 and CX3CR1, but is negative for at least one mature myeloid cell marker.

In an embodiment, a bone marrow-derived myeloid progenitor cell (MPC) of the invention is expressing CD34, c-Kit (CD117), CD 115, CD135 and CX3CR1, but is negative for at least one mature myeloid cell marker.

In an embodiment a bone marrow-derived myeloid progenitor cell (MPC) of the invention is expressing CD34 and c-Kit (CD117), but is IL-17Ra-negative and is negative for at least one mature myeloid cell marker.

In an embodiment, a bone marrow-derived myeloid progenitor cell (MPC) of the invention is expressing CD34, c-Kit (CD 117) and CD 115, but is IL-17Ra-negative and is negative for at least one mature myeloid cell marker.

In an embodiment, a bone marrow-derived myeloid progenitor cell (MPC) of the invention is expressing CD34, c-Kit (CD117) and CD135, but is IL-17Ra-negative.

In an embodiment, a bone marrow-derived myeloid progenitor cell (MPC) of the invention is expressing CD34, c-Kit (CD117), CD115 and CD135, but is IL-17Ra-negative and is negative for at least one mature myeloid cell marker.

In an embodiment, a bone marrow-derived myeloid progenitor cell (MPC) of the invention is expressing CD34 c-Kit (CD117) and CX3CR1, but is IL-17Ra-negative and is negative for at least one mature myeloid cell marker.

In an embodiment, a bone marrow-derived myeloid progenitor cell (MPC) of the invention is expressing CD34, c-Kit (CD117), CD115 and CX3CR1, but is IL-17Ra-negative and is negative for at least one mature myeloid cell marker.

In an embodiment, a bone marrow-derived myeloid progenitor cell (MPC) of the invention is expressing CD34, c-Kit (CD117), CD135 and CX3CR1, but is IL-17Ra-negative and is negative for at least one mature myeloid cell marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one mature myeloid cell marker.

In an embodiment, a bone marrow-derived myeloid progenitor cell (MPC) of the invention is expressing CD34 and c-Kit (CD 117), but is negative for at least one B-cell marker and negative for at least one mature myeloid cell marker.

In an embodiment, a bone marrow-derived myeloid progenitor cell (MPC) of the invention is expressing CD34, c-Kit (CD 117) and CD 115, but is negative for at least one B-cell marker and negative for at least one mature myeloid cell marker.

In an embodiment, a bone marrow-derived myeloid progenitor cell (MPC) of the invention is expressing CD34, c-Kit (CD 117) and CD 135, but is negative for at least one B-cell marker and negative for at least one mature myeloid cell marker.

In an embodiment, a bone marrow-derived myeloid progenitor cell (MPC) of the invention is expressing CD34, c-Kit (CD117), CD115 and CD135, but is negative for at least one B-cell marker and negative for at least one mature myeloid cell marker.

In an embodiment, a bone marrow-derived myeloid progenitor cell (MPC) of the invention is expressing CD34 c-Kit (CD117) and CX3CR1, but is negative for at least one B-cell marker and negative for at least one mature myeloid cell marker.

In an embodiment, a bone marrow-derived myeloid progenitor cell (MPC) of the invention is expressing CD34, c-Kit (CD117), CD115 and CX3CR1, but is negative for at least one B-cell marker and negative for at least one mature myeloid cell marker.

In an embodiment, a bone marrow-derived myeloid progenitor cell (MPC) of the invention is expressing CD34, c-Kit (CD117), CD135 and CX3CR1, but is negative for at least one B-cell marker and negative for at least one mature myeloid cell marker.

In an embodiment, a bone marrow-derived myeloid progenitor cell (MPC) of the invention is expressing CD34, c-Kit (CD117), CD115, CD135 and CX3CR1, but is negative for at least one B-cell marker and negative for at least one mature myeloid cell marker.

In an embodiment a bone marrow-derived myeloid progenitor cell (MPC) of the invention is expressing CD34 and c-Kit (CD117), but is IL-17Ra-negative and negative for at least one B-cell marker and negative for at least one mature myeloid cell marker.

In an embodiment, a bone marrow-derived myeloid progenitor cell (MPC) of the invention is expressing CD34, c-Kit (CD117) and CD115, but is IL-17Ra-negative and negative for at least one B-cell marker and negative for at least one mature myeloid cell marker.

In an embodiment, a bone marrow-derived myeloid progenitor cell (MPC) of the invention is expressing CD34, c-Kit (CD117) and CD135, but is IL-17Ra-negative and negative for at least one B-cell marker and negative for at least one mature myeloid cell marker.

In an embodiment, a bone marrow-derived myeloid progenitor cell (MPC) of the invention is expressing CD34, c-Kit (CD117), CD115 and CD135, but is IL-17Ra-negative and negative for at least one B-cell marker and negative for at least one mature myeloid cell marker.

In an embodiment, a bone marrow-derived myeloid progenitor cell (MPC) of the invention is expressing CD34 c-Kit (CD117) and CX3CR1, but is IL-17Ra-negative and negative for at least one B-cell marker and negative for at least one mature myeloid cell marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker and negative for at least one mature myeloid cell marker.

In an embodiment, a bone marrow-derived myeloid progenitor cell (MPC) of the invention is expressing CD34, c-Kit (CD117), CD135 and CX3CR1, but is IL-17Ra-negative and negative for at least one B-cell marker and negative for at least one mature myeloid cell marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one B-cell marker and negative for at least one mature myeloid cell marker.

In an embodiment, a bone marrow-derived myeloid progenitor cell (MPC) of the invention is expressing CD34 and c-Kit (CD 117), but is negative for at least one T-cell marker and at least one mature myeloid cell marker.

In an embodiment, a bone marrow-derived myeloid progenitor cell (MPC) of the invention is expressing CD34, c-Kit (CD 117) and CD 115, but is negative for at least one T-cell marker and at least one mature myeloid cell marker.

In an embodiment, a bone marrow-derived myeloid progenitor cell (MPC) of the invention is expressing CD34, c-Kit (CD117) and CD135, but is negative for at least one T-cell marker and at least one mature myeloid cell marker.

In an embodiment, a bone marrow-derived myeloid progenitor cell (MPC) of the invention is expressing CD34, c-Kit (CD117), CD115 and CD135, but is negative for at least one T-cell marker and at least one mature myeloid cell marker.

In an embodiment, a bone marrow-derived myeloid progenitor cell (MPC) of the invention is expressing CD34 c-Kit (CD117) and CX3CR1, but is negative for at least one T-cell marker and at least one mature myeloid cell marker.

In an embodiment, a bone marrow-derived myeloid progenitor cell (MPC) of the invention is expressing CD34, c-Kit (CD117), CD115 and CX3CR1, but is negative for at least one T-cell marker and at least one mature myeloid cell marker.

In an embodiment, a bone marrow-derived myeloid progenitor cell (MPC) of the invention is expressing CD34, c-Kit (CD117), CD135 and CX3CR1, but is negative for at least one T-cell marker and at least one mature myeloid cell marker.

In an embodiment, a bone marrow-derived myeloid progenitor cell (MPC) of the invention is expressing CD34, c-Kit (CD117), CD115, CD135 and CX3CR1, but is negative for at least one T-cell marker and at least one mature myeloid cell marker.

In an embodiment a bone marrow-derived myeloid progenitor cell (MPC) of the invention is expressing CD34 and c-Kit (CD117), but is IL-17Ra-negative and negative for at least one T-cell marker and at least one mature myeloid cell marker.

In an embodiment, a bone marrow-derived myeloid progenitor cell (MPC) of the invention is expressing CD34, c-Kit (CD 117) and CD 115, but is IL-17Ra-negative and negative for at least one T-cell marker and at least one mature myeloid cell marker.

In an embodiment, a bone marrow-derived myeloid progenitor cell (MPC) of the invention is expressing CD34, c-Kit (CD117) and CD135, but is IL-17Ra-negative and negative for at least one T-cell marker and at least one mature myeloid cell marker.

In an embodiment, a bone marrow-derived myeloid progenitor cell (MPC) of the invention is expressing CD34, c-Kit (CD117), CD115 and CD135, but is IL-17Ra-negative and negative for at least one T-cell marker and at least one mature myeloid cell marker.

In an embodiment, a bone marrow-derived myeloid progenitor cell (MPC) of the invention is expressing CD34 c-Kit (CD117) and CX3CR1, but is IL-17Ra-negative and negative for at least one T-cell marker and at least one mature myeloid cell marker.

In an embodiment, a bone marrow-derived myeloid progenitor cell (MPC) of the invention is expressing CD34, c-Kit (CD117), CD115 and CX3CR1, but is IL-17Ra-negative and negative for at least one T-cell marker and at least one mature myeloid cell marker.

In an embodiment, a bone marrow-derived myeloid progenitor cell (MPC) of the invention is expressing CD34, c-Kit (CD117), CD135 and CX3CR1, but is IL-17Ra-negative and negative for at least one T-cell marker and at least one mature myeloid cell marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one T-cell marker and at least one mature myeloid cell marker.

In an embodiment, a bone marrow-derived myeloid progenitor cell (MPC) of the invention is expressing CD34 and c-Kit (CD117), but is negative for at least one B-cell marker and negative for at least one T-cell marker and negative for at least one mature myeloid cell marker.

In an embodiment, a bone marrow-derived myeloid progenitor cell (MPC) of the invention is expressing CD34, c-Kit (CD 117) and CD 115, but is negative for at least one B-cell marker and negative for at least one T-cell marker and negative for at least one mature myeloid cell marker.

In an embodiment, a bone marrow-derived myeloid progenitor cell (MPC) of the invention is expressing CD34, c-Kit (CD 117) and CD 135, but is negative for at least one B-cell marker and negative for at least one T-cell marker and negative for at least one mature myeloid cell marker.

In an embodiment, a bone marrow-derived myeloid progenitor cell (MPC) of the invention is expressing CD34, c-Kit (CD117), CD115 and CD135, but is negative for at least one B-cell marker and negative for at least one T-cell marker and negative for at least one mature myeloid cell marker.

In an embodiment, a bone marrow-derived myeloid progenitor cell (MPC) of the invention is expressing CD34 c-Kit (CD117) and CX3CR1, but is negative for at least one B-cell marker and negative for at least one T-cell marker and negative for at least one mature myeloid cell marker.

In an embodiment, a bone marrow-derived myeloid progenitor cell (MPC) of the invention is expressing CD34, c-Kit (CD117), CD115 and CX3CR1, but is negative for at least one B-cell marker and negative for at least one T-cell marker and negative for at least one mature myeloid cell marker.

In an embodiment, a bone marrow-derived myeloid progenitor cell (MPC) of the invention is expressing CD34, c-Kit (CD117), CD135 and CX3CR1, but is negative for at least one B-cell marker and negative for at least one T-cell marker and negative for at least one mature myeloid cell marker.

In an embodiment, a bone marrow-derived myeloid progenitor cell (MPC) of the invention is expressing CD34, c-Kit (CD117), CD115, CD135 and CX3CR1, but is negative for at least one B-cell marker and negative for at least one T-cell marker and negative for at least one mature myeloid cell marker.

In an embodiment a bone marrow-derived myeloid progenitor cell (MPC) of the invention is expressing CD34 and c-Kit (CD117), but is IL-17Ra-negative and negative for at least one B-cell marker and negative for at least one T-cell marker and negative for at least one mature myeloid cell marker.

In an embodiment, a bone marrow-derived myeloid progenitor cell (MPC) of the invention is expressing CD34, c-Kit (CD117) and CD115, but is IL-17Ra-negative and negative for at least one B-cell marker and negative for at least one T-cell marker and negative for at least one mature myeloid cell marker.

In an embodiment, a bone marrow-derived myeloid progenitor cell (MPC) of the invention is expressing CD34, c-Kit (CD117) and CD135, but is IL-17Ra-negative and negative for at least one B-cell marker and negative for at least one T-cell marker and negative for at least one mature myeloid cell marker.

In an embodiment, a bone marrow-derived myeloid progenitor cell (MPC) of the invention is expressing CD34, c-Kit (CD117), CD115 and CD135, but is IL-17Ra-negative and negative for at least one B-cell marker and negative for at least one T-cell marker and negative for at least one mature myeloid cell marker.

In an embodiment, a bone marrow-derived myeloid progenitor cell (MPC) of the invention is expressing CD34 c-Kit (CD117) and CX3CR1, but is IL-17Ra-negative and negative for at least one B-cell marker and negative for at least one T-cell marker and negative for at least one mature myeloid cell marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker and negative for at least one T-cell marker and negative for at least one mature myeloid cell marker.

In an embodiment, a bone marrow-derived myeloid progenitor cell (MPC) of the invention is expressing CD34, c-Kit (CD117), CD135 and CX3CR1, but is IL-17Ra-negative and negative for at least one B-cell marker and negative for at least one T-cell marker and negative for at least one mature myeloid cell marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one B-cell marker and negative for at least one T-cell marker and negative for at least one mature myeloid cell marker.

In an embodiment a bone marrow-derived myeloid progenitor cell (MPC) of the invention is expressing CD34 and c-Kit (CD 117), but is negative for at least one granulocyte/monocyte marker.

In an embodiment, a bone marrow-derived myeloid progenitor cell (MPC) of the invention is expressing CD34, c-Kit (CD 117) and CD 115, but is negative for at least one granulocyte/monocyte marker.

In an embodiment, a bone marrow-derived myeloid progenitor cell (MPC) of the invention is expressing CD34, c-Kit (CD 117) and CD 135, but is negative for at least one granulocyte/monocyte marker.

In an embodiment, a bone marrow-derived myeloid progenitor cell (MPC) of the invention is expressing CD34, c-Kit (CD117), CD115 and CD135, but is negative for at least one granulocyte/monocyte marker.

In an embodiment, a bone marrow-derived myeloid progenitor cell (MPC) of the invention is expressing CD34 c-Kit (CD 117) and CX3CR1, but is negative for at least one granulocyte/monocyte marker.

In an embodiment, a bone marrow-derived myeloid progenitor cell (MPC) of the invention is expressing CD34, c-Kit (CD117), CD115 and CX3CR1, but is negative for at least one granulocyte/monocyte marker.

In an embodiment, a bone marrow-derived myeloid progenitor cell (MPC) of the invention is expressing CD34, c-Kit (CD117), CD135 and CX3CR1, but is negative for at least one granulocyte/monocyte marker.

In an embodiment, a bone marrow-derived myeloid progenitor cell (MPC) of the invention is expressing CD34, c-Kit (CD117), CD115, CD135 and CX3CR1, but is negative for at least one granulocyte/monocyte marker.

In an embodiment a bone marrow-derived myeloid progenitor cell (MPC) of the invention is expressing CD34 and c-Kit (CD117), but is IL-17Ra-negative and is negative for at least one granulocyte/monocyte marker.

In an embodiment, a bone marrow-derived myeloid progenitor cell (MPC) of the invention is expressing CD34, c-Kit (CD117) and CD115, but is IL-17Ra-negative and is negative for at least one granulocyte/monocyte marker.

In an embodiment, a bone marrow-derived myeloid progenitor cell (MPC) of the invention is expressing CD34, c-Kit (CD117) and CD135, but is IL-17Ra-negative and is negative for at least one granulocyte/monocyte marker.

In an embodiment, a bone marrow-derived myeloid progenitor cell (MPC) of the invention is expressing CD34, c-Kit (CD117), CD115 and CD135, but is IL-17Ra-negative and is negative for at least one granulocyte/monocyte marker.

In an embodiment, a bone marrow-derived myeloid progenitor cell (MPC) of the invention is expressing CD34 c-Kit (CD117) and CX3CR1, but is IL-17Ra-negative and is negative for at least one granulocyte/monocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one granulocyte/monocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one granulocyte/monocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one granulocyte/monocyte marker.

In an embodiment, a bone marrow-derived myeloid progenitor cell (MPC) of the invention is expressing CD34 and c-Kit (CD 117), but is negative for at least one B-cell marker and is negative for at least one granulocyte/monocyte marker.

In an embodiment, a bone marrow-derived myeloid progenitor cell (MPC) of the invention is expressing CD34, c-Kit (CD 117) and CD 115, but is negative for at least one B-cell marker and is negative for at least one granulocyte/monocyte marker.

In an embodiment, a bone marrow-derived myeloid progenitor cell (MPC) of the invention is expressing CD34, c-Kit (CD 117) and CD 135, but is negative for at least one B-cell marker and is negative for at least one granulocyte/monocyte marker.

In an embodiment, a bone marrow-derived myeloid progenitor cell (MPC) of the invention is expressing CD34, c-Kit (CD 117), CD 115 and CD 13 5, but is negative for at least one B-cell marker and is negative for at least one granulocyte/monocyte marker.

In an embodiment, a bone marrow-derived myeloid progenitor cell (MPC) of the invention is expressing CD34 c-Kit (CD117) and CX3CR1, but is negative for at least one B-cell marker and is negative for at least one granulocyte/monocyte marker.

In an embodiment, a bone marrow-derived myeloid progenitor cell (MPC) of the invention is expressing CD34, c-Kit (CD117), CD115 and CX3CR1, but is negative for at least one B-cell marker and is negative for at least one granulocyte/monocyte marker.

In an embodiment, a bone marrow-derived myeloid progenitor cell (MPC) of the invention is expressing CD34, c-Kit (CD117), CD135 and CX3CR1, but is negative for at least one B-cell marker and negative for at least one granulocyte/monocyte marker.

In an embodiment, a bone marrow-derived myeloid progenitor cell (MPC) of the invention is expressing CD34, c-Kit (CD 117), CD 115, CD135 and CX3CR1, but is negative for at least one B-cell marker and negative for at least one granulocyte/monocyte marker.

In an embodiment a bone marrow-derived myeloid progenitor cell (MPC) of the invention is expressing CD34 and c-Kit (CD117), but is IL-17Ra-negative and negative for at least one B-cell marker and negative for at least one granulocyte/monocyte marker.

In an embodiment, a bone marrow-derived myeloid progenitor cell (MPC) of the invention is expressing CD34, c-Kit (CD 117) and CD 115, but is IL-17Ra-negative and negative for at least one B-cell marker and negative for at least one granulocyte/monocyte marker.

In an embodiment, a bone marrow-derived myeloid progenitor cell (MPC) of the invention is expressing CD34, c-Kit (CD117) and CD135, but is IL-17Ra-negative and negative for at least one B-cell marker and negative for at least one granulocyte/monocyte marker.

In an embodiment, a bone marrow-derived myeloid progenitor cell (MPC) of the invention is expressing CD34, c-Kit (CD117), CD115 and CD135, but is IL-17Ra-negative and negative for at least one B-cell marker and negative for at least one granulocyte/monocyte marker.

In an embodiment, a bone marrow-derived myeloid progenitor cell (MPC) of the invention is expressing CD34 c-Kit (CD117) and CX3CR1, but is IL-17Ra-negative and negative for at least one B-cell marker and negative for at least one granulocyte/monocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker and negative for at least one granulocyte/monocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one B-cell marker and negative for at least one granulocyte/monocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one B-cell marker and negative for at least one granulocyte/monocyte marker.

In an embodiment, a bone marrow-derived myeloid progenitor cell (MPC) of the invention is expressing CD34 and c-Kit (CD117), but is negative for at least one T-cell marker and negative for at least one granulocyte/monocyte marker.

In an embodiment, a bone marrow-derived myeloid progenitor cell (MPC) of the invention is expressing CD34, c-Kit (CD 117) and CD 115, but is negative for at least one T-cell marker and negative for at least one granulocyte/monocyte marker.

In an embodiment, a bone marrow-derived myeloid progenitor cell (MPC) of the invention is expressing CD34, c-Kit (CD117) and CD135, but is negative for at least one T-cell marker and negative for at least one granulocyte/monocyte marker.

In an embodiment, a bone marrow-derived myeloid progenitor cell (MPC) of the invention is expressing CD34, c-Kit (CD 117), CD 115 and CD 135, but is negative for at least one T-cell marker and negative for at least one granulocyte/monocyte marker.

In an embodiment, a bone marrow-derived myeloid progenitor cell (MPC) of the invention is expressing CD34 c-Kit (CD117) and CX3CR1, but is negative for at least one T-cell marker and negative for at least one granulocyte/monocyte marker.

In an embodiment, a bone marrow-derived myeloid progenitor cell (MPC) of the invention is expressing CD34, c-Kit (CD117), CD115 and CX3CR1, but is negative for at least one T-cell marker and negative for at least one granulocyte/monocyte marker.

In an embodiment, a bone marrow-derived myeloid progenitor cell (MPC) of the invention is expressing CD34, c-Kit (CD117), CD135 and CX3CR1, but is negative for at least one T-cell marker and negative for at least one granulocyte/monocyte marker.

In an embodiment, a bone marrow-derived myeloid progenitor cell (MPC) of the invention is expressing CD34, c-Kit (CD117), CD115, CD135 and CX3CR1, but is negative for at least one T-cell marker and negative for at least one granulocyte/monocyte marker.

In an embodiment a bone marrow-derived myeloid progenitor cell (MPC) of the invention is expressing CD34 and c-Kit (CD117), but is IL-17Ra-negative and negative for at least one T-cell marker and negative for at least one granulocyte/monocyte marker.

In an embodiment, a bone marrow-derived myeloid progenitor cell (MPC) of the invention is expressing CD34, c-Kit (CD 117) and CD 115, but is IL-17Ra-negative and negative for at least one T-cell marker and negative for at least one granulocyte/monocyte marker.

In an embodiment, a bone marrow-derived myeloid progenitor cell (MPC) of the invention is expressing CD34, c-Kit (CD117) and CD135, but is IL-17Ra-negative and negative for at least one T-cell marker and negative for at least one granulocyte/monocyte marker.

In an embodiment, a bone marrow-derived myeloid progenitor cell (MPC) of the invention is expressing CD34, c-Kit (CD117), CD 115 and CD 135, but is IL-17Ra-negative and negative for at least one T-cell marker and negative for at least one granulocyte/monocyte marker.

In an embodiment, a bone marrow-derived myeloid progenitor cell (MPC) of the invention is expressing CD34 c-Kit (CD117) and CX3CR1, but is IL-17Ra-negative and negative for at least one T-cell marker and negative for at least one granulocyte/monocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one T-cell marker and negative for at least one granulocyte/monocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one T-cell marker and negative for at least one granulocyte/monocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one T-cell marker and negative for at least one granulocyte/monocyte marker.

In an embodiment, a bone marrow-derived myeloid progenitor cell (MPC) of the invention is expressing CD34 and c-Kit (CD 117), but is negative for at least one B-cell marker and negative for at least one T-cell marker and negative for at least one granulocyte/monocyte marker.

In an embodiment, a bone marrow-derived myeloid progenitor cell (MPC) of the invention is expressing CD34, c-Kit (CD 117) and CD 115, but is negative for at least one B-cell marker and negative for at least one T-cell marker and negative for at least one granulocyte/monocyte marker.

In an embodiment, a bone marrow-derived myeloid progenitor cell (MPC) of the invention is expressing CD34, c-Kit (CD 117) and CD 135, but is negative for at least one B-cell marker and negative for at least one T-cell marker and negative for at least one granulocyte/monocyte marker.

In an embodiment, a bone marrow-derived myeloid progenitor cell (MPC) of the invention is expressing CD34, c-Kit (CD117), CD115 and CD135, but is negative for at least one B-cell marker and negative for at least one T-cell marker and negative for at least one granulocyte/monocyte marker.

In an embodiment, a bone marrow-derived myeloid progenitor cell (MPC) of the invention is expressing CD34 c-Kit (CD117) and CX3CR1, but is negative for at least one B-cell marker and negative for at least one T-cell marker and negative for at least one granulocyte/monocyte marker.

In an embodiment, a bone marrow-derived myeloid progenitor cell (MPC) of the invention is expressing CD34, c-Kit (CD117), CD115 and CX3CR1, but is negative for at least one B-cell marker and negative for at least one T-cell marker.

In an embodiment, a bone marrow-derived myeloid progenitor cell (MPC) of the invention is expressing CD34, c-Kit (CD117), CD135 and CX3CR1, but is negative for at least one B-cell marker and negative for at least one T-cell marker and negative for at least one granulocyte/monocyte marker.

In an embodiment, a bone marrow-derived myeloid progenitor cell (MPC) of the invention is expressing CD34, c-Kit (CD117), CD115, CD135 and CX3CR1, but is negative for at least one B-cell marker and negative for at least one T-cell marker and negative for at least one granulocyte/monocyte marker.

In an embodiment a bone marrow-derived myeloid progenitor cell (MPC) of the invention is expressing CD34 and c-Kit (CD117), but is IL-17Ra-negative and negative for at least one B-cell marker and negative for at least one T-cell marker and negative for at least one granulocyte/monocyte marker.

In an embodiment, a bone marrow-derived myeloid progenitor cell (MPC) of the invention is expressing CD34, c-Kit (CD 117) and CD 115, but is IL-17Ra-negative and negative for at least one B-cell marker and negative for at least one T-cell marker and negative for at least one granulocyte/monocyte marker.

In an embodiment, a bone marrow-derived myeloid progenitor cell (MPC) of the invention is expressing CD34, c-Kit (CD117) and CD135, but is IL-17Ra-negative and negative for at least one B-cell marker and negative for at least one T-cell marker and negative for at least one granulocyte/monocyte marker.

In an embodiment, a bone marrow-derived myeloid progenitor cell (MPC) of the invention is expressing CD34, c-Kit (CD117), CD115 and CD135, but is IL-17Ra-negative and negative for at least one B-cell marker and negative for at least one T-cell marker and negative for at least one granulocyte/monocyte marker.

In an embodiment, a bone marrow-derived myeloid progenitor cell (MPC) of the invention is expressing CD34 c-Kit (CD117) and CX3CR1, but is IL-17Ra-negative and negative for at least one B-cell marker and negative for at least one T-cell marker and negative for at least one granulocyte/monocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker and negative for at least one T-cell marker and negative for at least one granulocyte/monocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one B-cell marker and negative for at least one T-cell marker and negative for at least one granulocyte/monocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one B-cell marker and negative for at least one T-cell marker and negative for at least one granulocyte/monocyte marker.

In an embodiment a bone marrow-derived myeloid progenitor cell (MPC) of the invention is expressing CD34 and c-Kit (CD117), but is negative for at least one mature myeloid cell marker and negative for at least one granulocyte/monocyte marker.

In an embodiment, a bone marrow-derived myeloid progenitor cell (MPC) of the invention is expressing CD34, c-Kit (CD117) and CD115, but is negative for at least one mature myeloid cell marker and negative for at least one granulocyte/monocyte marker.

In an embodiment, a bone marrow-derived myeloid progenitor cell (MPC) of the invention is expressing CD34, c-Kit (CD117) and CD135, but is negative for at least one mature myeloid cell marker and negative for at least one granulocyte/monocyte marker.

In an embodiment, a bone marrow-derived myeloid progenitor cell (MPC) of the invention is expressing CD34, c-Kit (CD117), CD115 and CD135, but is negative for at least one mature myeloid cell marker and negative for at least one granulocyte/monocyte marker.

In an embodiment, a bone marrow-derived myeloid progenitor cell (MPC) of the invention is expressing CD34 c-Kit (CD117) and CX3CR1, but is negative for at least one mature myeloid cell marker and negative for at least one granulocyte/monocyte marker.

In an embodiment, a bone marrow-derived myeloid progenitor cell (MPC) of the invention is expressing CD34, c-Kit (CD117), CD115 and CX3CR1, but is negative for at least one mature myeloid cell marker and negative for at least one granulocyte/monocyte marker.

In an embodiment, a bone marrow-derived myeloid progenitor cell (MPC) of the invention is expressing CD34, c-Kit (CD117), CD135 and CX3CR1, but is negative for at least one mature myeloid cell marker and negative for at least one granulocyte/monocyte marker.

In an embodiment, a bone marrow-derived myeloid progenitor cell (MPC) of the invention is expressing CD34, c-Kit (CD117), CD115, CD135 and CX3CR1, but is negative for at least one mature myeloid cell marker and negative for at least one granulocyte/monocyte marker.

In an embodiment a bone marrow-derived myeloid progenitor cell (MPC) of the invention is expressing CD34 and c-Kit (CD117), but is IL-17Ra-negative and is negative for at least one mature myeloid cell marker and negative for at least one granulocyte/monocyte marker.

In an embodiment, a bone marrow-derived myeloid progenitor cell (MPC) of the invention is expressing CD34, c-Kit (CD 117) and CD115, but is IL-17Ra-negative and is negative for at least one mature myeloid cell marker and negative for at least one granulocyte/monocyte marker.

In an embodiment, a bone marrow-derived myeloid progenitor cell (MPC) of the invention is expressing CD34, c-Kit (CD117) and CD135, but is IL-17Ra-negative and negative for at least one granulocyte/monocyte marker.

In an embodiment, a bone marrow-derived myeloid progenitor cell (MPC) of the invention is expressing CD34, c-Kit (CD117), CD115 and CD135, but is IL-17Ra-negative and is negative for at least one mature myeloid cell marker and negative for at least one granulocyte/monocyte marker.

In an embodiment, a bone marrow-derived myeloid progenitor cell (MPC) of the invention is expressing CD34 c-Kit (CD117) and CX3CR1, but is IL-17Ra-negative and is negative for at least one mature myeloid cell marker and negative for at least one granulocyte/monocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one mature myeloid cell marker and negative for at least one granulocyte/monocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one mature myeloid cell marker and negative for at least one granulocyte/monocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one mature myeloid cell marker and negative for at least one granulocyte/monocyte marker.

In an embodiment, a bone marrow-derived myeloid progenitor cell (MPC) of the invention is expressing CD34 and c-Kit (CD117), but is negative for at least one B-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD 117) and CD 115, but is negative for at least one B-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD 117) and CD 135, but is negative for at least one B-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD117), CD115 and CD135, but is negative for at least one B-cell marker and negative for at least one mature myeloid cell marker and negative for at least one granulocyte/monocyte marker.

In an embodiment, a bone marrow-derived myeloid progenitor cell (MPC) of the invention is expressing CD34 c-Kit (CD117) and CX3CR1, but is negative for at least one B-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD 117), CD 115 and CX3CR1, but is negative for at least one B-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD117), CD135 and CX3CR1, but is negative for at least one B-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD117), CD115, CD135 and CX3CR1, but is negative for at least one B-cell marker and negative for at least one mature myeloid cell marker and negative for at least one granulocyte/monocyte marker.

In an embodiment a bone marrow-derived myeloid progenitor cell (MPC) of the invention is expressing CD34 and c-Kit (CD117), but is IL-17Ra-negative and negative for at least one B-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD 117) and CD115, but is IL-17Ra-negative and negative for at least one B-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD117) and CD135, but is IL-17Ra-negative and negative for at least one B-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD117), CD115 and CD135, but is IL-17Ra-negative and negative for at least one B-cell marker and negative for at least one mature myeloid cell marker and negative for at least one granulocyte/monocyte marker.

In an embodiment, a bone marrow-derived myeloid progenitor cell (MPC) of the invention is expressing CD34 c-Kit (CD 117) and CX3CR1, but is IL-17Ra-negative and negative for at least one B-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one B-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one B-cell marker and negative for at least one mature myeloid cell marker and negative for at least one granulocyte/monocyte marker.

In an embodiment, a bone marrow-derived myeloid progenitor cell (MPC) of the invention is expressing CD34 and c-Kit (CD117), but is negative for at least one T-cell marker and at least one mature myeloid cell marker and negative for at least one granulocyte/monocyte marker.

In an embodiment, a bone marrow-derived myeloid progenitor cell (MPC) of the invention is expressing CD34, c-Kit (CD 117) and CD 115, but is negative for at least one T-cell marker and at least one mature myeloid cell marker and negative for at least one granulocyte/monocyte marker.

In an embodiment, a bone marrow-derived myeloid progenitor cell (MPC) of the invention is expressing CD34, c-Kit (CD117) and CD135, but is negative for at least one T-cell marker and at least one mature myeloid cell marker and negative for at least one granulocyte/monocyte marker.

In an embodiment, a bone marrow-derived myeloid progenitor cell (MPC) of the invention is expressing CD34, c-Kit (CD117), CD115 and CD135, but is negative for at least one T-cell marker and at least one mature myeloid cell marker and negative for at least one granulocyte/monocyte marker.

In an embodiment, a bone marrow-derived myeloid progenitor cell (MPC) of the invention is expressing CD34 c-Kit (CD117) and CX3CR1, but is negative for at least one T-cell marker and at least one mature myeloid cell marker and negative for at least one granulocyte/monocyte marker.

In an embodiment, a bone marrow-derived myeloid progenitor cell (MPC) of the invention is expressing CD34, c-Kit (CD117), CD115 and CX3CR1, but is negative for at least one T-cell marker and at least one mature myeloid cell marker and negative for at least one granulocyte/monocyte marker.

In an embodiment, a bone marrow-derived myeloid progenitor cell (MPC) of the invention is expressing CD34, c-Kit (CD117), CD135 and CX3CR1, but is negative for at least one T-cell marker and at least one mature myeloid cell marker. and negative for at least one granulocyte/monocyte marker

In an embodiment, a bone marrow-derived myeloid progenitor cell (MPC) of the invention is expressing CD34, c-Kit (CD117), CD115, CD135 and CX3CR1, but is negative for at least one T-cell marker and at least one mature myeloid cell marker and negative for at least one granulocyte/monocyte marker.

In an embodiment a bone marrow-derived myeloid progenitor cell (MPC) of the invention is expressing CD34 and c-Kit (CD117), but is IL-17Ra-negative and negative for at least one T-cell marker and at least one mature myeloid cell marker and negative for at least one granulocyte/monocyte marker.

In an embodiment, a bone marrow-derived myeloid progenitor cell (MPC) of the invention is expressing CD34, c-Kit (CD 117) and CD 115, but is IL-17Ra-negative and negative for at least one T-cell marker and at least one mature myeloid cell marker and negative for at least one granulocyte/monocyte marker.

In an embodiment, a bone marrow-derived myeloid progenitor cell (MPC) of the invention is expressing CD34, c-Kit (CD117) and CD135, but is IL-17Ra-negative and negative for at least one T-cell marker and at least one mature myeloid cell marker and negative for at least one granulocyte/monocyte marker.

In an embodiment, a bone marrow-derived myeloid progenitor cell (MPC) of the invention is expressing CD34, c-Kit (CD 117), CD 115 and CD 135, but is IL-17Ra-negative and negative for at least one T-cell marker and at least one mature myeloid cell marker and negative for at least one granulocyte/monocyte marker.

In an embodiment, a bone marrow-derived myeloid progenitor cell (MPC) of the invention is expressing CD34 c-Kit (CD117) and CX3CR1, but is IL-17Ra-negative and negative for at least one T-cell marker and at least one mature myeloid cell marker and negative for at least one granulocyte/monocyte marker.

In an embodiment, a bone marrow-derived myeloid progenitor cell (MPC) of the invention is expressing CD34, c-Kit (CD 117), CD115 and CX3CR1, but is IL-17Ra-negative and negative for at least one T-cell marker and at least one mature myeloid cell marker and negative for at least one granulocyte/monocyte marker.

In an embodiment, a bone marrow-derived myeloid progenitor cell (MPC) of the invention is expressing CD34, c-Kit (CD117), CD 135 and CX3CR1, but is IL-17Ra-negative and negative for at least one T-cell marker and at least one mature myeloid cell marker and negative for at least one granulocyte/monocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one T-cell marker and at least one mature myeloid cell marker and negative for at least one granulocyte/monocyte marker.

In an embodiment, a bone marrow-derived myeloid progenitor cell (MPC) of the invention is expressing CD34 and c-Kit (CD117), but is negative for at least one B-cell marker and negative for at least one T-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD117) and CD115, but is negative for at least one B-cell marker and negative for at least one T-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD117) and CD135, but is negative for at least one B-cell marker and negative for at least one T-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD 117), CD 115 and CD135, but is negative for at least one B-cell marker and negative for at least one T-cell marker and negative for at least one mature myeloid cell marker and negative for at least one granulocyte/monocyte marker.

In an embodiment, a bone marrow-derived myeloid progenitor cell (MPC) of the invention is expressing CD34 c-Kit (CD117) and CX3CR1, but is negative for at least one B-cell marker and negative for at least one T-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD117), CD115 and CX3CR1, but is negative for at least one B-cell marker and negative for at least one T-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD117), CD135 and CX3CR1, but is negative for at least one B-cell marker and negative for at least one T-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD117), CD115, CD135 and CX3CR1, but is negative for at least one B-cell marker and negative for at least one T-cell marker and negative for at least one mature myeloid cell marker and negative for at least one granulocyte/monocyte marker.

In an embodiment a bone marrow-derived myeloid progenitor cell (MPC) of the invention is expressing CD34 and c-Kit (CD117), but is IL-17Ra-negative and negative for at least one B-cell marker and negative for at least one T-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD 117) and CD 115, but is IL-17Ra-negative and negative for at least one B-cell marker and negative for at least one T-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD117) and CD135, but is IL-17Ra-negative and negative for at least one B-cell marker and negative for at least one T-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD117), CD115 and CD135, but is IL-17Ra-negative and negative for at least one B-cell marker and negative for at least one T-cell marker and negative for at least one mature myeloid cell marker and negative for at least one granulocyte/monocyte marker.

In an embodiment, a bone marrow-derived myeloid progenitor cell (MPC) of the invention is expressing CD34 c-Kit (CD117) and CX3CR1, but is IL-17Ra-negative and negative for at least one B-cell marker and negative for at least one T-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker and negative for at least one T-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one B-cell marker and negative for at least one T-cell marker and negative for at least one mature myeloid cell marker and negative for at least one granulocyte/monocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one B-cell marker and negative for at least one T-cell marker and negative for at least one mature myeloid cell marker and negative for at least one granulocyte/monocyte marker.

In an embodiment a bone marrow-derived myeloid progenitor cell (MPC) of the invention is expressing CD34 and c-Kit (CD 117), but is negative for at least one granulocyte marker.

In an embodiment, a bone marrow-derived myeloid progenitor cell (MPC) of the invention is expressing CD34, c-Kit (CD117) and CD115, but is negative for at least one granulocyte marker.

In an embodiment, a bone marrow-derived myeloid progenitor cell (MPC) of the invention is expressing CD34, c-Kit (CD 117) and CD 135, but is negative for at least one granulocyte marker.

In an embodiment, a bone marrow-derived myeloid progenitor cell (MPC) of the invention is expressing CD34, c-Kit (CD117), CD115 and CD135, but is negative for at least one granulocyte marker.

In an embodiment, a bone marrow-derived myeloid progenitor cell (MPC) of the invention is expressing CD34 c-Kit (CD117) and CX3CR1, but is negative for at least one granulocyte marker.

In an embodiment, a bone marrow-derived myeloid progenitor cell (MPC) of the invention is expressing CD34, c-Kit (CD117), CD115 and CX3CR1, but is negative for at least one granulocyte marker.

In an embodiment, a bone marrow-derived myeloid progenitor cell (MPC) of the invention is expressing CD34, c-Kit (CD117), CD135 and CX3CR1, but is negative for at least one granulocyte marker.

In an embodiment, a bone marrow-derived myeloid progenitor cell (MPC) of the invention is expressing CD34, c-Kit (CD117), CD115, CD135 and CX3CR1, but is negative for at least one granulocyte marker.

In an embodiment a bone marrow-derived myeloid progenitor cell (MPC) of the invention is expressing CD34 and c-Kit (CD117), but is IL-17Ra-negative and is negative for at least one granulocyte marker.

In an embodiment, a bone marrow-derived myeloid progenitor cell (MPC) of the invention is expressing CD34, c-Kit (CD 117) and CD 115, but is IL-17Ra-negative and is negative for at least one granulocyte marker.

In an embodiment, a bone marrow-derived myeloid progenitor cell (MPC) of the invention is expressing CD34, c-Kit (CD 117) and CD135, but is IL-17Ra-negative and is negative for at least one granulocyte marker.

In an embodiment, a bone marrow-derived myeloid progenitor cell (MPC) of the invention is expressing CD34, c-Kit (CD 117), CD115 and CD 135, but is IL-17Ra-negative and is negative for at least one granulocyte marker.

In an embodiment, a bone marrow-derived myeloid progenitor cell (MPC) of the invention is expressing CD34 c-Kit (CD117) and CX3CR1, but is IL-17Ra-negative and is negative for at least one granulocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one granulocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one granulocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one granulocyte marker.

In an embodiment, a bone marrow-derived myeloid progenitor cell (MPC) of the invention is expressing CD34 and c-Kit (CD117), but is negative for at least one B-cell marker and is negative for at least one granulocyte marker.

In an embodiment, a bone marrow-derived myeloid progenitor cell (MPC) of the invention is expressing CD34, c-Kit (CD117) and CD115, but is negative for at least one B-cell marker and is negative for at least one granulocyte marker.

In an embodiment, a bone marrow-derived myeloid progenitor cell (MPC) of the invention is expressing CD34, c-Kit (CD 117) and CD 135, but is negative for at least one B-cell marker and is negative for at least one granulocyte marker.

In an embodiment, a bone marrow-derived myeloid progenitor cell (MPC) of the invention is expressing CD34, c-Kit (CD117), CD115 and CD135, but is negative for at least one B-cell marker and is negative for at least one granulocyte marker.

In an embodiment, a bone marrow-derived myeloid progenitor cell (MPC) of the invention is expressing CD34 c-Kit (CD117) and CX3CR1, but is negative for at least one B-cell marker and is negative for at least one granulocyte marker.

In an embodiment, a bone marrow-derived myeloid progenitor cell (MPC) of the invention is expressing CD34, c-Kit (CD117), CD115 and CX3CR1, but is negative for at least one B-cell marker and is negative for at least one granulocyte marker.

In an embodiment, a bone marrow-derived myeloid progenitor cell (MPC) of the invention is expressing CD34, c-Kit (CD117), CD135 and CX3CR1, but is negative for at least one B-cell marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is negative for at least one B-cell marker and is negative for at least one granulocyte marker.

In an embodiment a bone marrow-derived myeloid progenitor cell (MPC) of the invention is expressing CD34 and c-Kit (CD117), but is IL-17Ra-negative and negative for at least one B-cell marker and is negative for at least one granulocyte marker.

In an embodiment, a bone marrow-derived myeloid progenitor cell (MPC) of the invention is expressing CD34, c-Kit (CD 117) and CD 115, but is IL-17Ra-negative and negative for at least one B-cell marker and is negative for at least one granulocyte marker.

In an embodiment, a bone marrow-derived myeloid progenitor cell (MPC) of the invention is expressing CD34, c-Kit (CD117) and CD135, but is IL-17Ra-negative and negative for at least one B-cell marker and is negative for at least one granulocyte marker.

In an embodiment, a bone marrow-derived myeloid progenitor cell (MPC) of the invention is expressing CD34, c-Kit (CD 117), CD115 and CD135, but is IL-17Ra-negative and negative for at least one B-cell marker and is negative for at least one granulocyte marker.

In an embodiment, a bone marrow-derived myeloid progenitor cell (MPC) of the invention is expressing CD34 c-Kit (CD117) and CX3CR1, but is IL-17Ra-negative and negative for at least one B-cell marker and is negative for at least one granulocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker and is negative for at least one granulocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one B-cell marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one B-cell marker and is negative for at least one granulocyte marker.

In an embodiment, a bone marrow-derived myeloid progenitor cell (MPC) of the invention is expressing CD34 and c-Kit (CD 117), but is negative for at least one T-cell marker and is negative for at least one granulocyte marker.

In an embodiment, a bone marrow-derived myeloid progenitor cell (MPC) of the invention is expressing CD34, c-Kit (CD 117) and CD 115, but is negative for at least one T-cell marker and is negative for at least one granulocyte marker.

In an embodiment, a bone marrow-derived myeloid progenitor cell (MPC) of the invention is expressing CD34, c-Kit (CD117) and CD135, but is negative for at least one T-cell marker and is negative for at least one granulocyte marker.

In an embodiment, a bone marrow-derived myeloid progenitor cell (MPC) of the invention is expressing CD34, c-Kit (CD117), CD115 and CD135, but is negative for at least one T-cell marker and is negative for at least one granulocyte marker.

In an embodiment, a bone marrow-derived myeloid progenitor cell (MPC) of the invention is expressing CD34 c-Kit (CD117) and CX3CR1, but is negative for at least one T-cell marker and is negative for at least one granulocyte marker.

In an embodiment, a bone marrow-derived myeloid progenitor cell (MPC) of the invention is expressing CD34, c-Kit (CD117), CD115 and CX3CR1, but is negative for at least one T-cell marker and is negative for at least one granulocyte marker.

In an embodiment, a bone marrow-derived myeloid progenitor cell (MPC) of the invention is expressing CD34, c-Kit (CD117), CD135 and CX3CR1, but is negative for at least one T-cell marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is negative for at least one T-cell marker and is negative for at least one granulocyte marker.

In an embodiment a bone marrow-derived myeloid progenitor cell (MPC) of the invention is expressing CD34 and c-Kit (CD117), but is IL-17Ra-negative and negative for at least one T-cell marker and is negative for at least one granulocyte marker.

In an embodiment, a bone marrow-derived myeloid progenitor cell (MPC) of the invention is expressing CD34, c-Kit (CD 117) and CD 115, but is IL-17Ra-negative and negative for at least one T-cell marker and is negative for at least one granulocyte marker.

In an embodiment, a bone marrow-derived myeloid progenitor cell (MPC) of the invention is expressing CD34, c-Kit (CD117) and CD135, but is IL-17Ra-negative and negative for at least one T-cell marker and is negative for at least one granulocyte marker.

In an embodiment, a bone marrow-derived myeloid progenitor cell (MPC) of the invention is expressing CD34, c-Kit (CD117), CD115 and CD135, but is IL-17Ra-negative and negative for at least one T-cell marker and is negative for at least one granulocyte marker.

In an embodiment, a bone marrow-derived myeloid progenitor cell (MPC) of the invention is expressing CD34 c-Kit (CD 117) and CX3CR1, but is IL-17Ra-negative and negative for at least one T-cell marker and is negative for at least one granulocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one T-cell marker and is negative for at least one granulocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one T-cell marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one T-cell marker and is negative for at least one granulocyte marker.

In an embodiment, a bone marrow-derived myeloid progenitor cell (MPC) of the invention is expressing CD34 and c-Kit (CD117), but is negative for at least one B-cell marker and negative for at least one T-cell marker and is negative for at least one granulocyte marker.

In an embodiment, a bone marrow-derived myeloid progenitor cell (MPC) of the invention is expressing CD34, c-Kit (CD 117) and CD 115, but is negative for at least one B-cell marker and negative for at least one T-cell marker and is negative for at least one granulocyte marker.

In an embodiment, a bone marrow-derived myeloid progenitor cell (MPC) of the invention is expressing CD34, c-Kit (CD117) and CD135, but is negative for at least one B-cell marker and negative for at least one T-cell marker and is negative for at least one granulocyte marker.

In an embodiment, a bone marrow-derived myeloid progenitor cell (MPC) of the invention is expressing CD34, c-Kit (CD117), CD115 and CD135, but is negative for at least one B-cell marker and negative for at least one T-cell marker and is negative for at least one granulocyte marker.

In an embodiment, a bone marrow-derived myeloid progenitor cell (MPC) of the invention is expressing CD34 c-Kit (CD117) and CX3CR1, but is negative for at least one B-cell marker and negative for at least one T-cell marker and is negative for at least one granulocyte marker.

In an embodiment, a bone marrow-derived myeloid progenitor cell (MPC) of the invention is expressing CD34, c-Kit (CD117), CD115 and CX3CR1, but is negative for at least one B-cell marker and negative for at least one T-cell marker and is negative for at least one granulocyte marker.

In an embodiment, a bone marrow-derived myeloid progenitor cell (MPC) of the invention is expressing CD34, c-Kit (CD117), CD135 and CX3CR1, but is negative for at least one B-cell marker and negative for at least one T-cell marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is negative for at least one B-cell marker and negative for at least one T-cell marker and is negative for at least one granulocyte marker.

In an embodiment a bone marrow-derived myeloid progenitor cell (MPC) of the invention is expressing CD34 and c-Kit (CD117), but is IL-17Ra-negative and negative for at least one B-cell marker and negative for at least one T-cell marker and is negative for at least one granulocyte marker.

In an embodiment, a bone marrow-derived myeloid progenitor cell (MPC) of the invention is expressing CD34, c-Kit (CD117) and CD115, but is IL-17Ra-negative and negative for at least one B-cell marker and negative for at least one T-cell marker and is negative for at least one granulocyte marker.

In an embodiment, a bone marrow-derived myeloid progenitor cell (MPC) of the invention is expressing CD34, c-Kit (CD117) and CD135, but is IL-17Ra-negative and negative for at least one B-cell marker and negative for at least one T-cell marker and is negative for at least one granulocyte marker.

In an embodiment, a bone marrow-derived myeloid progenitor cell (MPC) of the invention is expressing CD34, c-Kit (CD117), CD115 and CD135, but is IL-17Ra-negative and negative for at least one B-cell marker and negative for at least one T-cell marker and is negative for at least one granulocyte marker.

In an embodiment, a bone marrow-derived myeloid progenitor cell (MPC) of the invention is expressing CD34 c-Kit (CD117) and CX3CR1, but is IL-17Ra-negative and negative for at least one B-cell marker and negative for at least one T-cell marker and is negative for at least one granulocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker and negative for at least one T-cell marker and is negative for at least one granulocyte marker.

In an embodiment, a bone marrow-derived myeloid progenitor cell (MPC) of the invention is expressing CD34, c-Kit (CD117), CD 135 and CX3CR1, but is IL-17Ra-negative and negative for at least one B-cell marker and negative for at least one T-cell marker and is negative for at least one granulocyte marker.

In an embodiment, a bone marrow-derived myeloid progenitor cell (MPC) of the invention is expressing CD34, c-Kit (CD117), CD 115, CD 135 and CX3CR1, but is IL-17Ra-negative and negative for at least one B-cell marker and negative for at least one T-cell marker and is negative for at least one granulocyte marker.

In an embodiment a bone marrow-derived myeloid progenitor cell (MPC) of the invention is expressing CD34 and c-Kit (CD 117), but is negative for at least one mature myeloid cell marker and is negative for at least one granulocyte marker.

In an embodiment, a bone marrow-derived myeloid progenitor cell (MPC) of the invention is expressing CD34, c-Kit (CD117) and CD115, but is negative for at least one mature myeloid cell marker and is negative for at least one granulocyte marker.

In an embodiment, a bone marrow-derived myeloid progenitor cell (MPC) of the invention is expressing CD34, c-Kit (CD117) and CD135, but is negative for at least one mature myeloid cell marker and is negative for at least one granulocyte marker.

In an embodiment, a bone marrow-derived myeloid progenitor cell (MPC) of the invention is expressing CD34, c-Kit (CD117), CD115 and CD135, but is negative for at least one mature myeloid cell marker and is negative for at least one granulocyte marker.

In an embodiment, a bone marrow-derived myeloid progenitor cell (MPC) of the invention is expressing CD34 c-Kit (CD117) and CX3CR1, but is negative for at least one mature myeloid cell marker and is negative for at least one granulocyte marker.

In an embodiment, a bone marrow-derived myeloid progenitor cell (MPC) of the invention is expressing CD34, c-Kit (CD117), CD115 and CX3CR1, but is negative for at least one mature myeloid cell marker and is negative for at least one granulocyte marker.

In an embodiment, a bone marrow-derived myeloid progenitor cell (MPC) of the invention is expressing CD34, c-Kit (CD117), CD135 and CX3CR1, but is negative for at least one mature myeloid cell marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is negative for at least one mature myeloid cell marker and is negative for at least one granulocyte marker.

In an embodiment a bone marrow-derived myeloid progenitor cell (MPC) of the invention is expressing CD34 and c-Kit (CD117), but is IL-17Ra-negative and is negative for at least one mature myeloid cell marker and is negative for at least one granulocyte marker.

In an embodiment, a bone marrow-derived myeloid progenitor cell (MPC) of the invention is expressing CD34, c-Kit (CD117) and CD115, but is IL-17Ra-negative and is negative for at least one mature myeloid cell marker and is negative for at least one granulocyte marker.

In an embodiment, a bone marrow-derived myeloid progenitor cell (MPC) of the invention is expressing CD34, c-Kit (CD117) and CD135, but is IL-17Ra-negative and is negative for at least one granulocyte marker.

In an embodiment, a bone marrow-derived myeloid progenitor cell (MPC) of the invention is expressing CD34, c-Kit (CD117), CD115 and CD135, but is IL-17Ra-negative and is negative for at least one mature myeloid cell marker and is negative for at least one granulocyte marker.

In an embodiment, a bone marrow-derived myeloid progenitor cell (MPC) of the invention is expressing CD34 c-Kit (CD117) and CX3CR1, but is IL-17Ra-negative and is negative for at least one mature myeloid cell marker and is negative for at least one granulocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one mature myeloid cell marker and is negative for at least one granulocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one mature myeloid cell marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one mature myeloid cell marker and is negative for at least one granulocyte marker.

In an embodiment, a bone marrow-derived myeloid progenitor cell (MPC) of the invention is expressing CD34 and c-Kit (CD117), but is negative for at least one B-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD 117) and CD 115, but is negative for at least one B-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD117) and CD135, but is negative for at least one B-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD117), CD115 and CD135, but is negative for at least one B-cell marker and negative for at least one mature myeloid cell marker and is negative for at least one granulocyte marker.

In an embodiment, a bone marrow-derived myeloid progenitor cell (MPC) of the invention is expressing CD34 c-Kit (CD117) and CX3CR1, but is negative for at least one B-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD117), CD115 and CX3CR1, but is negative for at least one B-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD117), CD135 and CX3CR1, but is negative for at least one B-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is negative for at least one B-cell marker and negative for at least one mature myeloid cell marker and is negative for at least one granulocyte marker.

In an embodiment a bone marrow-derived myeloid progenitor cell (MPC) of the invention is expressing CD34 and c-Kit (CD117), but is IL-17Ra-negative and negative for at least one B-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD 117) and CD 115, but is IL-17Ra-negative and negative for at least one B-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD117) and CD135, but is IL-17Ra-negative and negative for at least one B-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD117), CD115 and CD135, but is IL-17Ra-negative and negative for at least one B-cell marker and negative for at least one mature myeloid cell marker and is negative for at least one granulocyte marker.

In an embodiment, a bone marrow-derived myeloid progenitor cell (MPC) of the invention is expressing CD34 c-Kit (CD117) and CX3CR1, but is IL-17Ra-negative and negative for at least one B-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD117), CD 135 and CX3CR1, but is IL-17Ra-negative and negative for at least one B-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one B-cell marker and negative for at least one mature myeloid cell marker and is negative for at least one granulocyte marker.

In an embodiment, a bone marrow-derived myeloid progenitor cell (MPC) of the invention is expressing CD34 and c-Kit (CD 117), but is negative for at least one T-cell marker and at least one mature myeloid cell marker and is negative for at least one granulocyte marker.

In an embodiment, a bone marrow-derived myeloid progenitor cell (MPC) of the invention is expressing CD34, c-Kit (CD 117) and CD 115, but is negative for at least one T-cell marker and at least one mature myeloid cell marker and is negative for at least one granulocyte marker.

In an embodiment, a bone marrow-derived myeloid progenitor cell (MPC) of the invention is expressing CD34, c-Kit (CD117) and CD135, but is negative for at least one T-cell marker and at least one mature myeloid cell marker and is negative for at least one granulocyte marker.

In an embodiment, a bone marrow-derived myeloid progenitor cell (MPC) of the invention is expressing CD34, c-Kit (CD117), CD115 and CD135, but is negative for at least one T-cell marker and at least one mature myeloid cell marker and is negative for at least one granulocyte marker.

In an embodiment, a bone marrow-derived myeloid progenitor cell (MPC) of the invention is expressing CD34 c-Kit (CD117) and CX3CR1, but is negative for at least one T-cell marker and at least one mature myeloid cell marker and is negative for at least one granulocyte marker.

In an embodiment, a bone marrow-derived myeloid progenitor cell (MPC) of the invention is expressing CD34, c-Kit (CD117), CD115 and CX3CR1, but is negative for at least one T-cell marker and at least one mature myeloid cell marker and is negative for at least one granulocyte marker.

In an embodiment, a bone marrow-derived myeloid progenitor cell (MPC) of the invention is expressing CD34, c-Kit (CD117), CD135 and CX3CR1, but is negative for at least one T-cell marker and at least one mature myeloid cell marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is negative for at least one T-cell marker and at least one mature myeloid cell marker and is negative for at least one granulocyte marker.

In an embodiment a bone marrow-derived myeloid progenitor cell (MPC) of the invention is expressing CD34 and c-Kit (CD117), but is IL-17Ra-negative and negative for at least one T-cell marker and at least one mature myeloid cell marker and is negative for at least one granulocyte marker.

In an embodiment, a bone marrow-derived myeloid progenitor cell (MPC) of the invention is expressing CD34, c-Kit (CD117) and CD115, but is IL-17Ra-negative and negative for at least one T-cell marker and at least one mature myeloid cell marker and is negative for at least one granulocyte marker.

In an embodiment, a bone marrow-derived myeloid progenitor cell (MPC) of the invention is expressing CD34, c-Kit (CD117) and CD135, but is IL-17Ra-negative and negative for at least one T-cell marker and at least one mature myeloid cell marker and is negative for at least one granulocyte marker.

In an embodiment, a bone marrow-derived myeloid progenitor cell (MPC) of the invention is expressing CD34, c-Kit (CD 117), CD115 and CD135, but is IL-17Ra-negative and negative for at least one T-cell marker and at least one mature myeloid cell marker and is negative for at least one granulocyte marker.

In an embodiment, a bone marrow-derived myeloid progenitor cell (MPC) of the invention is expressing CD34 c-Kit (CD117) and CX3CR1, but is IL-17Ra-negative and negative for at least one T-cell marker and at least one mature myeloid cell marker and is negative for at least one granulocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one T-cell marker and at least one mature myeloid cell marker and is negative for at least one granulocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one T-cell marker and at least one mature myeloid cell marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one T-cell marker and at least one mature myeloid cell marker and is negative for at least one granulocyte marker.

In an embodiment, a bone marrow-derived myeloid progenitor cell (MPC) of the invention is expressing CD34 and c-Kit (CD117), but is negative for at least one B-cell marker and negative for at least one T-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD117) and CD115, but is negative for at least one B-cell marker and negative for at least one T-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD117) and CD 135, but is negative for at least one B-cell marker and negative for at least one T-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD 117), CD 115 and CD 135, but is negative for at least one B-cell marker and negative for at least one T-cell marker and negative for at least one mature myeloid cell marker and is negative for at least one granulocyte marker.

In an embodiment, a bone marrow-derived myeloid progenitor cell (MPC) of the invention is expressing CD34 c-Kit (CD117) and CX3CR1, but is negative for at least one B-cell marker and negative for at least one T-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD117), CD115 and CX3CR1, but is negative for at least one B-cell marker and negative for at least one T-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD117), CD135 and CX3CR1, but is negative for at least one B-cell marker and negative for at least one T-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is negative for at least one B-cell marker and negative for at least one T-cell marker and negative for at least one mature myeloid cell marker and is negative for at least one granulocyte marker.

In an embodiment a bone marrow-derived myeloid progenitor cell (MPC) of the invention is expressing CD34 and c-Kit (CD117), but is IL-17Ra-negative and negative for at least one B-cell marker and negative for at least one T-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD117) and CD115, but is IL-17Ra-negative and negative for at least one B-cell marker and negative for at least one T-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD117) and CD135, but is IL-17Ra-negative and negative for at least one B-cell marker and negative for at least one T-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD117), CD115 and CD135, but is IL-17Ra-negative and negative for at least one B-cell marker and negative for at least one T-cell marker and negative for at least one mature myeloid cell marker and is negative for at least one granulocyte marker.

In an embodiment, a bone marrow-derived myeloid progenitor cell (MPC) of the invention is expressing CD34 c-Kit (CD117) and CX3CR1, but is IL-17Ra-negative and negative for at least one B-cell marker and negative for at least one T-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker and negative for at least one T-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one B-cell marker and negative for at least one T-cell marker and negative for at least one mature myeloid cell marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one B-cell marker and negative for at least one T-cell marker and negative for at least one mature myeloid cell marker and is negative for at least one granulocyte marker.

In an embodiment a bone marrow-derived myeloid progenitor cell (MPC) of the invention is expressing CD34 and c-Kit (CD117), but is negative for at least one granulocyte/monocyte marker and is negative for at least one granulocyte marker.

In an embodiment, a bone marrow-derived myeloid progenitor cell (MPC) of the invention is expressing CD34, c-Kit (CD 117) and CD 115, but is negative for at least one granulocyte/monocyte marker and is negative for at least one granulocyte marker.

In an embodiment, a bone marrow-derived myeloid progenitor cell (MPC) of the invention is expressing CD34, c-Kit (CD 117) and CD135, but is negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CD135, but is negative for at least one granulocyte/monocyte marker and is negative for at least one granulocyte marker.

In an embodiment, a bone marrow-derived myeloid progenitor cell (MPC) of the invention is expressing CD34 c-Kit (CD117) and CX3CR1, but is negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CX3CR1, but is negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD135 and CX3CR1, but is negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is negative for at least one granulocyte/monocyte marker and is negative for at least one granulocyte marker.

In an embodiment a bone marrow-derived myeloid progenitor cell (MPC) of the invention is expressing CD34 and c-Kit (CD117), but is IL-17Ra-negative and is negative for at least one granulocyte/monocyte marker and is negative for at least one granulocyte marker.

In an embodiment, a bone marrow-derived myeloid progenitor cell (MPC) of the invention is expressing CD34, c-Kit (CD117) and CD115, but is IL-17Ra-negative and is negative for at least one granulocyte/monocyte marker and is negative for at least one granulocyte marker.

In an embodiment, a bone marrow-derived myeloid progenitor cell (MPC) of the invention is expressing CD34, c-Kit (CD117) and CD135, but is IL-17Ra-negative and is negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CD135, but is IL-17Ra-negative and is negative for at least one granulocyte/monocyte marker and is negative for at least one granulocyte marker.

In an embodiment, a bone marrow-derived myeloid progenitor cell (MPC) of the invention is expressing CD34 c-Kit (CD117) and CX3CR1, but is IL-17Ra-negative and is negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115, CD 135 and CX3CR1, but is IL-17Ra-negative and is negative for at least one granulocyte/monocyte marker and is negative for at least one granulocyte marker.

In an embodiment, a bone marrow-derived myeloid progenitor cell (MPC) of the invention is expressing CD34 and c-Kit (CD117), but is negative for at least one B-cell marker and is negative for at least one granulocyte/monocyte marker and is negative for at least one granulocyte marker.

In an embodiment, a bone marrow-derived myeloid progenitor cell (MPC) of the invention is expressing CD34, c-Kit (CD 117) and CD 115, but is negative for at least one B-cell marker and is negative for at least one granulocyte/monocyte marker and is negative for at least one granulocyte marker.

In an embodiment, a bone marrow-derived myeloid progenitor cell (MPC) of the invention is expressing CD34, c-Kit (CD117) and CD 135, but is negative for at least one B-cell marker and is negative for at least one granulocyte/monocyte marker and is negative for at least one granulocyte marker.

In an embodiment, a bone marrow-derived myeloid progenitor cell (MPC) of the invention is expressing CD34, c-Kit (CD 117), CD 115 and CD 135, but is negative for at least one B-cell marker and is negative for at least one granulocyte/monocyte marker and is negative for at least one granulocyte marker.

In an embodiment, a bone marrow-derived myeloid progenitor cell (MPC) of the invention is expressing CD34 c-Kit (CD117) and CX3CR1, but is negative for at least one B-cell marker and is negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CX3CR1, but is negative for at least one B-cell marker and is negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD135 and CX3CR1, but is negative for at least one B-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is negative for at least one B-cell marker and negative for at least one granulocyte/monocyte marker and is negative for at least one granulocyte marker.

In an embodiment a bone marrow-derived myeloid progenitor cell (MPC) of the invention is expressing CD34 and c-Kit (CD117), but is IL-17Ra-negative and negative for at least one B-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117) and CD115, but is IL-17Ra-negative and negative for at least one B-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117) and CD135, but is IL-17Ra-negative and negative for at least one B-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD 117), CD 115 and CD 135, but is IL-17Ra-negative and negative for at least one B-cell marker and negative for at least one granulocyte/monocyte marker and is negative for at least one granulocyte marker.

In an embodiment, a bone marrow-derived myeloid progenitor cell (MPC) of the invention is expressing CD34 c-Kit (CD117) and CX3CR1, but is IL-17Ra-negative and negative for at least one B-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one B-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one B-cell marker and negative for at least one granulocyte/monocyte marker and is negative for at least one granulocyte marker.

In an embodiment, a bone marrow-derived myeloid progenitor cell (MPC) of the invention is expressing CD34 and c-Kit (CD117), but is negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117) and CD 115, but is negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD 117) and CD135, but is negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD 117), CD 115 and CD 135, but is negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34 c-Kit (CD117) and CX3CR1, but is negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CX3CR1, but is negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD135 and CX3CR1, but is negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is negative for at least one T-cell marker and negative for at least one granulocyte/monocyte marker and is negative for at least one granulocyte marker.

In an embodiment a bone marrow-derived myeloid progenitor cell (MPC) of the invention is expressing CD34 and c-Kit (CD117), but is IL-17Ra-negative and negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117) and CD115, but is IL-17Ra-negative and negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD 117) and CD135, but is IL-17Ra-negative and negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CD135, but is IL-17Ra-negative and negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34 c-Kit (CD117) and CX3CR1, but is IL-17Ra-negative and negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34 and c-Kit (CD117), but is negative for at least one B-cell marker and negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD 117) and CD 115, but is negative for at least one B-cell marker and negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD 117) and CD135, but is negative for at least one B-cell marker and negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CD135, but is negative for at least one B-cell marker and negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34 c-Kit (CD117) and CX3CR1, but is negative for at least one B-cell marker and negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CX3CR1, but is negative for at least one B-cell marker and negative for at least one T-cell marker and is negative for at least one granulocyte marker.

In an embodiment, a bone marrow-derived myeloid progenitor cell (MPC) of the invention is expressing CD34, c-Kit (CD117), CD135 and CX3CR1, but is negative for at least one B-cell marker and negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is negative for at least one B-cell marker and negative for at least one T-cell marker and negative for at least one granulocyte/monocyte marker and is negative for at least one granulocyte marker.

In an embodiment a bone marrow-derived myeloid progenitor cell (MPC) of the invention is expressing CD34 and c-Kit (CD117), but is IL-17Ra-negative and negative for at least one B-cell marker and negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD 117) and CD 115, but is IL-17Ra-negative and negative for at least one B-cell marker and negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117) and CD135, but is IL-17Ra-negative and negative for at least one B-cell marker and negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CD135, but is IL-17Ra-negative and negative for at least one B-cell marker and negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34 c-Kit (CD117) and CX3CR1, but is IL-17Ra-negative and negative for at least one B-cell marker and negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker and negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one B-cell marker and negative for at least one T-cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one B-cell marker and negative for at least one T-cell marker and negative for at least one granulocyte/monocyte marker and is negative for at least one granulocyte marker.

In an embodiment a bone marrow-derived myeloid progenitor cell (MPC) of the invention is expressing CD34 and c-Kit (CD117), but is negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117) and CD115, but is negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117) and CD 135, but is negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CD135, but is negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34 c-Kit (CD117) and CX3CR1, but is negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CX3CR1, but is negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD135 and CX3CR1, but is negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD 117), CD 115, CD135 and CX3CR1, but is negative for at least one mature myeloid cell marker and negative for at least one granulocyte/monocyte marker and is negative for at least one granulocyte marker.

In an embodiment a bone marrow-derived myeloid progenitor cell (MPC) of the invention is expressing CD34 and c-Kit (CD117), but is IL-17Ra-negative and is negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD 117) and CD 115, but is IL-17Ra-negative and is negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117) and CD135, but is IL-17Ra-negative and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CD135, but is IL-17Ra-negative and is negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34 c-Kit (CD117) and CX3CR1, but is IL-17Ra-negative and is negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD 117), CD135 and CX3CR1, but is IL-17Ra-negative and is negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34 and c-Kit (CD117), but is negative for at least one B-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD 117) and CD 115, but is negative for at least one B-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD 117) and CD 135, but is negative for at least one B-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD 115 and CD 135, but is negative for at least one B-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34 c-Kit (CD117) and CX3CR1, but is negative for at least one B-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CX3CR1, but is negative for at least one B-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD135 and CX3CR1, but is negative for at least one B-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is negative for at least one B-cell marker and negative for at least one mature myeloid cell marker and negative for at least one granulocyte/monocyte marker and is negative for at least one granulocyte marker.

In an embodiment a bone marrow-derived myeloid progenitor cell (MPC) of the invention is expressing CD34 and c-Kit (CD117), but is IL-17Ra-negative and negative for at least one B-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117) and CD115, but is IL-17Ra-negative and negative for at least one B-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117) and CD135, but is IL-17Ra-negative and negative for at least one B-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CD135, but is IL-17Ra-negative and negative for at least one B-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34 c-Kit (CD117) and CX3CR1, but is IL-17Ra-negative and negative for at least one B-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD 135 and CX3CR1, but is IL-17Ra-negative and negative for at least one B-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one B-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34 and c-Kit (CD117), but is negative for at least one T-cell marker and at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117) and CD115, but is negative for at least one T-cell marker and at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117) and CD 135, but is negative for at least one T-cell marker and at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD 117), CD115 and CD135, but is negative for at least one T-cell marker and at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34 c-Kit (CD117) and CX3CR1, but is negative for at least one T-cell marker and at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CX3CR1, but is negative for at least one T-cell marker and at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD135 and CX3CR1, but is negative for at least one T-cell marker and at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD 115, CD 135 and CX3CR1, but is negative for at least one T-cell marker and at least one mature myeloid cell marker and negative for at least one granulocyte/monocyte marker and is negative for at least one granulocyte marker.

In an embodiment a bone marrow-derived myeloid progenitor cell (MPC) of the invention is expressing CD34 and c-Kit (CD117), but is IL-17Ra-negative and negative for at least one T-cell marker and at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117) and CD115, but is IL-17Ra-negative and negative for at least one T-cell marker and at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117) and CD135, but is IL-17Ra-negative and negative for at least one T-cell marker and at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD 117), CD115 and CD135, but is IL-17Ra-negative and negative for at least one T-cell marker and at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34 c-Kit (CD117) and CX3CR1, but is IL-17Ra-negative and negative for at least one T-cell marker and at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one T-cell marker and at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one T-cell marker and at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one T-cell marker and at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34 and c-Kit (CD117), but is negative for at least one B-cell marker and negative for at least one T-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117) and CD115, but is negative for at least one B-cell marker and negative for at least one T-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117) and CD135, but is negative for at least one B-cell marker and negative for at least one T-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CD 135, but is negative for at least one B-cell marker and negative for at least one T-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34 c-Kit (CD117) and CX3CR1, but is negative for at least one B-cell marker and negative for at least one T-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CX3CR1, but is negative for at least one B-cell marker and negative for at least one T-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD135 and CX3CR1, but is negative for at least one B-cell marker and negative for at least one T-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is negative for at least one B-cell marker and negative for at least one T-cell marker and negative for at least one mature myeloid cell marker and negative for at least one granulocyte/monocyte marker and is negative for at least one granulocyte marker.

In an embodiment a bone marrow-derived myeloid progenitor cell (MPC) of the invention is expressing CD34 and c-Kit (CD117), but is IL-17Ra-negative and negative for at least one B-cell marker and negative for at least one T-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD 117) and CD 115, but is IL-17Ra-negative and negative for at least one B-cell marker and negative for at least one T-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117) and CD135, but is IL-17Ra-negative and negative for at least one B-cell marker and negative for at least one T-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD 117), CD 115 and CD 135, but is IL-17Ra-negative and negative for at least one B-cell marker and negative for at least one T-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34 c-Kit (CD117) and CX3CR1, but is IL-17Ra-negative and negative for at least one B-cell marker and negative for at least one T-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker and negative for at least one T-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one B-cell marker and negative for at least one T-cell marker and negative for at least one mature myeloid cell marker and negative for at least one granulocyte/monocyte marker and is negative for at least one granulocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one B-cell marker and negative for at least one T-cell marker and negative for at least one mature myeloid cell marker and negative for at least one granulocyte/monocyte marker.

In an embodiment a bone marrow-derived myeloid progenitor cell (MPC) of the invention is expressing CD34 and c-Kit (CD 117), but is negative for at least one erythrocyte marker.

In an embodiment, a bone marrow-derived myeloid progenitor cell (MPC) of the invention is expressing CD34, c-Kit (CD117) and CD115, but is negative for at least one erythrocyte marker.

In an embodiment, a bone marrow-derived myeloid progenitor cell (MPC) of the invention is expressing CD34, c-Kit (CD 117) and CD 135, but is negative for at least one erythrocyte marker.

In an embodiment, a bone marrow-derived myeloid progenitor cell (MPC) of the invention is expressing CD34, c-Kit (CD117), CD115 and CD135, but is negative for at least one erythrocyte marker.

In an embodiment, a bone marrow-derived myeloid progenitor cell (MPC) of the invention is expressing CD34 c-Kit (CD117) and CX3CR1, but is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one erythrocyte marker.

In an embodiment, a bone marrow-derived myeloid progenitor cell (MPC) of the invention is expressing CD34, c-Kit (CD117), CD 115, CD135 and CX3CR1, but is negative for at least one erythrocyte marker.

In an embodiment a bone marrow-derived myeloid progenitor cell (MPC) of the invention is expressing CD34 and c-Kit (CD117), but is IL-17Ra-negative and is negative for at least one erythrocyte marker.

In an embodiment, a bone marrow-derived myeloid progenitor cell (MPC) of the invention is expressing CD34, c-Kit (CD 117) and CD 115, but is IL-17Ra-negative and is negative for at least one erythrocyte marker.

In an embodiment, a bone marrow-derived myeloid progenitor cell (MPC) of the invention is expressing CD34, c-Kit (CD117) and CD135, but is IL-17Ra-negative and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one erythrocyte marker.

In an embodiment, a bone marrow-derived myeloid progenitor cell (MPC) of the invention is expressing CD34 and c-Kit (CD 117), but is negative for at least one B-cell marker and is negative for at least one erythrocyte marker.

In an embodiment, a bone marrow-derived myeloid progenitor cell (MPC) of the invention is expressing CD34, c-Kit (CD117) and CD 115, but is negative for at least one B-cell marker and is negative for at least one erythrocyte marker.

In an embodiment, a bone marrow-derived myeloid progenitor cell (MPC) of the invention is expressing CD34, c-Kit (CD117) and CD135, but is negative for at least one B-cell marker and is negative for at least one erythrocyte marker.

In an embodiment, a bone marrow-derived myeloid progenitor cell (MPC) of the invention is expressing CD34, c-Kit (CD 117), CD 115 and CD135, but is negative for at least one B-cell marker and is negative for at least one erythrocyte marker.

In an embodiment, a bone marrow-derived myeloid progenitor cell (MPC) of the invention is expressing CD34 c-Kit (CD117) and CX3CR1, but is negative for at least one B-cell marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one B-cell marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one B-cell marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is negative for at least one B-cell marker and is negative for at least one erythrocyte marker.

In an embodiment a bone marrow-derived myeloid progenitor cell (MPC) of the invention is expressing CD34 and c-Kit (CD117), but is IL-17Ra-negative and negative for at least one B-cell marker and is negative for at least one erythrocyte marker.

In an embodiment, a bone marrow-derived myeloid progenitor cell (MPC) of the invention is expressing CD34, c-Kit (CD 117) and CD 115, but is IL-17Ra-negative and negative for at least one B-cell marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and negative for at least one B-cell marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and negative for at least one B-cell marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and negative for at least one B-cell marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one B-cell marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one B-cell marker and is negative for at least one erythrocyte marker.

In an embodiment, a bone marrow-derived myeloid progenitor cell (MPC) of the invention is expressing CD34 and c-Kit (CD117), but is negative for at least one T-cell marker and is negative for at least one erythrocyte marker.

In an embodiment, a bone marrow-derived myeloid progenitor cell (MPC) of the invention is expressing CD34, c-Kit (CD117) and CD 115, but is negative for at least one T-cell marker and is negative for at least one erythrocyte marker.

In an embodiment, a bone marrow-derived myeloid progenitor cell (MPC) of the invention is expressing CD34, c-Kit (CD117) and CD135, but is negative for at least one T-cell marker and is negative for at least one erythrocyte marker.

In an embodiment, a bone marrow-derived myeloid progenitor cell (MPC) of the invention is expressing CD34, c-Kit (CD 117), CD115 and CD 135, but is negative for at least one T-cell marker and is negative for at least one erythrocyte marker.

In an embodiment, a bone marrow-derived myeloid progenitor cell (MPC) of the invention is expressing CD34 c-Kit (CD117) and CX3CR1, but is negative for at least one T-cell marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one T-cell marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one T-cell marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is negative for at least one T-cell marker and is negative for at least one erythrocyte marker.

In an embodiment a bone marrow-derived myeloid progenitor cell (MPC) of the invention is expressing CD34 and c-Kit (CD117), but is IL-17Ra-negative and negative for at least one T-cell marker and is negative for at least one erythrocyte marker.

In an embodiment, a bone marrow-derived myeloid progenitor cell (MPC) of the invention is expressing CD34, c-Kit (CD117) and CD 115, but is IL-17Ra-negative and negative for at least one T-cell marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and negative for at least one T-cell marker and is negative for at least one erythrocyte marker.

In an embodiment, a bone marrow-derived myeloid progenitor cell (MPC) of the invention is expressing CD34, c-Kit (CD 117), CD115 and CD135, but is IL-17Ra-negative and negative for at least one T-cell marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and negative for at least one T-cell marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one T-cell marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one T-cell marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one T-cell marker and is negative for at least one erythrocyte marker.

In an embodiment, a bone marrow-derived myeloid progenitor cell (MPC) of the invention is expressing CD34 and c-Kit (CD117), but is negative for at least one B-cell marker and negative for at least one T-cell marker and is negative for at least one erythrocyte marker.

In an embodiment, a bone marrow-derived myeloid progenitor cell (MPC) of the invention is expressing CD34, c-Kit (CD117) and CD115, but is negative for at least one B-cell marker and negative for at least one T-cell marker and is negative for at least one erythrocyte marker.

In an embodiment, a bone marrow-derived myeloid progenitor cell (MPC) of the invention is expressing CD34, c-Kit (CD117) and CD 135, but is negative for at least one B-cell marker and negative for at least one T-cell marker and is negative for at least one erythrocyte marker.

In an embodiment, a bone marrow-derived myeloid progenitor cell (MPC) of the invention is expressing CD34, c-Kit (CD117), CD 115 and CD 13 5, but is negative for at least one B-cell marker and negative for at least one T-cell marker and is negative for at least one erythrocyte marker.

In an embodiment, a bone marrow-derived myeloid progenitor cell (MPC) of the invention is expressing CD34 c-Kit (CD117) and CX3CR1, but is negative for at least one B-cell marker and negative for at least one T-cell marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one B-cell marker and negative for at least one T-cell marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one B-cell marker and negative for at least one T-cell marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is negative for at least one B-cell marker and negative for at least one T-cell marker and is negative for at least one erythrocyte marker.

In an embodiment a bone marrow-derived myeloid progenitor cell (MPC) of the invention is expressing CD34 and c-Kit (CD117), but is IL-17Ra-negative and negative for at least one B-cell marker and negative for at least one T-cell marker and is negative for at least one erythrocyte marker.

In an embodiment, a bone marrow-derived myeloid progenitor cell (MPC) of the invention is expressing CD34, c-Kit (CD 117) and CD 115, but is IL-17Ra-negative and negative for at least one B-cell marker and negative for at least one T-cell marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and negative for at least one B-cell marker and negative for at least one T-cell marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and negative for at least one B-cell marker and negative for at least one T-cell marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and negative for at least one B-cell marker and negative for at least one T-cell marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker and negative for at least one T-cell marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one B-cell marker and negative for at least one T-cell marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one B-cell marker and negative for at least one T-cell marker and is negative for at least one erythrocyte marker.

In an embodiment a bone marrow-derived myeloid progenitor cell (MPC) of the invention is expressing CD34 and c-Kit (CD117), but is negative for at least one mature myeloid cell marker and is negative for at least one erythrocyte marker.

In an embodiment, a bone marrow-derived myeloid progenitor cell (MPC) of the invention is expressing CD34, c-Kit (CD117) and CD115, but is negative for at least one mature myeloid cell marker and is negative for at least one erythrocyte marker.

In an embodiment, a bone marrow-derived myeloid progenitor cell (MPC) of the invention is expressing CD34, c-Kit (CD 117) and CD 135, but is negative for at least one mature myeloid cell marker and is negative for at least one erythrocyte marker.

In an embodiment, a bone marrow-derived myeloid progenitor cell (MPC) of the invention is expressing CD34, c-Kit (CD117), CD115 and CD135, but is negative for at least one mature myeloid cell marker and is negative for at least one erythrocyte marker.

In an embodiment, a bone marrow-derived myeloid progenitor cell (MPC) of the invention is expressing CD34 c-Kit (CD117) and CX3CR1, but is negative for at least one mature myeloid cell marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one mature myeloid cell marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one mature myeloid cell marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is negative for at least one mature myeloid cell marker and is negative for at least one erythrocyte marker.

In an embodiment a bone marrow-derived myeloid progenitor cell (MPC) of the invention is expressing CD34 and c-Kit (CD117), but is IL-17Ra-negative and is negative for at least one mature myeloid cell marker and is negative for at least one erythrocyte marker.

In an embodiment, a bone marrow-derived myeloid progenitor cell (MPC) of the invention is expressing CD34, c-Kit (CD 117) and CD 115, but is IL-17Ra-negative and is negative for at least one mature myeloid cell marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and is negative for at least one mature myeloid cell marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and is negative for at least one mature myeloid cell marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one mature myeloid cell marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one mature myeloid cell marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one mature myeloid cell marker and is negative for at least one erythrocyte marker.

In an embodiment, a bone marrow-derived myeloid progenitor cell (MPC) of the invention is expressing CD34 and c-Kit (CD117), but is negative for at least one B-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD 117) and CD 115, but is negative for at least one B-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD117) and CD135, but is negative for at least one B-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD 117), CD 115 and CD135, but is negative for at least one B-cell marker and negative for at least one mature myeloid cell marker and is negative for at least one erythrocyte marker.

In an embodiment, a bone marrow-derived myeloid progenitor cell (MPC) of the invention is expressing CD34 c-Kit (CD117) and CX3CR1, but is negative for at least one B-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one B-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one B-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is negative for at least one B-cell marker and negative for at least one mature myeloid cell marker and is negative for at least one erythrocyte marker.

In an embodiment a bone marrow-derived myeloid progenitor cell (MPC) of the invention is expressing CD34 and c-Kit (CD117), but is IL-17Ra-negative and negative for at least one B-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD 117) and CD 115, but is IL-17Ra-negative and negative for at least one B-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and negative for at least one B-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and negative for at least one B-cell marker and negative for at least one mature myeloid cell marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and negative for at least one B-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one B-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one B-cell marker and negative for at least one mature myeloid cell marker and is negative for at least one erythrocyte marker.

In an embodiment, a bone marrow-derived myeloid progenitor cell (MPC) of the invention is expressing CD34 and c-Kit (CD117), but is negative for at least one T-cell marker and at least one mature myeloid cell marker and is negative for at least one erythrocyte marker.

In an embodiment, a bone marrow-derived myeloid progenitor cell (MPC) of the invention is expressing CD34, c-Kit (CD117) and CD115, but is negative for at least one T-cell marker and at least one mature myeloid cell marker and is negative for at least one erythrocyte marker.

In an embodiment, a bone marrow-derived myeloid progenitor cell (MPC) of the invention is expressing CD34, c-Kit (CD117) and CD135, but is negative for at least one T-cell marker and at least one mature myeloid cell marker and is negative for at least one erythrocyte marker.

In an embodiment, a bone marrow-derived myeloid progenitor cell (MPC) of the invention is expressing CD34, c-Kit (CD 117), CD 115 and CD135, but is negative for at least one T-cell marker and at least one mature myeloid cell marker and is negative for at least one erythrocyte marker.

In an embodiment, a bone marrow-derived myeloid progenitor cell (MPC) of the invention is expressing CD34 c-Kit (CD117) and CX3CR1, but is negative for at least one T-cell marker and at least one mature myeloid cell marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one T-cell marker and at least one mature myeloid cell marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one T-cell marker and at least one mature myeloid cell marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is negative for at least one T-cell marker and at least one mature myeloid cell marker and is negative for at least one erythrocyte marker.

In an embodiment a bone marrow-derived myeloid progenitor cell (MPC) of the invention is expressing CD34 and c-Kit (CD117), but is IL-17Ra-negative and negative for at least one T-cell marker and at least one mature myeloid cell marker and is negative for at least one erythrocyte marker.

In an embodiment, a bone marrow-derived myeloid progenitor cell (MPC) of the invention is expressing CD34, c-Kit (CD 117) and CD 115, but is IL-17Ra-negative and negative for at least one T-cell marker and at least one mature myeloid cell marker and is negative for at least one erythrocyte marker.

In an embodiment, a bone marrow-derived myeloid progenitor cell (MPC) of the invention is expressing CD34, c-Kit (CD 117) and CD135, but is IL-17Ra-negative and negative for at least one T-cell marker and at least one mature myeloid cell marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and negative for at least one T-cell marker and at least one mature myeloid cell marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and negative for at least one T-cell marker and at least one mature myeloid cell marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one T-cell marker and at least one mature myeloid cell marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one T-cell marker and at least one mature myeloid cell marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one T-cell marker and at least one mature myeloid cell marker and is negative for at least one erythrocyte marker.

In an embodiment, a bone marrow-derived myeloid progenitor cell (MPC) of the invention is expressing CD34 and c-Kit (CD117), but is negative for at least one B-cell marker and negative for at least one T-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD 117) and CD 115, but is negative for at least one B-cell marker and negative for at least one T-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD117) and CD135, but is negative for at least one B-cell marker and negative for at least one T-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD117), CD115 and CD135, but is negative for at least one B-cell marker and negative for at least one T-cell marker and negative for at least one mature myeloid cell marker and is negative for at least one erythrocyte marker.

In an embodiment, a bone marrow-derived myeloid progenitor cell (MPC) of the invention is expressing CD34 c-Kit (CD117) and CX3CR1, but is negative for at least one B-cell marker and negative for at least one T-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one B-cell marker and negative for at least one T-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one B-cell marker and negative for at least one T-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD117), CD 115, CD135 and CX3CR1, but is negative for at least one B-cell marker and negative for at least one T-cell marker and negative for at least one mature myeloid cell marker and is negative for at least one erythrocyte marker.

In an embodiment a bone marrow-derived myeloid progenitor cell (MPC) of the invention is expressing CD34 and c-Kit (CD117), but is IL-17Ra-negative and negative for at least one B-cell marker and negative for at least one T-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD 117) and CD 115, but is IL-17Ra-negative and negative for at least one B-cell marker and negative for at least one T-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and negative for at least one B-cell marker and negative for at least one T-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD117), CD 115 and CD 135, but is IL-17Ra-negative and negative for at least one B-cell marker and negative for at least one T-cell marker and negative for at least one mature myeloid cell marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and negative for at least one B-cell marker and negative for at least one T-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker and negative for at least one T-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one B-cell marker and negative for at least one T-cell marker and negative for at least one mature myeloid cell marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one B-cell marker and negative for at least one T-cell marker and negative for at least one mature myeloid cell marker and is negative for at least one erythrocyte marker.

In an embodiment a bone marrow-derived myeloid progenitor cell (MPC) of the invention is expressing CD34 and c-Kit (CD117), but is negative for at least one granulocyte/monocyte marker and is negative for at least one erythrocyte marker.

In an embodiment, a bone marrow-derived myeloid progenitor cell (MPC) of the invention is expressing CD34, c-Kit (CD 117) and CD 115, but is negative for at least one granulocyte/monocyte marker and is negative for at least one erythrocyte marker.

In an embodiment, a bone marrow-derived myeloid progenitor cell (MPC) of the invention is expressing CD34, c-Kit (CD 117) and CD135, but is negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CD135, but is negative for at least one granulocyte/monocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is negative for at least one granulocyte/monocyte marker and is negative for at least one erythrocyte marker.

In an embodiment a bone marrow-derived myeloid progenitor cell (MPC) of the invention is expressing CD34 and c-Kit (CD117), but is IL-17Ra-negative and is negative for at least one granulocyte/monocyte marker and is negative for at least one erythrocyte marker.

In an embodiment, a bone marrow-derived myeloid progenitor cell (MPC) of the invention is expressing CD34, c-Kit (CD 117) and CD 115, but is IL-17Ra-negative and is negative for at least one granulocyte/monocyte marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and is negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and is negative for at least one granulocyte/monocyte marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and is negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one granulocyte/monocyte marker and is negative for at least one erythrocyte marker.

In an embodiment, a bone marrow-derived myeloid progenitor cell (MPC) of the invention is expressing CD34 and c-Kit (CD117), but is negative for at least one B-cell marker and is negative for at least one granulocyte/monocyte marker and is negative for at least one erythrocyte marker.

In an embodiment, a bone marrow-derived myeloid progenitor cell (MPC) of the invention is expressing CD34, c-Kit (CD 117) and CD 115, but is negative for at least one B-cell marker and is negative for at least one granulocyte/monocyte marker and is negative for at least one erythrocyte marker.

In an embodiment, a bone marrow-derived myeloid progenitor cell (MPC) of the invention is expressing CD34, c-Kit (CD117) and CD135, but is negative for at least one B-cell marker and is negative for at least one granulocyte/monocyte marker and is negative for at least one erythrocyte marker.

In an embodiment, a bone marrow-derived myeloid progenitor cell (MPC) of the invention is expressing CD34, c-Kit (CD 117), CD 115 and CD 135, but is negative for at least one B-cell marker and is negative for at least one granulocyte/monocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one B-cell marker and is negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one B-cell marker and is negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one B-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD 117), CD115, CD135 and CX3CR1, but is negative for at least one B-cell marker and negative for at least one granulocyte/monocyte marker and is negative for at least one erythrocyte marker.

In an embodiment a bone marrow-derived myeloid progenitor cell (MPC) of the invention is expressing CD34 and c-Kit (CD117), but is IL-17Ra-negative and negative for at least one B-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD 117) and CD 115, but is IL-17Ra-negative and negative for at least one B-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and negative for at least one B-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD 117), CD115 and CD135, but is IL-17Ra-negative and negative for at least one B-cell marker and negative for at least one granulocyte/monocyte marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and negative for at least one B-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one B-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one B-cell marker and negative for at least one granulocyte/monocyte marker and is negative for at least one erythrocyte marker.

In an embodiment, a bone marrow-derived myeloid progenitor cell (MPC) of the invention is expressing CD34 and c-Kit (CD117), but is negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117) and CD115, but is negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117) and CD 135, but is negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CD135, but is negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is negative for at least one T-cell marker and negative for at least one granulocyte/monocyte marker and is negative for at least one erythrocyte marker.

In an embodiment a bone marrow-derived myeloid progenitor cell (MPC) of the invention is expressing CD34 and c-Kit (CD117), but is IL-17Ra-negative and negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117) and CD115, but is IL-17Ra-negative and negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD 135 and CX3CR1, but is IL-17Ra-negative and negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34 and c-Kit (CD117), but is negative for at least one B-cell marker and negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117) and CD115, but is negative for at least one B-cell marker and negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD 117) and CD135, but is negative for at least one B-cell marker and negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CD135, but is negative for at least one B-cell marker and negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one B-cell marker and negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one B-cell marker and negative for at least one T-cell marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one B-cell marker and negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is negative for at least one B-cell marker and negative for at least one T-cell marker and negative for at least one granulocyte/monocyte marker and is negative for at least one erythrocyte marker.

In an embodiment a bone marrow-derived myeloid progenitor cell (MPC) of the invention is expressing CD34 and c-Kit (CD117), but is IL-17Ra-negative and negative for at least one B-cell marker and negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117) and CD115, but is IL-17Ra-negative and negative for at least one B-cell marker and negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and negative for at least one B-cell marker and negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and negative for at least one B-cell marker and negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and negative for at least one B-cell marker and negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker and negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one B-cell marker and negative for at least one T-cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one B-cell marker and negative for at least one T-cell marker and negative for at least one granulocyte/monocyte marker and is negative for at least one erythrocyte marker.

In an embodiment a bone marrow-derived myeloid progenitor cell (MPC) of the invention is expressing CD34 and c-Kit (CD117), but is negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117) and CD115, but is negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117) and CD135, but is negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CD135, but is negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one mature myeloid cell marker and negative for at least one granulocyte/monocyte marker.

In an embodiment, a bone marrow-derived myeloid progenitor cell (MPC) of the invention is expressing CD34, c-Kit (CD117), CD115 and CX3CR1, but is negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is negative for at least one mature myeloid cell marker and negative for at least one granulocyte/monocyte marker and is negative for at least one erythrocyte marker.

In an embodiment a bone marrow-derived myeloid progenitor cell (MPC) of the invention is expressing CD34 and c-Kit (CD117), but is IL-17Ra-negative and is negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD 117) and CD 115, but is IL-17Ra-negative and is negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and is negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and is negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34 and c-Kit (CD117), but is negative for at least one B-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD 117) and CD 115, but is negative for at least one B-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117) and CD135, but is negative for at least one B-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CD135, but is negative for at least one B-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one B-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one B-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one B-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD 117), CD115, CD 135 and CX3CR1, but is negative for at least one B-cell marker and negative for at least one mature myeloid cell marker and negative for at least one granulocyte/monocyte marker and is negative for at least one erythrocyte marker.

In an embodiment a bone marrow-derived myeloid progenitor cell (MPC) of the invention is expressing CD34 and c-Kit (CD117), but is IL-17Ra-negative and negative for at least one B-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD 117) and CD 115, but is IL-17Ra-negative and negative for at least one B-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and negative for at least one B-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and negative for at least one B-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34 c-Kit (CD 117) and CX3CR1, but is IL-17Ra-negative and negative for at least one B-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one B-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one B-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34 and c-Kit (CD 117), but is negative for at least one T-cell marker and at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD 117) and CD 115, but is negative for at least one T-cell marker and at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD 117) and CD 135, but is negative for at least one T-cell marker and at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CD135, but is negative for at least one T-cell marker and at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one T-cell marker and at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one T-cell marker and at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one T-cell marker and at least one mature myeloid cell marker. and negative for at least one granulocyte/monocyte marker and is negative for at least one erythrocyte marker

In an embodiment, a bone marrow-derived myeloid progenitor cell (MPC) of the invention is expressing CD34, c-Kit (CD117), CD115, CD135 and CX3CR1, but is negative for at least one T-cell marker and at least one mature myeloid cell marker and negative for at least one granulocyte/monocyte marker and is negative for at least one erythrocyte marker.

In an embodiment a bone marrow-derived myeloid progenitor cell (MPC) of the invention is expressing CD34 and c-Kit (CD117), but is IL-17Ra-negative and negative for at least one T-cell marker and at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117) and CD 115, but is IL-17Ra-negative and negative for at least one T-cell marker and at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and negative for at least one T-cell marker and at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and negative for at least one T-cell marker and at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and negative for at least one T-cell marker and at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one T-cell marker and at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one T-cell marker and at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one T-cell marker and at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34 and c-Kit (CD117), but is negative for at least one B-cell marker and negative for at least one T-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD 117) and CD 115, but is negative for at least one B-cell marker and negative for at least one T-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117) and CD135, but is negative for at least one B-cell marker and negative for at least one T-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CD135, but is negative for at least one B-cell marker and negative for at least one T-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one B-cell marker and negative for at least one T-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one B-cell marker and negative for at least one T-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one B-cell marker and negative for at least one T-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is negative for at least one B-cell marker and negative for at least one T-cell marker and negative for at least one mature myeloid cell marker and negative for at least one granulocyte/monocyte marker and is negative for at least one erythrocyte marker.

In an embodiment a bone marrow-derived myeloid progenitor cell (MPC) of the invention is expressing CD34 and c-Kit (CD117), but is IL-17Ra-negative and negative for at least one B-cell marker and negative for at least one T-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117) and CD115, but is IL-17Ra-negative and negative for at least one B-cell marker and negative for at least one T-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and negative for at least one B-cell marker and negative for at least one T-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and negative for at least one B-cell marker and negative for at least one T-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and negative for at least one B-cell marker and negative for at least one T-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and negative for at least one B-cell marker and negative for at least one T-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and negative for at least one B-cell marker and negative for at least one T-cell marker and negative for at least one mature myeloid cell marker and negative for at least one granulocyte/monocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and negative for at least one B-cell marker and negative for at least one T-cell marker and negative for at least one mature myeloid cell marker and negative for at least one granulocyte/monocyte marker and is negative for at least one erythrocyte marker.

In an embodiment a bone marrow-derived myeloid progenitor cell (MPC) of the invention is expressing CD34 and c-Kit (CD 117), but is negative for at least one granulocyte marker and is negative for at least one erythrocyte marker.

In an embodiment, a bone marrow-derived myeloid progenitor cell (MPC) of the invention is expressing CD34, c-Kit (CD 117) and CD 115, but is negative for at least one granulocyte marker and is negative for at least one erythrocyte marker.

In an embodiment, a bone marrow-derived myeloid progenitor cell (MPC) of the invention is expressing CD34, c-Kit (CD117) and CD135, but is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is negative for at least one granulocyte marker and is negative for at least one erythrocyte marker.

In an embodiment a bone marrow-derived myeloid progenitor cell (MPC) of the invention is expressing CD34 and c-Kit (CD117), but is IL-17Ra-negative and is negative for at least one granulocyte marker and is negative for at least one erythrocyte marker.

In an embodiment, a bone marrow-derived myeloid progenitor cell (MPC) of the invention is expressing CD34, c-Kit (CD117) and CD 115, but is IL-17Ra-negative and is negative for at least one granulocyte marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one granulocyte marker and is negative for at least one erythrocyte marker.

In an embodiment, a bone marrow-derived myeloid progenitor cell (MPC) of the invention is expressing CD34 and c-Kit (CD117), but is negative for at least one B-cell marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD 115, but is negative for at least one B-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD 135, but is negative for at least one B-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is negative for at least one B-cell marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one B-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one B-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one B-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD 115, CD 135 and CX3CR1, but is negative for at least one B-cell marker and is negative for at least one granulocyte marker and is negative for at least one erythrocyte marker.

In an embodiment a bone marrow-derived myeloid progenitor cell (MPC) of the invention is expressing CD34 and c-Kit (CD117), but is IL-17Ra-negative and is negative for at least one B-cell marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD 115, but is IL-17Ra-negative and is negative for at least one B-cell marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD135, but is IL-17Ra-negative and is negative for at least one B-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and is negative for at least one B-cell marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and is negative for at least one B-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one B-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one B-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one B-cell marker and is negative for at least one granulocyte marker and is negative for at least one erythrocyte marker.

In an embodiment, a bone marrow-derived myeloid progenitor cell (MPC) of the invention is expressing CD34 and c-Kit (CD117), but is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD 115, but is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD135, but is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is negative for at least one T-cell marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is negative for at least one T-cell marker and is negative for at least one granulocyte marker and is negative for at least one erythrocyte marker.

In an embodiment a bone marrow-derived myeloid progenitor cell (MPC) of the invention is expressing CD34 and c-Kit (CD117), but is IL-17Ra-negative and is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD 115, but is IL-17Ra-negative and is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and is negative for at least one T-cell marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one T-cell marker and is negative for at least one granulocyte marker and is negative for at least one erythrocyte marker.

In an embodiment, a bone marrow-derived myeloid progenitor cell (MPC) of the invention is expressing CD34 and c-Kit (CD117), but is negative for at least one B-cell marker and is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD115, but is negative for at least one B-cell marker and is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD 135, but is negative for at least one B-cell marker and is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD 117), CD115 and CD135, but is negative for at least one B-cell marker and is negative for at least one T-cell marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one B-cell marker and is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one B-cell marker and is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one B-cell marker and is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is negative for at least one B-cell marker and is negative for at least one T-cell marker and is negative for at least one granulocyte marker and is negative for at least one erythrocyte marker.

In an embodiment a bone marrow-derived myeloid progenitor cell (MPC) of the invention is expressing CD34 and c-Kit (CD117), but is IL-17Ra-negative and is negative for at least one B-cell marker and is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD115, but is IL-17Ra-negative and is negative for at least one B-cell marker and is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD135, but is IL-17Ra-negative and is negative for at least one B-cell marker and is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and is negative for at least one B-cell marker and is negative for at least one T-cell marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and is negative for at least one B-cell marker and is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one B-cell marker and is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one B-cell marker and is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one B-cell marker and is negative for at least one T-cell marker and is negative for at least one granulocyte marker and is negative for at least one erythrocyte marker.

In an embodiment a bone marrow-derived myeloid progenitor cell (MPC) of the invention is expressing CD34 and c-Kit (CD117), but is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD115, but is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD135, but is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD 135, but is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is negative for at least one mature myeloid cell marker and is negative for at least one granulocyte marker and is negative for at least one erythrocyte marker.

In an embodiment a bone marrow-derived myeloid progenitor cell (MPC) of the invention is expressing CD34 and c-Kit (CD117), but is IL-17Ra-negative and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD115, but is IL-17Ra-negative and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD 135, but is IL-17Ra-negative and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34 and c-Kit (CD117), but is negative for at least one B-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD 115, but is negative for at least one B-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD 135, but is negative for at least one B-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD 117), CD 115 and CD 135, but is negative for at least one B-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one B-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one B-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one B-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is negative for at least one B-cell marker and is negative for at least one mature myeloid cell marker and is negative for at least one granulocyte marker and is negative for at least one erythrocyte marker.

In an embodiment a bone marrow-derived myeloid progenitor cell (MPC) of the invention is expressing CD34 and c-Kit (CD 117), but is IL-17Ra-negative and is negative for at least one B-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD 115, but is IL-17Ra-negative and is negative for at least one B-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and is negative for at least one B-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and is negative for at least one B-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and is negative for at least one B-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one B-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one B-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one B-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34 and c-Kit (CD117), but is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD115, but is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD 135, but is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD 117), CD115 and CD135, but is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one T-cell marker and at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is negative for at least one T-cell marker and is negative for at least one mature myeloid cell marker and is negative for at least one granulocyte marker and is negative for at least one erythrocyte marker.

In an embodiment a bone marrow-derived myeloid progenitor cell (MPC) of the invention is expressing CD34 and c-Kit (CD117), but is IL-17Ra-negative and is negative for negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD 115, but is IL-17Ra-negative and is negative for negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34 and c-Kit (CD117), but is negative for at least one B-cell marker and is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD115, but is negative for at least one B-cell marker and is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD 135, but is negative for at least one B-cell marker and is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is negative for at least one B-cell marker and is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one B-cell marker and is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one B-cell marker and is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one B-cell marker and is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is negative for at least one B-cell marker and is negative for at least one T-cell marker and is negative for at least one mature myeloid cell marker and is negative for at least one granulocyte marker and is negative for at least one erythrocyte marker.

In an embodiment a bone marrow-derived myeloid progenitor cell (MPC) of the invention is expressing CD34 and c-Kit (CD117), but is IL-17Ra-negative and is negative for at least one B-cell marker and is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD 115, but is IL-17Ra-negative and is negative for at least one B-cell marker and is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and is negative for at least one B-cell marker and is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and is negative for at least one B-cell marker and is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and is negative for at least one B-cell marker and is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one B-cell marker and is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one B-cell marker and is negative for at least one T-cell marker and is negative for at least one mature myeloid cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one B-cell marker and is negative for at least one T-cell marker and is negative for at least one mature myeloid cell marker and is negative for at least one granulocyte marker and is negative for at least one erythrocyte marker.

In an embodiment a bone marrow-derived myeloid progenitor cell (MPC) of the invention is expressing CD34 and c-Kit (CD 117), but is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD115, but is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD 135, but is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD 117), CD115 and CD 135, but is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is negative for at least one granulocyte/monocyte marker and is negative for at least one granulocyte marker and is negative for at least one erythrocyte marker.

In an embodiment a bone marrow-derived myeloid progenitor cell (MPC) of the invention is expressing CD34 and c-Kit (CD117), but is IL-17Ra-negative and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD 115, but is IL-17Ra-negative and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34 and c-Kit (CD 117), but is negative for at least one B-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD115, but is negative for at least one B-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD135, but is negative for at least one B-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is negative for at least one B-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one B-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one B-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one B-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD 115, CD135 and CX3CR1, but is negative for at least one B-cell marker and is negative for at least one granulocyte/monocyte marker and is negative for at least one granulocyte marker and is negative for at least one erythrocyte marker.

In an embodiment a bone marrow-derived myeloid progenitor cell (MPC) of the invention is expressing CD34 and c-Kit (CD 117), but is IL-17Ra-negative and is negative for at least one B-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD 115, but is IL-17Ra-negative and is negative for at least one B-cell marker and is negative for at least one granulocyte/monocyte marker and is negative for at least one granulocyte marker.

In an embodiment, a bone marrow-derived myeloid progenitor cell (MPC) of the invention is expressing CD34, c-Kit (CD 117) and CD135, but is IL-17Ra-negative and is negative for at least one B-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and is negative for at least one B-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and is negative for at least one B-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one B-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one B-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one B-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34 and c-Kit (CD117), but is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD 115, but is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD135, but is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD 115, CD135 and CX3CR1, but is negative for at least one T-cell marker and is negative for at least one granulocyte/monocyte marker and is negative for at least one granulocyte marker and is negative for at least one erythrocyte marker.

In an embodiment a bone marrow-derived myeloid progenitor cell (MPC) of the invention is expressing CD34 and c-Kit (CD117), but is IL-17Ra-negative and is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD 115, but is IL-17Ra-negative and is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34 and c-Kit (CD117), but is negative for at least one B-cell marker and is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD 115, but is negative for at least one B-cell marker and is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD135, but is negative for at least one B-cell marker and is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is negative for at least one B-cell marker and is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one B-cell marker and is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one B-cell marker and is negative for at least one T-cell marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one B-cell marker and is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is negative for at least one B-cell marker and is negative for at least one T-cell marker and is negative for at least one granulocyte/monocyte marker and is negative for at least one granulocyte marker and is negative for at least one erythrocyte marker.

In an embodiment a bone marrow-derived myeloid progenitor cell (MPC) of the invention is expressing CD34 and c-Kit (CD117), but is IL-17Ra-negative and is negative for at least one B-cell marker and is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD 115, but is IL-17Ra-negative and is negative for at least one B-cell marker and is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and is negative for at least one B-cell marker and is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD 117), CD 115 and CD 135, but is IL-17Ra-negative and is negative for at least one B-cell marker and is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and is negative for at least one B-cell marker and is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one B-cell marker and is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one B-cell marker and is negative for at least one T-cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one B-cell marker and is negative for at least one T-cell marker and is negative for at least one granulocyte/monocyte marker and is negative for at least one granulocyte marker and is negative for at least one erythrocyte marker.

In an embodiment a bone marrow-derived myeloid progenitor cell (MPC) of the invention is expressing CD34 and c-Kit (CD117), but is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD115, but is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD135, but is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is negative for at least one mature myeloid cell marker and is negative for at least one granulocyte/monocyte marker and is negative for at least one granulocyte marker and is negative for at least one erythrocyte marker.

In an embodiment a bone marrow-derived myeloid progenitor cell (MPC) of the invention is expressing CD34 and c-Kit (CD117), but is IL-17Ra-negative and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD115, but is IL-17Ra-negative and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD135, but is IL-17Ra-negative and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34 and c-Kit (CD117), but is negative for at least one B-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD115, but is negative for at least one B-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD 135, but is negative for at least one B-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD 117), CD115 and CD135, but is negative for at least one B-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one B-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one B-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one B-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is negative for at least one B-cell marker and is negative for at least one mature myeloid cell marker and is negative for at least one granulocyte/monocyte marker and is negative for at least one granulocyte marker and is negative for at least one erythrocyte marker.

In an embodiment a bone marrow-derived myeloid progenitor cell (MPC) of the invention is expressing CD34 and c-Kit (CD117), but is IL-17Ra-negative and is negative for at least one B-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD115, but is IL-17Ra-negative and is negative for at least one B-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD135, but is IL-17Ra-negative and is negative for at least one B-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and is negative for at least one B-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and is negative for at least one B-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one B-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one B-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one B-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34 and c-Kit (CD117), but is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD 115, but is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD 135, but is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment a bone marrow-derived myeloid progenitor cell (MPC) of the invention is expressing CD34 and c-Kit (CD117), but is IL-17Ra-negative and is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD115, but is IL-17Ra-negative and is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD135, but is IL-17Ra-negative and is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34 and c-Kit (CD117), but is negative for at least one B-cell marker and is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD115, but is negative for at least one B-cell marker and is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD 117) and CD135, but is negative for at least one B-cell marker and is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is negative for at least one B-cell marker and is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is negative for at least one B-cell marker and is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is negative for at least one B-cell marker and is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is negative for at least one B-cell marker and is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD 117), CD 115, CD 135 and CX3CR1, but is negative for at least one B-cell marker and is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment a bone marrow-derived myeloid progenitor cell (MPC) of the invention is expressing CD34 and c-Kit (CD117), but is IL-17Ra-negative and is negative for at least one B-cell marker and is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD115, but is IL-17Ra-negative and is negative for at least one B-cell marker and is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117) and CD 135, but is IL-17Ra-negative and is negative for at least one B-cell marker and is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CD135, but is IL-17Ra-negative and is negative for at least one B-cell marker and is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34 c-Kit (CD117) and CX3CR1, but is IL-17Ra-negative and is negative for at least one B-cell marker and is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115 and CX3CR1, but is IL-17Ra-negative and is negative for at least one B-cell marker and is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD135 and CX3CR1, but is IL-17Ra-negative and is negative for at least one B-cell marker and is negative for at least one T-cell marker and is negative for at least one mature myeloid cell marker and is negative for at least one granulocyte/monocyte marker and is negative for at least one granulocyte marker and is negative for at least one erythrocyte marker.

In an embodiment, a bone marrow-derived myeloid progenitor cell (MPC) of the invention is expressing CD34, c-Kit (CD117), CD115, CD135 and CX3CR1, but is IL-17Ra-negative and is negative for at least one B-cell marker and is negative for at least one T-cell marker and is negative for at least one mature myeloid cell marker and is negative for at least one granulocyte/monocyte marker and is negative for at least one erythrocyte marker.

In an embodiment of the invention and in particular of any of the above embodiments specifying distinct combinations of surface antigens, at least one B-cell marker is a membrane-bound B-cell marker. In a preferred embodiment thereof at least one B-cell marker is selected from the group comprising CD45R, CD 19, CD20, CD22 and B220.

In an embodiment of the invention and in particular of any of the above embodiments specifying distinct combinations of surface antigens, at least one T-cell marker is selected from the group comprising CD5, CD3, CD4 and CD8.

In an embodiment of the invention and in particular of any of the above embodiments specifying distinct combinations of surface antigens, at least one mature myeloid cell marker is selected from the group comprising CD11b, CD14, CD16 and 7-4.

In an embodiment of the invention and in particular of any of the above embodiments specifying distinct combinations of surface antigens, at least one granulocyte/monocyte marker is selected from the group comprising Gr-1 and Ly6-G and Ly6-C.

In an embodiment of the invention and in particular of any of the above embodiments specifying distinct combinations of surface antigens, at least one granulocyte marker is selected from the group comprising Ly6-G.

In an embodiment of the invention and in particular of any of the above embodiments specifying distinct combinations of surface antigens, at least one erythrocyte marker is selected from the group comprising Ter-119.

In an embodiment a bone marrow-derived myeloid progenitor cell (MPC) of the invention is a recombinant bone marrow-derived myeloid progenitor cell (MPC). In an embodiment of the present invention a recombinant bone marrow-derived myeloid progenitor cell (MPC) is a cell that has been subject to genetic engineering. In connection with such genetic engineering, the genome of the cell or the genetic information contained and/or expressed was changed compared to the wild type cell and/or compared to the genome of the cell prior to subjecting the same to genetic engineering. In an embodiment of the invention the genome of a recombinant comprises additional genetic information, less genetic information or different genetic information, in each case compared to the cell prior to subjecting the same to genetic engineering. In an embodiment a recombinant cell is one into which nucleic acid has been introduced which is as such is not present in the cell prior to subjecting the same to genetic engineering, or which as such is not expressed in the cell prior to subjecting the same to genetic engineering or which is present in the cell in a different regulatory context after subjecting the cell to genetic engineering. Such different regulatory context is, in an embodiment, realized by putting a nucleic acid which is transcribed and/or translated in the cell, under the control of a promoter which is different from the promoter which controls the expression in the cell prior to subjecting the same to genetic engineering. In a further embodiment such different regulatory context is realized by putting a nucleic acid which is transcribed and/or translated in the cell under the control of a regulatory RNA, such as an miRNA, which is different from the regulatory RNA that controls the expression in the cell prior to subjecting the same to genetic engineering.

In an embodiment a bone marrow-derived myeloid progenitor cell (MPC) of the invention comprises a heterologous nucleic acid. In an embodiment, a heterologous nucleic acid is a nucleic acid which is one is not present in and/or not expressed by the cell prior to subjecting the cell to genetic engineering. In a further embodiment, a heterologous nucleic acid is one which is present in the cell in a different regulatory context as illustrated above, compared to the regulatory context of such nucleic acid in the cell prior to subjecting the cell to genetic engineering.

In an embodiment the heterologous nucleic acid may code for a therapeutically active peptide, polypeptide or protein. In a preferred embodiment such therapeutically active peptide, polypeptide or protein is effective in the treatment of a disease in the treatment of which a bone marrow-derived of the invention is used. Preferably, the disease is one as disclosed herein.

In an embodiment the heterologous nucleic acid may code for a therapeutically active nucleic acid. In a preferred embodiment such therapeutically active nucleic acid is effective in the treatment of a disease in the treatment of which a bone marrow-derived myeloid progenitor cell (MPC) of the invention is used. Preferably, the disease is one as disclosed herein.

In a further embodiment, a bone marrow-derived myeloid progenitor cell (MPC) is labelled with a therapeutically active isotope. In preferred embodiment, the therapeutically active isotope is one selected from the group comprising ¹¹¹In, ¹⁷⁷Lu, ⁸⁹Zr, ⁶⁷Ga, ⁶⁸Ga, ⁶⁴Cu and ⁹⁰Y.

A pharmaceutical composition of the present invention comprises at least a bone marrow-derived myeloid progenitor cell (MPC) of the invention and, optionally, one or more carrier substances, excipients and/or adjuvants. The pharmaceutical composition may additionally comprise, for example, one or more of water, buffers such as, e.g., neutral buffered saline or phosphate buffered saline, ethanol, mineral oil, vegetable oil, dimethylsulfoxide, carbohydrates such as e.g., glucose, mannose, sucrose or dextrans, mannitol, proteins, adjuvants, polypeptides or amino acids such as glycine, antioxidants, chelating agents such as EDTA or glutathione and/or preservatives. Furthermore, one or more other active ingredients may, but need not, be included in the pharmaceutical composition of the invention.

The pharmaceutical composition of the invention may be formulated for any appropriate route of administration, including, for example, parenteral administration. The term parenteral as used herein includes subcutaneous, intradermal, intravascular such as, e.g., intravenous, intramuscular, intrathecal and intra-arterial injection, as well as any similar injection or infusion technique. A preferred route of administration is intravenous administration.

A bone marrow-derived myeloid progenitor cell (MPC) of the invention and/or a pharmaceutical composition of the invention may be used in the treatment of a disease and/or in the treatment of a subject suffering from or being at risk of suffering from a disease. In a preferred embodiment the disease is a neurodegenerative disease.

In an embodiment the method of treatment is an adoptive transfer of a bone marrow-derived myeloid progenitor cell (MPC) of the invention. Such transfer can be either an allogeneic adoptive transfer or an autologous adoptive transfer. In case of an allogenic adoptive transfer, prior to administering to a subject the bone marrow-derived myeloid progenitor cell (MPC) of the invention or a pharmaceutical composition of the invention, matching between the donor subject and the recipient subject will be required. Typically, such matching is performed on the basis of HLA and the matching is HLA matching.

It is within the present invention that in an embodiment the bone marrow-derived myeloid progenitor cell (MPC) subject to adoptive transfer is actually a recombinant bone marrow-derived myeloid progenitor cell (MPC) of the invention. In a preferred embodiment, the recombinant bone marrow-derived myeloid progenitor cell (MPC) is one where one or several genetic defects have been corrected, whereby, preferably, in such embodiment the adoptive transfer is an autologous adoptive transfer.

The therapeutic use and applicability of a bone marrow-derived myeloid progenitor cell (MPC) of the invention and/or a pharmaceutical composition of the invention is based on the finding of the present inventors that a bone marrow-derived myeloid progenitor cell (MPC) of the invention gives rise to myeloid cells that engraft in the central nervous system (CNS), preferably after bone marrow/hematopoietic stem cell (HSC) transplantation. In light thereof, a bone marrow-derived myeloid progenitor cell (MPC) of the invention is a suitable means for cell-based therapy, preferably cell-based therapy in the CNS.

As shown in the example part of the instant application, neurodegenerative diseases that are characterized by dysfunction or loss of neurons and preferably progressive dysfunction or loss of neurons, can be treated by means of adoptive transfer of bone marrow-derived myeloid progenitor cells (MPCs) of the invention. Most importantly, such treatment was successful even when administered after onset of such neurodegenerative disease. Accordingly, the disease for the treatment of which a bone marrow-derived myeloid progenitor cell (MPC) of the invention may be used, is a neurodegenerative disease which is characterized by or goes along with dysfunction or loss of neurons, preferably progressive dysfunction or loss of neurons. Furthermore, as to the stage of such neurodegenerative disease in the treatment of which a bone marrow-derived myeloid progenitor cell (MPC) of the invention is used, in an embodiment of the present invention, said stage is one after onset of the disease. In accordance therewith, in an embodiment the subject which is to be treated in accordance with the present invention is one where the disease is manifest.

In connection with the results shown in the instant application for amyotrophic lateral sclerosis and Alzheimer's disease, such applicability of a bone marrow-derived myeloid progenitor cell (MPC) is plausible for neurodegenerative diseases and inflammatory diseases of the central nervous system. More specifically, it has been shown in the art that amyotrophic lateral sclerosis which is an inevitably fatal, adult-onset neurodegenerative disease characterized by progressive loss of both cortical and spinal motor neurons leading to muscle atrophy, paralysis, and death due to respiratory failure typically within 2-5 years can, in principle, be treated by anti-inflammatory drugs. Exemplary anti-inflammatory drugs and anti-inflammatory cells which were used in the treatment of amyotrophic lateral sclerosis include minocycline (Van Den Bosch et al. Neuroreport. 2002, 13:1067-1070), bone marrow-derived cells (Corti et al. Brain. 2004, 127:2518-2532; Corti et al. Hum Mol Genet. 2010, 19:3782-3796), or mesenchymal stem cells (MSCs) derived from bone marrow (Karussis et al. Arch Neurol. 2010, 67:1187-1194) or umbilical cord (Garbuzova-Davis et al. Plos One. 2012, 7:e31254). However, these studies failed to show therapeutic effects when the treatment was applied after disease onset. The present inventors showed that adoptive transfer of bone marrow-derived myeloid progenitor cells (MPCs) of the invention for treatment purposes after disease onset resulted in an attenuation of inflammatory pathology and delay in disease progression, extension of lifespan and improvement of motor activity. It has also been shown in the art that perivascular macrophages play a crucial role in amyloid (Aß) clearance in Alzheimer's disease (Hawkes et al. PNAS. 2009, 106:1261-1266; Zaghi et al. Acta Neuropathol. 2009, 117:111-124; Mildner et al. J Neurosci. 2011, 31:11159-11171; Michaud et al. Cell Rep. 2013, 5:646-653). The present inventors showed that adoptive transfer for treatment purposes of bone marrow-derived myeloid progenitor cells (MPCs) of the invention gave rise to perivascular macrophages in the brain, after intravenous injection, which allows amelioration of the condition of a subject suffering from Alzheimer's disease.

Without wishing to be bound by any theory, the mode of action of a bone marrow-derived myeloid progenitor cell (MPC) of the invention is based on an immunomodulatory and/or anti-inflammatory action.

In accordance therewith, a disease, for the treatment of which a bone marrow-derived myeloid progenitor cell (MPC) of the invention and/or a pharmaceutical composition of the invention may be used, is in an embodiment a disease which is selected from the group comprising Alzheimer's disease, mild cognitive impairment, vascular dementia, frontotemporal lobar degeneration, amyotrophic lateral sclerosis, Huntington's disease, Tourette syndrome, Parkinson's disease, multisystem atrophy, multiple sclerosis, stroke, epilepsy, autoimmune encephalitis, meningitis, migraine, leukodystrophy, inborn error of metabolism, brain/spinal cord injury, brain tumour, cranial/peripheral nerve injury, myopathy, polyneuropathy, major depression, bipolar disorder, attention deficit hyperactivity disorder, psychotic disorder including schizophrenia, autism/autism spectrum disorders, Rett syndrome, alcohol/drug addiction, anxiety disorder and eating disorder.

Also in accordance therewith, a disease for the treatment of which a bone marrow-derived myeloid progenitor cell (MPC) of the invention and/or a pharmaceutical composition of the invention may be used, is any disease which goes along with or which is characterized and/or caused by a damage of the blood-brain barrier such as, for example, multiple sclerosis, stroke, Alzheimer's disease, encephalitis and meningo-encephalitis.

In an embodiment of the various aspects of the present invention and more specifically the use of a bone marrow-derived myeloid progenitor cell (MPC) of the invention and a pharmaceutical composition for the treatment of a subject suffering from or being at risk of suffering from a disease, preferably a disease as disclosed herein, the subject is one which has not been subject to irradiation. Such subject is preferably one suffering from a disease or being at risk of suffering from a disease, whereby such disease is any disease which goes along with or is characterized and/or are caused by a damage of or a damaged blood-brain barrier such as, for example, multiple sclerosis, stroke, Alzheimer's disease, and (meningo-) encephalitis.

It is also within the present invention that apart from a therapeutic effect which arises immediately from a bone marrow-derived myeloid progenitor cell (MPC) of the invention, a therapeutic effect and/or an additional therapeutic effect can be created if a bone marrow-derived myeloid progenitor cell (MPC) of the invention is a recombinant bone marrow-derived myeloid progenitor cell (MPC) of the invention delivering a therapeutically active agent into a subject to which the recombinant bone marrow-derived myeloid progenitor cell (MPC) of the invention is administered. Such therapeutically active agent is typically one that is encoded by the recombinant bone marrow-derived myeloid progenitor cell (MPC) of the invention. In an embodiment, the therapeutically active agent is a peptide, a polypeptide or a protein. In another embodiment the therapeutically active agent is a therapeutically active nucleic acid. Such therapeutically active nucleic acid may be selected from the group comprising an aptamer, an antisense molecule, a ribozyme, an siRNA and other regulatory RNA, including but not limited to miRNA. The therapeutically active agent is one which is suitable for ameliorating the disease from which a subject to which such recombinant bone marrow-derived myeloid progenitor cell (MPC) of the invention is administered, is suffering or at risk of suffering. In an embodiment, the therapeutically active agent is an agonist, for example a peptide, a polypeptide or a protein. In a preferred embodiment the therapeutically active agent is a compound selected from the group comprising a growth factor and a neuroprotective agent. Preferably the growth factor is selected from the group comprising brain-derived neurotrophic factor (BDNF), glial cell-derived neurotrophic factor (GDNF), ciliary neurotrophic factor (CNTF) and insulin-like growth factors (IGFs). Preferably, the neuroprotective agent is selected from the group comprising an erythropoietin (EPO), EPO variant and an antioxidant. In another embodiment the therapeutically active agent is an antagonist targeting a target molecule which when antagonized ameliorates the disease outcome. Such antagonist is, for example, an aptamer, an antisense molecule, a ribozyme, an siRNA or other regulatory RNA, including but not limited to miRNA.

It is within the present invention that a bone marrow-derived myeloid progenitor cell (MPC) of the invention, in particular a recombinant bone marrow-derived myeloid progenitor cell (MPC) of the invention, is suitable for or is actually delivering or is used for the delivery of a therapeutically active agent into the central nervous system of a subject to which such bone marrow-derived myeloid progenitor cell (MPC) of the invention is administered. In an embodiment, the thus delivered therapeutically active agent is a therapeutically active peptide, polypeptide or protein, as disclosed herein. In another embodiment, the thus delivered therapeutically active nucleic acid is a therapeutically active nucleic acid as disclosed herein. With regard to this aspect of the use of a bone marrow-derived myeloid progenitor cell (MPC) of the invention, it will be acknowledged by a person skilled in the art that such use is similar to the one of bone marrow-derived mesenchymal stromal cells (MSC).

In a further embodiment of a bone marrow-derived myeloid progenitor cell (MPC), the bone marrow-derived myeloid progenitor cell (MPC) is labelled with a detectable label. In a preferred embodiment the detectable label is a diagnostically active label. In a more preferred embodiment the diagnostically active label is selected from the group comprising a magnetic nanoparticle and an isotope. Magnetic nanoparticles which are suitable for use in a method of diagnosis, both an *in vivo* method of diagnosis and an *in vitro* method of diagnosis, are known to a person skilled in the art. In an embodiment the isotope is selected from the group comprising ^{113m}In 99^{m}Tc, ⁶⁷Ga, ⁵²Fe, ⁵⁴Fe, ⁶⁸Ga ⁷²As, ¹¹¹In, ⁹⁷Ru, ²⁰³Pb, ⁶²Cu, ⁶⁴Cu, ⁵¹Cr, ^{52m}Mn, ¹⁵⁷Gd, ⁶⁴Cu, ⁸⁹Zr, ¹⁷⁷Lu, ¹⁸F, ¹⁹F, ¹²³I, ¹²⁴I, ¹²⁵I, ¹³¹I, ⁷⁵Br, ⁷⁶Br, ⁷⁷Br, ⁸²Br, and ²¹¹At. The diagnostic composition of the invention comprises a bone marrow-derived myeloid progenitor cell (MPC) of the invention and a diagnostically acceptable excipient. In an embodiment, the bone marrow-derived myeloid progenitor cell (MPC) of the invention is a bone marrow-derived myeloid progenitor cell (MPC) of the invention that is labelled with a detectable label. Preferably, such label is a detectable label as disclosed herein. The diagnostically acceptable excipient is an excipient that allows the use of the diagnostic composition of the invention for the diagnosis of a disease. In an embodiment, the diagnostically acceptable excipient is an excipient as disclosed herein in connection with a pharmaceutical composition of the invention.

The invention is further illustrated by the following embodiments:
Embodiment 1: A bone marrow-derived myeloid progenitor cell (MPC), wherein the bone marrow-derived myeloid progenitor cell (MPC) is expressing CD34 and c-Kit (CD 117).
Embodiment 2: The bone marrow-derived myeloid progenitor cell (MPC) of embodiment 1, wherein the bone marrow-derived myeloid progenitor cell (MPC) is expressing CD115.
Embodiment 3: The bone marrow-derived myeloid progenitor cell (MPC) of any one of embodiments 1 to 2, wherein the bone marrow-derived myeloid progenitor cell (MPC) is expressing CD135.
Embodiment 4: The bone marrow-derived myeloid progenitor cell (MPC) of any one of embodiments 1 to 3, wherein the bone marrow-derived myeloid progenitor cell (MPC) is expressing CD115 and CD 135
Embodiment 5: The bone marrow-derived myeloid progenitor cell (MPC) of any one of embodiments 1 to 4, wherein the bone marrow-derived myeloid progenitor cell (MPC) is expressing CX3CR1.
Embodiment 6: The bone marrow-derived myeloid progenitor cell (MPC) of any one of embodiments 1 to 5, wherein the bone marrow-derived myeloid progenitor cell (MPC) is expressing CD115, CD135 and CX3CR1.
Embodiment 7: The bone marrow-derived myeloid progenitor cell (MPC) of any one of embodiments 1 to 6, wherein the bone marrow-derived myeloid progenitor cell (MPC) is IL-17Ra-negative.
Embodiment 8: The bone marrow-derived myeloid progenitor cell (MPC) of any one of embodiments 1 to 7, wherein the bone marrow-derived myeloid progenitor cell (MPC) is negative for at least one B-cell marker, preferably at least one membrane-bound B-cell marker.
Embodiment 9: The bone marrow-derived myeloid progenitor cell (MPC) of embodiment 8, wherein at least one B-cell marker is selected from the group comprising CD45R, CD19, CD20, CD22 und B220.
Embodiment 10: The bone marrow-derived myeloid progenitor cell (MPC) of any one of embodiments 1 to 9, wherein the bone marrow-derived myeloid progenitor cell (MPC) is negative for at least one T-cell marker.
Embodiment 11: The bone marrow-derived myeloid progenitor cell (MPC) of embodiment 10, wherein the at least one T-cell marker is selected from the group comprising CD5, CD3, CD4 and CD8.
Embodiment 12: The bone marrow-derived myeloid progenitor cell (MPC) of any one of embodiments 1 to 11, wherein the bone marrow-derived myeloid progenitor cell (MPC) is negative for at least one mature myeloid cell marker.
Embodiment 13: The bone marrow-derived myeloid progenitor cell (MPC) of embodiment 10, wherein the at least one mature myeloid cell marker is selected from the group comprising CD11b, CD 14, CD16 and 7-4.
Embodiment 14: The bone marrow-derived myeloid progenitor cell (MPC) of any one of embodiments 1 to 13, wherein the bone marrow-derived myeloid progenitor cell (MPC) is negative for at least one granulocyte/monocyte marker.
Embodiment 15: The bone marrow-derived myeloid progenitor cell (MPC) of embodiment 14, wherein the at least one granulocyte/monocyte marker is selected from the group comprising Gr-1 and Ly6-G and Ly6-C.
Embodiment 16: The bone marrow-derived myeloid progenitor cell (MPC) of any one of embodiments 1 to 15, wherein the bone marrow-derived myeloid progenitor cell (MPC) is negative for at least one granulocyte marker.
Embodiment 17: The bone marrow-derived myeloid progenitor cell (MPC) of embodiment 16, wherein, the at least one granulocyte marker is selected from the group comprising Ly6-G.
Embodiment 18: The bone marrow-derived myeloid progenitor cell (MPC) of any one of embodiments 1 to 17, wherein the bone marrow-derived myeloid progenitor cell (MPC) is negative for at least one erythrocyte marker.
Embodiment 19: The bone marrow-derived myeloid progenitor cell (MPC) of embodiment 18, wherein the at least one erythrocyte marker is selected from the group comprising Ter-119.
Embodiment 20: The bone marrow-derived myeloid progenitor cell (MPC) of any one of embodiments 1 to 19, wherein the bone marrow-derived myeloid progenitor cell (MPC) is a recombinant bone marrow-derived myeloid progenitor cell (MPC).
Embodiment 21: The bone marrow-derived myeloid progenitor cell (MPC) of embodiment 20, wherein the recombinant bone marrow-derived myeloid progenitor cell (MPC) comprises a heterologous nucleic acid.
Embodiment 22: The bone marrow-derived myeloid progenitor cell (MPC) of embodiment 21, wherein the heterologous nucleic acid encodes a therapeutically active peptide, polypeptide or protein.
Embodiment 23: The bone marrow-derived myeloid progenitor cell (MPC) of embodiment 21, wherein the heterologous nucleic acid is a therapeutically active nucleic acid. Embodiment 24: The bone marrow-derived myeloid progenitor cell (MPC) of any one of embodiments 1 to 23, wherein the bone marrow-derived myeloid progenitor cell (MPC) is labelled with a therapeutically active isotope, preferably a therapeutically active radioisotope.
Embodiment 25: The bone marrow-derived myeloid progenitor cell (MPC) of any one of embodiments 1 to 23, wherein the bone marrow-derived myeloid progenitor cell (MPC) is labelled with a diagnostically active label, preferably the label is an isotope, more preferably a diagnostically active radioisotope.
Embodiment 26: The bone marrow-derived myeloid progenitor cell (MPC) of any one of embodiments 1 to 25, wherein the bone marrow-derived myeloid progenitor cell (MPC) is a human bone marrow-derived myeloid progenitor cell (hMPC).
Embodiment 27: The bone marrow-derived myeloid progenitor cell (MPC) of any one of embodiments 1 to 26, for use in a method for treating a subject suffering from a disease or being at risk of suffering from a disease.
Embodiment 28: The bone marrow-derived myeloid progenitor cell (MPC) of embodiment 27, wherein the method is adoptive transfer of the myeloid progenitor cell (MPC) derived from bone marrow or mobilized human peripheral blood stem cells, preferably adoptive transfer in the treatment of the disease.
Embodiment 29: The bone marrow-derived myeloid progenitor cell (MPC) of embodiment 28, wherein the adoptive transfer is an allogeneic adoptive transfer.
Embodiment 30: The bone marrow-derived myeloid progenitor cell (MPC) of embodiment 29, wherein the adoptive transfer is an autologous adoptive transfer.
Embodiment 31: The bone marrow-derived myeloid progenitor cell (MPC) of any one of embodiments 28 to 30, wherein the adoptively transferred bone marrow-derived myeloid progenitor cell (MPC) is a recombinant bone marrow-derived myeloid progenitor cell (MPC). Embodiment 32: The bone marrow-derived myeloid progenitor cell (MPC) of embodiment 27, wherein the disease is a neurodegenerative disease.
Embodiment 33: The bone marrow-derived myeloid progenitor cell (MPC) of embodiment 32, wherein the disease is an inflammatory nervous system disease.
Embodiment 34: The bone marrow-derived myeloid progenitor cell (MPC) of any one of embodiments 32 to 33, wherein the disease is selected from the group comprising Alzheimer's disease, mild cognitive impairment, vascular dementia, frontotemporal lobar degeneration, amyotrophic lateral sclerosis, Huntington's disease, Tourette syndrome, Parkinson's disease, multisystem atrophy, multiple sclerosis, stroke, epilepsy, autoimmune encephalitis, meningitis, migraine, leukodystrophy, inborn error of metabolism, brain/spinal cord injury, brain tumour, cranial/peripheral nerve injury, myopathy, polyneuropathy, major depression, bipolar disorder, attention deficit hyperactivity disorder, psychotic disorder including schizophrenia, autism/autism spectrum disorders, Rett syndrome, alcohol/drug addiction, anxiety disorder, eating disorder.
Embodiment 35: The bone marrow-derived myeloid progenitor cell (MPC) of any one of embodiments 27 to 34, wherein the subject has not been subject to irradiation, preferably brain irradiation.
Embodiment 36: The bone marrow-derived myeloid progenitor cell (MPC) of any one of embodiments 1 to 26, for use in a method for treating a subject suffering from a disease or being at risk of suffering from a disease, wherein the bone marrow-derived myeloid progenitor cell (MPC) is a recombinant bone marrow-derived myeloid progenitor cell (MPC) delivering a therapeutically active agent into the subject.
Embodiment 37: The bone marrow-derived myeloid progenitor cell (MPC) of embodiment 36, wherein the therapeutically active agent is selected from the group comprising a peptide, a polypeptide and a protein.
Embodiment 38: The bone marrow-derived myeloid progenitor cell (MPC) of embodiment 36, wherein the therapeutically active agent is a therapeutically active nucleic acid.
Embodiment 39: The bone marrow-derived myeloid progenitor cell (MPC) of any one of embodiments 36 to 38, wherein the therapeutically active agent is delivered into the central nervous system of the subject.
Embodiment 40: The bone marrow-derived myeloid progenitor cell (MPC) of any one of embodiment 1 to 26, for use in a method for diagnosing a disease in a subject.
Embodiment 41: The bone marrow-derived myeloid progenitor cell (MPC) of embodiment 40, wherein the disease is a disease of the CNS.
Embodiment 42: The bone marrow-derived myeloid progenitor cell (MPC) of any one of embodiments 40 to 41, wherein the disease is an inflammatory disease.
Embodiment 43: The bone marrow-derived myeloid progenitor cell (MPC) of any one of embodiment 40 to 42, wherein the bone marrow-derived myeloid progenitor cell (MPC) is labeled with a detectable label.
Embodiment 44: The bone marrow-derived myeloid progenitor cell (MPC) of embodiment 43, wherein the detectable label is a magnetic nanoparticle or an isotope.
Embodiment 45: The bone marrow-derived myeloid progenitor cell (MPC) of embodiment 44, wherein the isotope is a radioisotope.
Embodiment 46: The bone marrow-derived myeloid progenitor cell (MPC) of embodiment 45, wherein the method comprises a non-invasive detection step.
Embodiment 47: The bone marrow-derived myeloid progenitor cell (MPC) of embodiment 46, wherein the non-invasive detection step comprises imaging techniques, preferably the non-invasive detection step comprises CT and/or MRI and/or PET and/or SPECT.
Embodiment 48: The bone marrow-derived myeloid progenitor cell (MPC) of any one of embodiments 27 to 47, wherein the bone marrow-derived myeloid progenitor cell (MPC) is administered to the subject by means of intravenous injection.
Embodiment 49: The bone marrow-derived myeloid progenitor cell (MPC) of any one of embodiments 27 to 48, wherein the bone marrow-derived myeloid progenitor cell (MPC) is administered only once to the subject.
Embodiment 50: The bone marrow-derived myeloid progenitor cell (MPC) of any one of embodiments 27 to 49, wherein the subject is a mammal, preferably a human.
Embodiment 51: A pharmaceutical composition comprising a bone marrow-derived myeloid progenitor cell (MPC) of any one of embodiments 1 to 26 and a pharmaceutically acceptable excipient.
Embodiment 52: The pharmaceutical composition for use in a method of treatment as defined in any of embodiments 27 to 39 and 48 to 50.
Embodiment 53: A method for the treatment of a disease, wherein the method comprises administering to a subject a bone marrow-derived myeloid progenitor cell (MPC) according to any one of embodiments 1 to 26 or a pharmaceutical composition according to any one of embodiments 51 to 52.
Embodiment 54: The method of embodiment 53, wherein the disease is a disease as defined in any one of embodiments 27 to 39.
Embodiment 55: A diagnostic composition comprising a bone marrow-derived myeloid progenitor cell (MPC) of any one of embodiments 1 to 26 and a diagnostically acceptable excipient.
Embodiment 56: The diagnostic composition for use in a method of treatment as defined in any of embodiments 40 to 50.
Embodiment 57: A method for the diagnosis of a disease, wherein the method comprises administering to a subject a bone marrow-derived myeloid progenitor cell (MPC) according to any one of embodiments 1 to 26 or a diagnostic composition according to any one of embodiments 55 to 56.
Embodiment 58: The method of embodiment 57, wherein the disease is a disease as defined in any one of embodiments 27 to 39.
Embodiment 59: A kit comprising a bone marrow-derived myeloid progenitor cell (MPC) according to any one of embodiments 1 to 26 and at least one constituent selected from the group comprising a container, a buffer, a diluent, an excipient and an instruction leaflet.

Without wishing to be bound by any theory, the diagnostic use of a bone marrow-derived myeloid progenitor cell (MPC) of the invention and/or of a diagnostic composition of the invention is based on the bone marrow-derived myeloid progenitor cell (MPC) being attracted to site of damage in the central nervous system, in particular inflammatory site. Due to the detectable label of a bone marrow-derived myeloid progenitor cell (MPC) of the invention such labeled bone marrow-derived myeloid progenitor cell (MPC) can be detected at the site of damage by a suitable detection device, preferably an imaging device and thus the site of damage be identified.

The diagnostic composition of the invention may be formulated for any appropriate route of administration, including, for example, parenteral administration. The term parenteral as used herein includes subcutaneous, intradermal, intravascular such as, e.g., intravenous, intramuscular, intrathecal and intra-arterial injection, as well as any similar injection or infusion technique. A preferred route of administration is intravenous administration.

A bone marrow-derived myeloid progenitor cell (MPC) of the invention and/or a diagnostic composition of the invention may be used in the diagnosis of a disease and/or in the diagnosis of a subject suffering from or being at risk of suffering from a disease. In a preferred embodiment the disease is a neurodegenerative disease. It is within the present invention that any disease disclosed herein in connection with the use of a bone marrow-derived myeloid progenitor cell (MPC) for the treatment of a disease is also a disease which can be diagnosed by the means and methods of the present invention and a bone marrow-derived myeloid progenitor cell (MPC) of the invention and/or a diagnostic composition of the invention in particular.

It will be acknowledged that techniques and methods suitable for use in the diagnosis of a disease using a bone marrow-derived myeloid progenitor cell (MPC) of the invention and/or a diagnostic composition of the invention, are known to a person skilled in the art. In an embodiment, the method of diagnosis is a non-invasive diagnosis technique or comprises a non-invasive detection step. In a preferred embodiment the non-invasive detection step comprises one or any combination of computer tomography (CT), Magnetic resonance imaging (MRI), nuclear magnetic resonance imaging (NMRI), or magnetic resonance tomography (MRT), single photon emission computer tomography (SPECT) and/or Positron emission tomography (PET).

Dosages employed in practicing the methods for treatment and diagnosis, respectively, where bone marrow-derived myeloid progenitor cells (MPCs) of the invention are used and, respectively, administered to a subject will vary depending e.g. on the particular condition to be treated, and feature of the subject such as age, size and the like. A typical dosage and/or dose of bone marrow-derived myeloid progenitor cells (MPCs) of the invention administered to the subject is from 0.1 to 10 million bone marrow-derived myeloid progenitor cells (MPCs) or from 0.5 to 1 million bone marrow-derived myeloid progenitor cells (MPCs). It is within the present invention that in the treatment of a disease in accordance with the present invention only a single dose of bone marrow-derived myeloid progenitor cells (MPCs) is administered to a subject. It is also within the present invention that in the diagnosis of a disease in accordance with the present invention only a single dose of bone marrow-derived myeloid progenitor cells (MPCs) is administered to a subject.

In connection with each and any aspect of the present invention, a subject is preferably a mammal and more preferably a human being.

A kit according to the present invention comprising a bone marrow-derived myeloid progenitor cell (MPC) of the invention and at least one constituent selected from the group comprising a container, a buffer, a diluent, an excipient and an instruction leaflet. In an embodiment, the kit is a ready-for-use kit. In an embodiment the kit comprises a therapeutically active agent, preferable in case the kit is for therapeutic use, more preferably for use in a method of treatment of a disease as disclosed herein. In another embodiment the kit comprises a detectable label, preferable in case the kit is for diagnostic use, more preferably for use in a method of diagnosing a disease as disclosed herein.

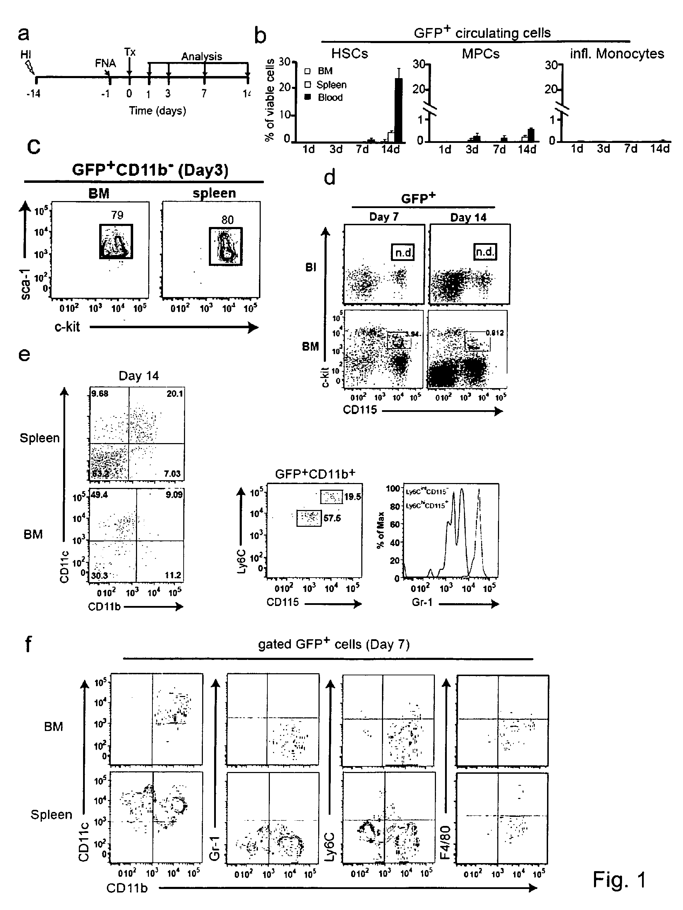

The present invention is now further illustrated by reference to the following figures and examples from which further advantages, features, and embodiments may be taken, wherein
Fig. 1a is a diagram illustrating the experimental set up for adoptive transfer of HSCs, MPCs and monocytes, wherein a single cell population was intravenously injected into recipients that were previously conditioned with 11 Gy focal head irradiation (HI) and facial nerve axotomy (FNA); immunohistochemical and FACS analyses were performed at 1, 3, 7 and 14 days after transplantation;
Fig. 1b is a panel of three diagrams indicating donor-derived cells in peripheral blood, bone marrow and spleen 1, 3, 7 and 14 days after adoptive transfer, wherein the left diagram is indicating HSCs as percentage of viable cells, the middle diagram is indicating MPCs as percentage of viable cells, and the right diagram is indicating inflammatory monocytes as percentage of viable cells (the error bar represents SEM);
Fig. 1c is a panel of two diagrams indicating FACS analysis of gated GFP⁺CD11b⁻ cells in BM and spleen of mice at 3 days after transplantation of GFP⁺ HSCs; donor-derived cells remained sca1⁺c-kit⁺Lin⁻ (HSCs). Contour plot (in grey) represents host HSCs (GFP⁻);
Fig. 1d is a panel of four diagrams indicating development of MPCs (sca1⁻c-kit⁺Cd115⁺CD11b⁻) from donor HSCs in blood (B1) and BM at 7 and 14 days after transplantation. Contour plot (in grey) represents host MPCs (GFP⁻);
Fig. 1e is a panel of four diagrams indicating differentiation assays of HSCs in spleen and bone marrow (BM) by FACS; HSC-derived CD11c⁺CD11b⁺, CD11c⁺CD11b⁻, CD11b+Ly6C^{hi}CD115⁺Gr-1^{lo} monocytes and CD11b+Ly6C^{int}CD115⁻Gr-1⁺ granulocytes were identified;
Fig. If is a panel of eight diagrams indicating differentiation assays of donor MPCs in bone marrow and spleen by FACS at 7 days after transplantation; DCs, macrophages (mΦ) and monocytes were identified, whereas no MPC-derived Gr-1⁺ neutrophils were detected;

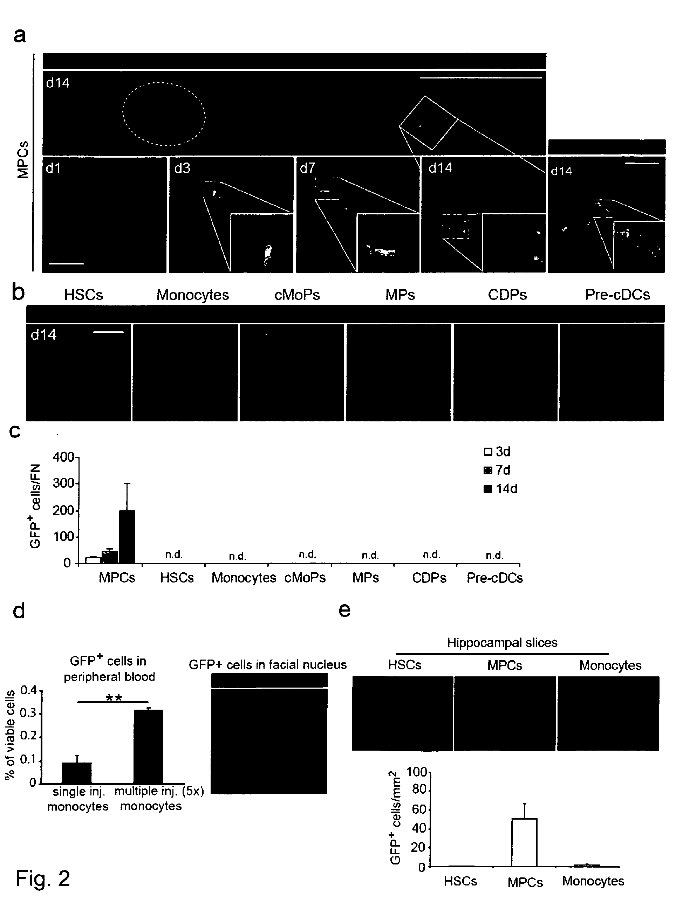

Fig. 2a is a panel of six laser confocal microscopic images (scale bar: 25 µm) showing the dorsal part of the brain stem of a focally head-irradiated mouse that was subjected to unilateral facial nerve axotomy followed by adoptive transfer of sorted MPCs (upper panel); the microglia/mΦ markers, Iba-1 and F4/80, are shown in red and GFP is shown in green, identifying donor-derived cells; nuclei are counterstained with DAPI (blue); the contralateral unlesioned facial nucleus (white circle) shows minimal Iba-1 immunoreactivity compared to the ipsilateral facial nucleus (white square); the lower panel shows higher magnification images (scale bar: 100 µm) of the axotomized facial nucleus in MPC-transplanted mice at days 1 (d1), 3 (d3), 7 (d7) and 14 (d14) after transplantation; of note, the increase in Iba-1 immunoreactivity reveals increased inflammation with time; GFP⁺Iba-1⁺ cells were detected at 3, 7 and 14 days after transplantation (n = 3-5);
Fig. 2b is a panel of six laser confocal microscopic images indicating lack of engraftment of HSC-, monocyte-, cMoP-, MP-, CDP-, and Pre-cDC-derived cells in the lesioned facial nucleus at 14 days after adoptive transfer (scale bar: 100 µm); no donor-derived GFP⁺ cells were detected at any time point up to 14 days (n = 3-5);
Fig. 2c is a diagram showing quantification of donor-derived cell engraftment in the lesioned facial nucleus at 3, 7 and 14 days after transplantation; three to five mice were used per group. n.d. = not detected;
Fig. 2d is a diagram (left) and a laser confocal microscopic image (right) indicating the results of daily transplantations of Ly6C^{hi} monocytes for five consecutive days; focally head-irradiated mice received a facial nerve axotomy and daily intravenous injections of 1x10⁶ Ly6C^{hi} monocytes for five consecutive days starting on day 1 after surgery; mice were sacrificed at 7 days after the first injection; FACS analysis of GFP⁺ cells in peripheral blood revealed higher chimerism in mice receiving multiple (5x) injections of monocytes compared to a single injection (the error bar represents SEM); the left laser confocal microscopic image indicates that no monocyte-derived GFP⁺ cells were found in the axotomized facial nucleus (scale bar: 100 µm); three animals per groups were used;

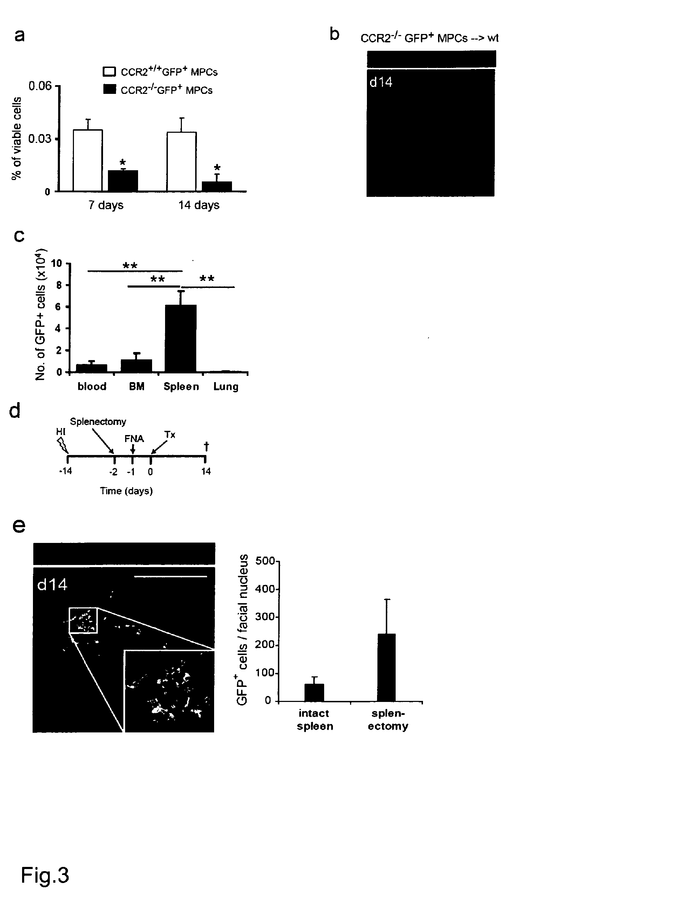

Fig. 3a is a diagram indicating the role of chemokine receptor 2 (CCR2) for the homing of MPCs to the spleen; CCR2^{-/-} or CCR2^{+/+} MPCs that express GFP were intravenously injected into mice after focal head irradiation and facial nerve axotomy; seven and fourteen days after transplantation, engraftment of GFP⁺ cells in the spleen was quantified by FACS; homing of MPCs to the spleen was reduced in the absence of CCR2 (n=3, means + SEM, unpaired *t-test* *p<0.05);
Fig. 3b is a laser confocal microscopic image of the axotomized facial nucleus of a focally head-irradiated mouse that received CCR2^{-/-} GFP⁺ MPCs. At 14 days after transplantation, no donor-derived GFP⁺ cells were observed in the facial nucleus (scale bar: 100 µm). Iba-1 is shown in red and GFP in green. Nuclei are counterstained with DAPI (blue).
Fig. 3c is a diagram showing the quantification of MPC-derived cells in different tissue compartments of mice that were intravenously injected with GFP⁺ MPCs after focal head irradiation and facial nerve axotomy ; most GFP⁺ cells were found in the spleen at 14 days after transplantation (n=5, means + SEM, unpaired *t-test* **p<0.01);
Fig. 3d is a diagram illustrating the experimental set up for adoptive transfer of MPCs into mice that received focal head irradiation (HI), followed by splenectomy 12 days later and facial nerve axotomy (FNA) on day 13, i.e. one day prior to intravenous transplantation (Tx) of 2x10⁴ sorted MPCs from GFP-expressing mice; immunohistochemical and FACS analyses were performed at 14 days after transplantation;
Fig. 3e is a laser confocal microscopic image (left) and a diagram (right) indicating the impact of splenectomy on the engraftment of MPC-derived GFP⁺ cells in the axotomized facial nucleus; Iba-1 immunoreactivity is shown in red and GFP in green; nuclei are counterstained with DAPI (blue); scale bar: 100 µm; MPCs do not require the passage through the spleen to engraft in the CNS;

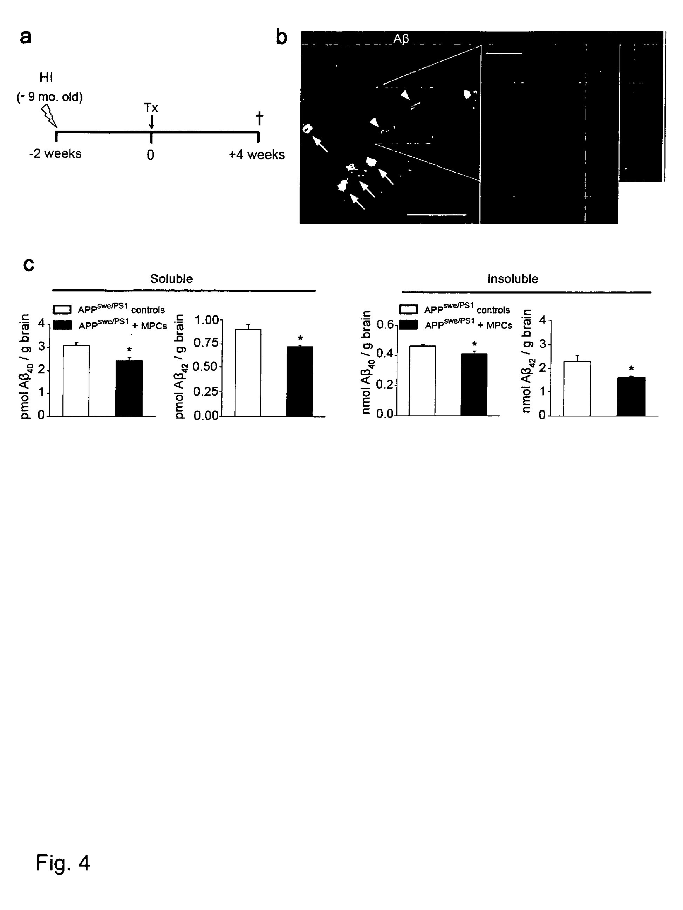

Fig. 4a is a diagram illustrating the experimental set up for transplantation of MPCs into Alzheimer's disease (AD) transgenic mice; *APP*^{*swe*/*PS1*} mice (8-9 months old) received focal head irradiation (HI) two weeks before intravenous transplantation (Tx) of 5x10⁵ GFP⁺ MPCs; all animals, including non-transplanted *APP*^{*swe*/*PS1*} controls, received daily intraperitoneal injections of 1.5 mg/kg rapamycin; mice were analyzed at 4 weeks after transplantation;
Fig. 4b is a representative laser confocal microscopic image of the brain of an *APP^{sewlPS1}* mouse at 4 weeks after transplantation of GFP⁺ MPCs (scale bar: 100 µm); GFP-expressing cells (green) are exclusively found at perivascular sites; Aβ amyloid deposits are shown in white, endothelial cells are identified by CD31 immunoreactivity (red); nuclei are counterstained with DAPI (blue); the right panel shows a higher magnification image and 3-dimensional analysis of the area indicated by the square in the confocal image.;
Fig. 4c is a panel of four diagrams indicating the amounts of soluble and insoluble Aβ₁₋₄₀ and Aβ₁₋₄₂ as determined by ELISA in brain lysates from non-transplanted (white columns) and MPC-transplanted (black columns) *APP*^{*swe*/*PS1*} animals at 4 weeks after transplantation; data are means + SEM from 3-4 mice per group; unpaired *t-test* *p<0.05;

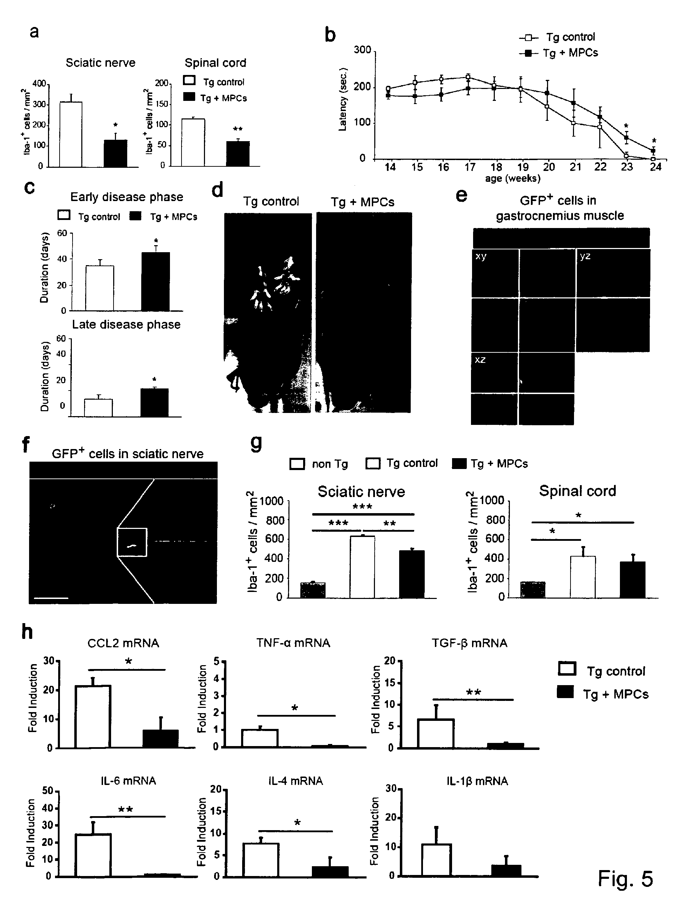

Fig. 5a is a panel of two diagrams indicating short-term anti-inflammatory effects of MPCs in a transgenic mouse model of amyotrophic lateral sclerosis (ALS); *Tg* denotes non-irradiated *SOD1^{G93A}* transgenic mice (13-14 weeks old) that received either PBS (control) or a single intravenous injection of 5x10⁵ GFP⁺ MPCs, followed by daily intraperitoneal injections of 1.5 mg/kg rapamycin for 4 weeks; the nervous system of the mice was analyzed by immunohistochemistry using the microglia/macrophage marker, Iba1, at 4 weeks after transplantation; MPC-treated *SOD1^{G93A}* transgenic animals (Tg + MPCs, black columns) showed significantly less Iba-1-immunoreactive cells in the sciatic nerve and the spinal cord (lumbar region) compared with controls (means + SEM, n = 3, unpaired *t-test,* *p<0.05, **p<0.01);
Fig. 5b is a diagram depicting the motor performance of *SOD1^{G93A}* transgenic mice after transplantation of MPCs at 13-14 weeks of age (black squares) compared with non-transplanted *SOD1^{G93A}* transgenic mice (white squares) as determined by the rotarod test; MPC-transplanted mice showed significantly improved motor performance at more progressed stages of disease (n=6-8, means ± SEM, unpaired *t-test,* *p<0.05);
Fig. 5c is a panel of two diagrams depicting the slowing of disease progression in *SOD1^{G93A}* transgenic mice after a single intravenous transplantation of MPCs at 13-14 weeks of age; Compared to the PBS-treated animals (Tg control, white columns), MPC-treated *SOD1^{G93A}* transgenic mice (Tg + MPCs, black columns) showed delayed disease progression as indicated by the longer duration of the early disease phase (time at peak weight to time at 5% weight loss) and the longer duration of the late disease phase (time at > 5% weight loss to end stage) (n=6-8, means + SEM, unpaired *t-test,* *p<0.05);
Fig. 5d are two images showing improved motor function in end-stage *SOD1^{G93A}* transgenic mice after MPC transplantation; note that MPC-treated animals (Tg + MPCs) maintain the capacity to balance out with their hindlimbs, which non-transplanted *SOD1^{G93A}* transgenic mice (Tg control) cannot;
Fig. 5e is a laser confocal microscopic image depicting long-term engraftment of MPC-derived cells in the hindlimb musculature of *SOD1^{G93A}* transgenic mice; a GFP⁺ cell (green) that expresses the macrophage marker, Ibal (red), is detected in the hindlimb musculature of a *SOD1^{G93A}* transgenic mouse at 10 weeks after transplantation of GFP⁺ MPCs; nuclei are counterstained with DAPI (blue), three-dimensional analysis of the high magnification image is provided;
Fig. 5f is a laser confocal microscopic image depicting long-term engraftment of MPC-derived cells in the sciatic nerve of *SOD1^{G93A}* transgenic mice; GFP⁺ cells (green) that express the macrophage marker, Iba1 (red), are detected in the sciatic nerve of a *SOD1^{G93A}* transgenic mouse at 10 weeks after transplantation of GFP⁺ MPCs; nuclei are counterstained with DAPI (blue); scale bar: 100 µm; the right panel shows a higher magnification image of the area indicated by the square in the confocal image.;
Fig. 5g is a panel of two diagrams indicating long-term anti-inflammatory effects of MPCs in a transgenic mouse model of ALS; non-irradiated *SOD1^{G93A}* transgenic mice (13-14 weeks old) received either PBS (Tg control) or a single intravenous injection of 5x10⁵ GFP⁺ MPCs (Tg + MPCs), followed by daily intraperitoneal injections of 1.5 mg/kg rapamycin until the end stage of disease (> 20% weight loss or failure of the mouse to right itself within 30 seconds); wild type mice (non Tg) served as controls; the nervous system of the mice was analyzed by immunohistochemistry using the microglia/macrophage marker, Ibal, at end stage of disease; MPC-treated *SOD1^{G93A}* transgenic animals (black columns) showed less Iba-1-immunoreactive cells in the sciatic nerve and the spinal cord (lumbar region) compared to non-transplanted *SOD1^{G93A}* transgenic mice (white columns); all groups of transgenic animals had significantly more Iba-1-immunoreactive cells in the sciatic nerve and the spinal cord (lumbar region) compared to wild type mice (grey columns) of comparable age and treatment (means + SEM, n = 3, One-Way ANOVA with Bonferroni's Test *p<0.05; **p<0.01; ***p<0.001);
Fig.Sh is a panel of six diagrams indicating attenuated expression of proinflammatory cytokines and chemokines in the nervous system of ALS transgenic mice after MPC transplantation; *SOD1^{G93A}* transgenic mice (13-14 weeks old) received either PBS (Tg control) or a single intravenous injection of 5x10⁵ GFP⁺ MPCs (Tg + MPCs); quantitative real-time PCR analysis of CCL2, TNF-α, TGF-β, IL-6, IL-4 and IL-1β mRNA expression was performed in the spinal cords of Tg control (white columns) and Tg + MPCs (black columns) at end stage of disease; the mRNA expression levels were normalized to GAPDH mRNA and expressed as fold induction compared to non-transgenic wild type mice; MPC administration had significant anti-inflammatory effects in the CNS of ALS transgenic mice as indicated by the reduced gene expression of proinflammatory cytokines and chemokines in the spinal cord; data are means + SEM from 3 animals per group; statistical significance is indicated by asterisks (unpaired *t-test* *p<0.05; **p<0.01);

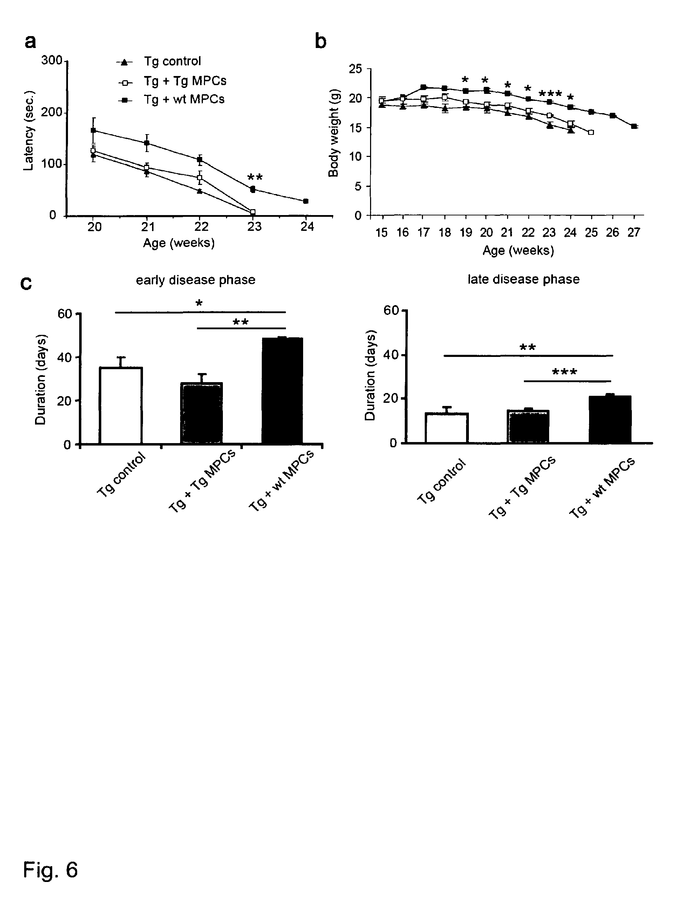

Fig. 6a is a diagram indicating that wild type, but not SOD1 mutant MPCs delay disease progression in ALS transgenic mice; non-irradiated *SOD1^{G93A}* transgenic mice (13-14 weeks old) received either PBS (Tg control, black triangles) or a single intravenous injection of 5x10⁵ MPCs from either *SOD1^{G93A}* transgenic mice (Tg + Tg MPCs, white squares) or non-transgenic wild type mice (Tg + wt MPCs, black squares), followed by daily intraperitoneal injections of 1.5 mg/kg rapamycin until the end stage of disease; motor performance of *SOD1^{G93A}* transgenic mice transplanted with wild type MPCs was significantly improved in the rotarod test at the end stage of disease compared with non-transplanted transgenic animals and *SOD1^{G93A}* transgenic mice transplanted with SOD1 mutant MPCs; data are means ± SEM from 3-4 animals per group; statistical significance is indicated by asterisks (unpaired *t-test* **p<0.01);
Fig. 6b is a diagram indicating that wild type, but not SOD1 mutant MPCs delay disease progression in ALS transgenic mice; weight loss of *SOD1^{G93A}* transgenic mice transplanted with non-transgenic wild type MPCs (Tg + wt MPCs, black squares) was significantly attenuated during disease compared with non-transplanted *SOD1^{G93A}* transgenic animals (Tg control, black triangles) and *SOD1^{G93A}* transgenic mice transplanted with SOD1 mutant MPCs (Tg + Tg MPCs, white squares); data are means ± SEM from 3-4 animals per group. Statistical significance is indicated by asterisks (unpaired *t-test* *p<0.05; ***p<0.001);
Fig. 6c is a panel of two diagrams depicting the slowing of disease progression in *SOD1^{G93A}* transgenic mice transplanted with wild type MPCs; compared to the non-transplanted *SOD1^{G93a}* transgenic animals (Tg control, white columns) and the *SOD1^{G93A}* transgenic mice transplanted with MPCs from *SOD1^{G93A}* transgenic mice (Tg + Tg MPCs, grey columns), *SOD1^{G93A}* transgenic mice transplanted with non-transgenic wild type MPCs (Tg + wt MPCs, black columns) showed delayed disease progression as indicated by the longer duration of the early disease phase (time at peak weight to time at 5% weight loss) and the longer duration of the late disease phase (time at > 5% weight loss to end stage) (3-4 animals per group, means + SEM, unpaired *t-test,* *p<0.05; **p<0.01; ***p<0.001).

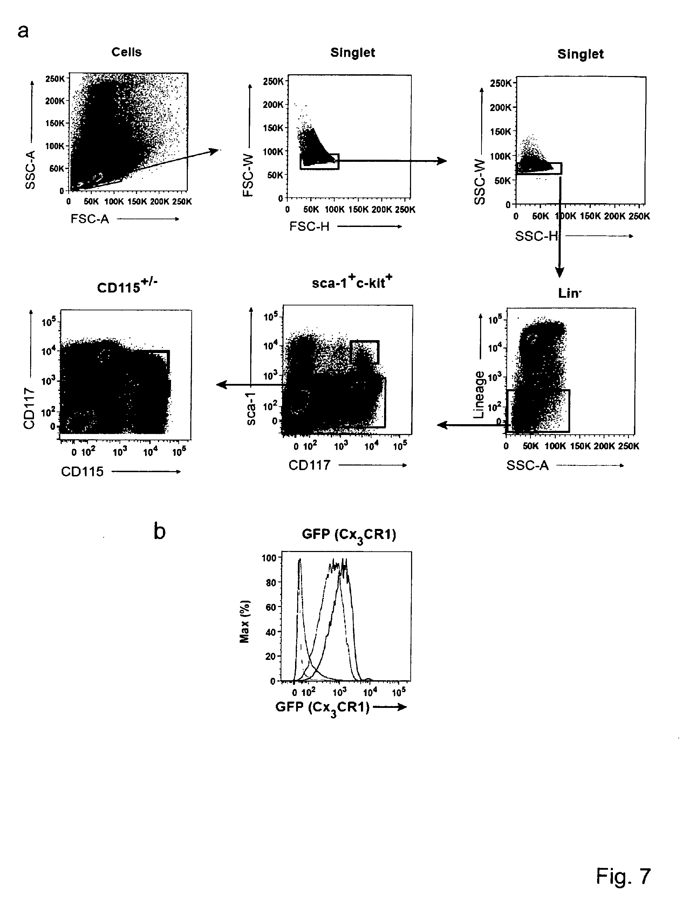

Fig. 7a is a panel of six diagrams depicting the gating strategy for the identification of hematopoietic stem cells (HSCs), myeloid precursors (MPs), myeloid progenitor cells (MPCs) and common dendritic cell precursors (CDPs) by flow cytometry of murine bone marrow. Single Lin⁻ (CD45R (B220), CD5, CD11b, Gr-1(Ly6-G/C), Ly6-G, 7-4 and Ter-119 negative) cells were plotted for sca-1 and CD117 expression; CD117⁺, sca-1⁻precursors were then subdivided based on their expression of CD117 and CD115;
Fig. 7b is a diagram showing the expression of GFP (Cx₃CR1) in sorted HSCs, MPs, MPCs and CDPs from the bone marrow of Cx₃CR1^{GFP/+} mice;

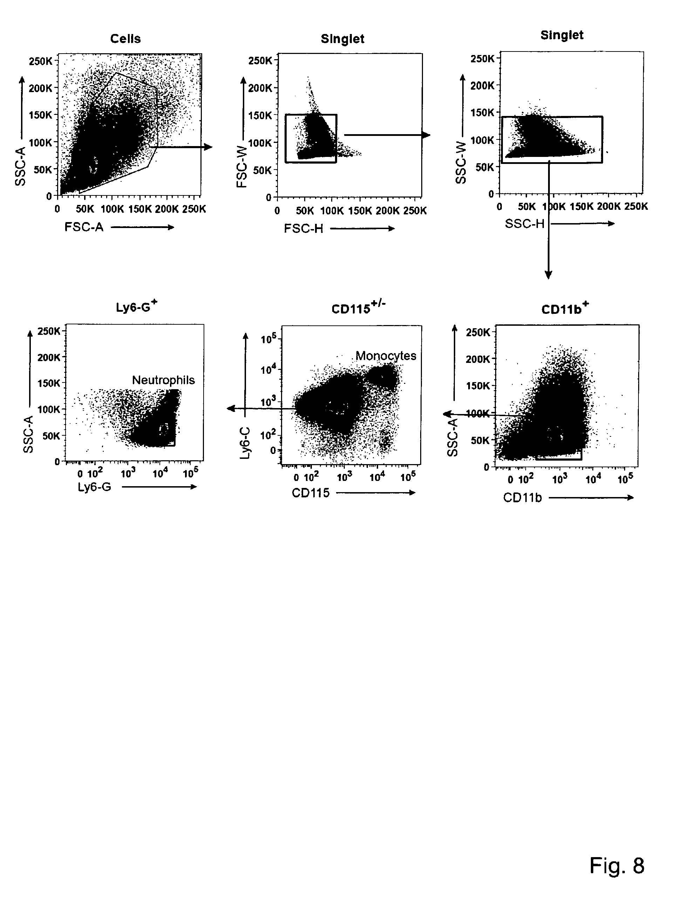

Fig. 8 is a panel of six diagrams depicting the gating strategy for the identification of inflammatory monocytes (CD11b⁺Ly6-C^{hi}CD115⁺) and neutrophils (CD11b⁺Ly6-C^{lo}CD115⁻Ly6-G⁺) by flow cytometry of murine bone marrow; single Lin-(CD4, CD8α, CD 19, Ter-119 negative) cells were plotted for CD11b expression; CD11b⁺ cells were then subdivided based on their expression of Ly6-C and CD115; Ly6-G⁺ cells of the Ly6C^{lo}CD115⁻ population were then sorted;

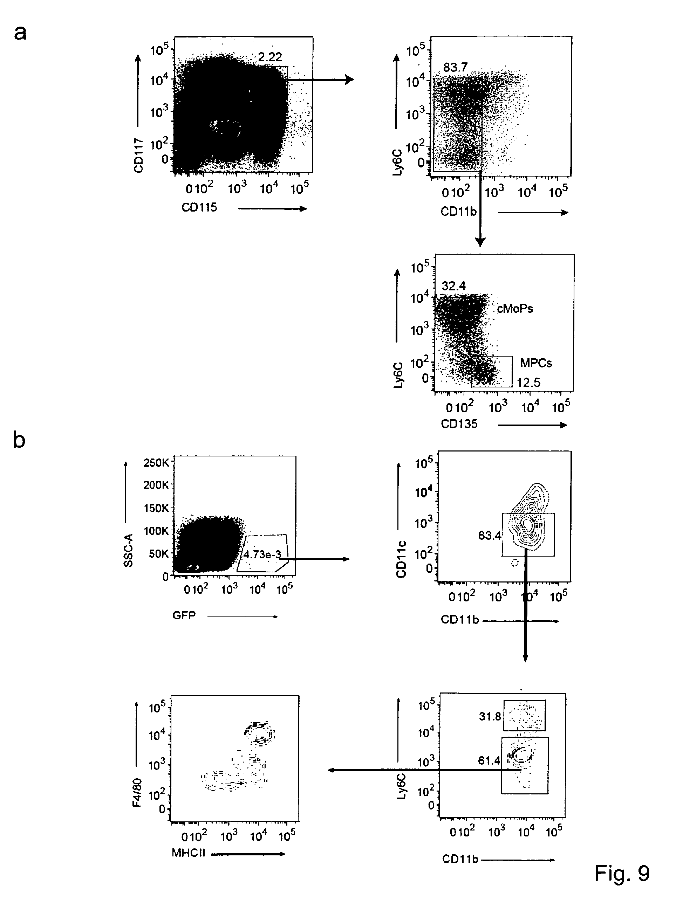

Fig. 9a is a panel of three diagrams depicting the gating strategy for the identification of common monocyte progenitors (cMoPs; CD117^{hi}CD115⁺CD135⁻CD11b⁻Ly6-C⁺) and myeloid progenitor cells (MPCs; CD117^{hi}CD115⁺CD135⁺CD11b⁻Ly6-C⁻) by flow cytometry of murine bone marrow; cells were plotted for CD 117 and CD 115 expression; CD117^{hi}CD115⁺CD11b⁻ cells were then subdivided based on their expression of Ly6-C and CD135;
Fig. 9b is a panel of four diagrams indicating the differentiation of adoptively transferred cMoPs into monocytes, macrophages and CD11^{b}+CD11c⁺ cells in the spleen at 7 days after transplantation; donor-derived monocytes, macrophages, and CD11b⁺CD11c⁺ cells were identified by flow cytometry in the spleen, whereas no CD11c⁺CD11b⁻ DCs were detected;

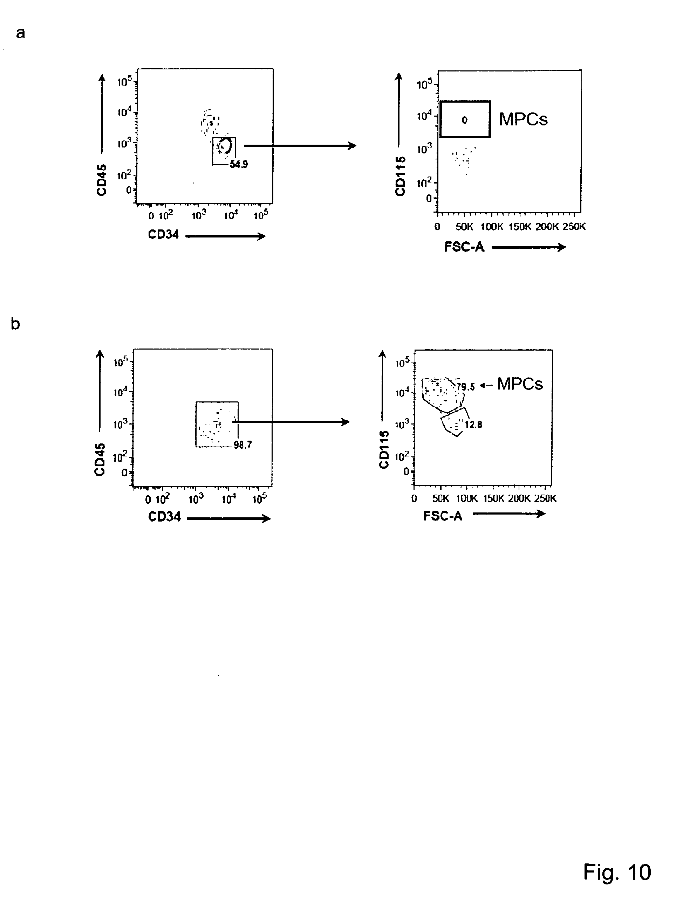

Fig. 10 is a panel of four diagrams showing the characterization of human MPCs by flow cytometry; a) CD34⁺ hematopoietic cells were isolated by magnetic-activated cell sorting from mobilized peripheral blood (frozen apheresis sample); no MPCs (SSC^{lo}FSC^{lo}CD45^{lo}CD34⁺CD115⁺) were detected in the sorted cell population before culturing; b) In the presence of growth factors (IL-3, IL-6, SCF, Flt3ligand and GM-CSF) and serum-free medium, the majority of living cells *in vitro* were identified as SSC^{lo}FSC^{lo}CD45^{lo}CD34⁺ (98.7 % of the entire cell population); 79.5 % of the SSC^{lo}FSC^{lo}CD45^{lo}CD34⁺ cell population expressed the MPC marker, CD115 (CSF-1R), after differentiation in culture; and

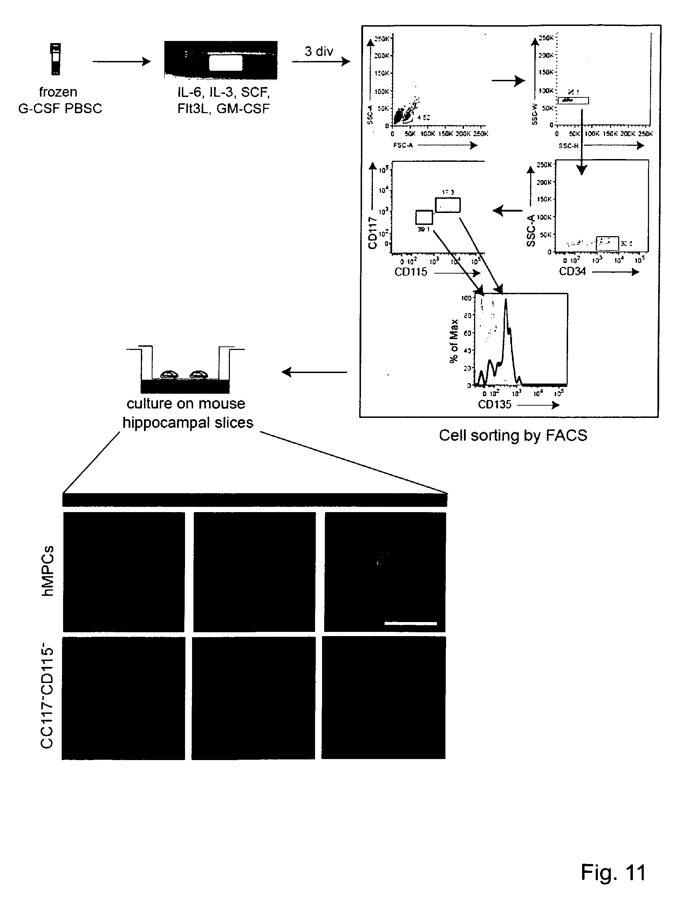

Fig. 11 is a composite panel of five diagrams illustrating the results of the *ex vivo* differentiation of human MPCs; frozen mobilized human peripheral blood stem cells obtained by apheresis (G-CSF PBSC) were cultured in the presence of growth factors (IL-3, IL-6, SCF, Flt3ligand and GM-CSF) in serum-free medium for 3 days (div); the third diagram consists of five small diagrams depicting the gating strategy for the identification of myeloid precursor cells (MPCs) using flow cytometry; single SSC^{lo}CD34⁺ was plotted for CD117 and CD115 expression; MPCs were sorted as SSC^{lo}FSC^{lo}CD34⁺c-kit⁺CD115⁺CD135⁺ cells (red rectangle); the sorted SSC^{lo}FSC^{lo}CD34⁺c-kit⁻CD115⁻CD135⁻ cells served as a negative control population (black rectangle); the bottom small diagram shows the histogram plot of the expression of CD135 in sorted MPCs (red) and CD34⁺CD115⁻ cells (grey); the sorted cells (2x10³ cells) were then cultured on the murine hippocampal slices for 3 days; the bottom panel consisting of six laser confocal microscopic images (scale bar: 10 µm) depicts the differentiation human MPCs into Iba-1-immunoreactive macrophages on hippocampal slices (top row). Iba-1 immunoreactivity is shown in red and human nuclei (HuNu) are shown in green; the control population of CD34⁺CD115⁻ cells did not give rise to human tissue macrophages (bottom row).

### Example 1: Materials and methods

If not indicated differently in the examples, the following materials and methods were used.

**Mice.** C57BL/6 mice were purchased from Charles River (Sulzbach, Germany). C57BL/6 mice expressing enhanced green fluorescent protein (EGFP) under the control of β-action and Cx₃CR1 promoters were obtained from Charité Breeding Facility (Berlin, Germany). All donor and recipient mice were 7-12 weeks old at the time of bone marrow transplantation. All animal protocols were reviewed and approved by the state authorities (LaGeSo), Berlin, Germany.

**Focal head irradiation and adoptive transfer experiments.** Mice were anaesthetized by subcutaneous injection of a mixture of ketamine (75 mg/kg) and xylazine (15 mg/kg). Focal head irradiation (11Gy) was carried out using a Gammacell 40 Exactor (Theratronics). The body was protected from irradiation using a lead bar (3 cm thick). The total dose of γ-irradiation to the shielded areas was less than 0.6 Gy. To avoid acute irradiation-induced BBB damage, sorted cell populations were adoptively transferred 14 days after irradiation and one day after facial nerve axotomy (Fig. 1). Unilateral facial nerve axotomy was induced by transaction of the facial nerve at the stylomastoid foramen, resulting in ipsilateral whisker paresis. Subsequently, rapamycin (3 mg/kg) was administered for suppressing the graft rejection.

**Cell sorting.** Bone marrow-derived HSCs, MPCs, monocytes and granulocytes were sorted according to a published protocol (Kollet, O. et al., J. Clin. Invest. 112, 160-1698, 2003) with some modifications (see Fig. 7). MPCs from β-actin-EGFP transgenic mice were shown to have the same homing and differentiation characters as the ones isolated from the more widely used Cx₃CR1^{GFP/+} donors (Hanna, R. N. et al., Nat. Immunol. 12, 778-785, 2011). Bone marrow from 0-action (ATCβ)-EGFP transgenic mice was first enriched for monocytes and progenitors by negative selection with isolation kits according to the manufacturer's protocol (CD4, CD8α, CD19 & Ter119 custom-made cocktail for enrichment of progenitors and CD11b+ populations; Miltenyi Biotec). Lineage-negative (Lin⁻) cells were isolated utilizing AutoMACS (Miltenyi Biotec) and subsequently stained for sca-1, CD117, CD11b, CD115, Ly-6C, and Ly-6G. Cell sorting was performed on FACSAria II (BD Biosciences). HSCs were identified as lin⁻CD11b⁻CD117⁺Sca-1⁺; MPCs were identified as lin⁻CD11b⁻CD115⁺CD117⁺; inflammatory monocytes were identified as Lin⁻CD11b⁺CD115⁺Ly-6C^{hi}; granulocytes were identified as Lin⁻CD11b⁺CD115⁻Ly6C^{int}Ly-6G⁺ (see Fig. 8).

**Flow cytometry.** Spleens were excised and pushed through a 70-µm strainer, bone marrow cells were collected from both femurs and tibias, and blood was collected from Vena cava caudalis using a citrate-coated syringe. Red blood cells were lysed in Pharm LyseTM lysing buffer according to the manufacturer's protocol (BD Biosciences).

The following antibodies were used: sca-1 (D7); CD117 (2B8); CD115 (AFS98); Ly-6C (AL-21 and HK1.4); CD4 (RM4-5); CD8 (53-6.7); Ly6G (1A8); CD11c (N418) (Biolegend) and CD11b (M1/70) & B220 (RA3-6B2) (BD Pharmingen).

Forward- and side-scatter parameters were used for exclusion of doublets from analysis. Cellular fluorescence was assessed with MACSQuant (Miltenyi Biotec) and data were analyzed with FlowJo software (TreeStar).

**Immunohistochemistry.** Three, seven or fourteen days after an adoptive transfer, mice were anaesthetized. Peripheral blood was collected for flow cytometry. Subsequently, the animals were transcardially perfused with cold PBS. Spleen and bone marrow were then collected for flow cytometry. Brains were dissected, fixed in 4% paraformaldehyde (PFA) and cryoprotected with 30% sucrose. Coronal sections (30 µm) of fixed tissues were obtained on a cryostat.

Sections were blocked at room temperature with 20% normal goat/donkey serum in TBS containing 0.3% Triton X-100 for 1 hr and washed three times in TBS. Incubation at 4 °C with anti-Iba-1 [1:200] (Wako), anti-F4/80 [1:200] (AbD Serotec), anti-GFP [1:200] (Invitrogen) or anti-human nuclei [1:200] (Millipore) was performed overnight. After washing, sections were incubated with secondary antibodies (Alexa 488-/594-IgG 1:250; Invitrogen) at room temperature for 3 hrs. All antibodies were diluted in TBS containing 1% normal goat/donkey serum and 0.3% Triton X-100. Nuclei were counterstained with 4,6-diamidino-2-phenylindole (1:10000; Sigma). The stained sections were examined using a fluorescence or laser confocal scanning microscope (Leica TCS SPE, Leica Microsystems).

### Example 2: BM-derived MPCs give rise to brain macrophages

To identify the BM-derived cell population that is a precursor of brain macrophages, GFP⁺ HSCs (Lin⁻c-kit⁺sca-1⁺CD115⁻; 10⁵ cells), MPCs (Lin⁻c-kit^{hi}sca-1⁻CD115⁺; 10⁵ cells), or inflammatory monocytes (CD4⁻CD8a⁻CD19⁻CD11b⁺Ly6-C^{hi}CD115⁺; 10⁶ cells) (Figs. 7 and 8) were adoptively transferred into the recipient mice that were previously conditioned by focal head irradiation (11Gy) and facial nerve axotomy as described previously (Böttcher, C. et al., PLoS One 8, e80260, 2013) (see Fig. 1a).

Engrafhment of donor-derived cells at sites of damage, as well as in peripheral blood, spleen and bone marrow was analyzed at 1, 3, 7 and 14 days after adoptive transfer. As expected, only HSCs could repopulate the peripheral blood, spleen and bone marrow of recipients (see Fig. 1b). On day 1 & 3 after transplantation, the vast majority of GFP⁺ cells remained HSCs (see Fig. 1c). Of note, HSC-derived MPCs were detected in the bone marrow and spleen but not in the bloodstream of HSC-transplanted animals, suggesting that MPCs were not mobilized spontaneously from their bone marrow/spleen niche (see Fig. 1d). Fourteen days after transplantation, donor-derived monocytes, granulocytes, CD11b⁺CD11c⁺ macrophages and CD11b⁻CD11c⁺ DCs were detected in the spleen and bone marrow of mice that received GFP⁺ HSCs (see Fig. 1e). Despite the highest presence of donor-derived cells in the circulation of recipient mice at any time point analyzed, adoptive transfer of BM-derived HSCs did not give rise to brain macrophages under our conditions (see Fig. 2b).

In MPC-transplanted recipients, > 95% of donor-derived cells were found to express CD11b at 24 hrs after adoptive transfer. As expected, they were identified as monocytes, dendritic cells and macrophages (see Fig. 1f) but not neutrophils. As early as 3 days after adoptive transfer, MPCs were able to give rise to brain macrophages that selectively engrafted in the lesioned facial nucleus, although < 0.5% of cells present in the host circulation expressed GFP (see Figs. 1b and 2a). The cells engrafting in the CNS were immunoreactive for the macrophage markers, Iba-1 and F4/80, which ruled out a dendritic cell phenotype (see Figs. 2a and 9a). The number of GFP⁺ cells at sites of brain damage increased with the progression of pathology (see Fig. 2c).

Up to 14 days after transplantation, none of Ly6C^{hi} inflammatory monocytes gave rise to macrophages at sites of brain damage (see Fig. 2b). Notably, adoptively transferred monocytes were barely detected in the host circulation at any time point studied (see Fig. 1b). To rule out that Ly6C^{hi} monocytes missed a window of opportunity for CNS engraftment due to their short lifespan, Ly6C^{hi} inflammatory monocytes were infused repeatedly over five consecutive days (10⁶ cells per day) and CNS tissue was analyzed at 7 days after the first injection (d7). Although the number of GFP⁺ cells in the bloodstream was significantly increased to a level comparable to that of MPC-transplanted mice (see Fig. 2d), no monocyte-derived cells were found in the axotomized facial nucleus (see Fig. 2d). Thus, BM-derived Ly6-C^{hi} inflammatory monocytes are ineffective at engrafting in the lesioned CNS.

Next, the engraftment capacities of HSCs, MPCs and Ly6-C^{hi} monocytes were assessed ex *vivo* using organotypic hippocampal slices. Again, MPCs showed a greater ability to engraft in the hippocampal slices compared to HSCs and inflammatory monocytes (see Fig. 2e).

Up to 14 days after adoptive transfer, CDPs (Lin⁻c-kit^{lo}sca-1⁻CD115⁺; 10⁵ cells), pre-cDCs (CD4⁻CD8a⁻CD19⁻c-kit^{lo}CD11b⁺CD11c⁺CD115⁺Cx₃CR1⁺CD135⁺; 10⁵ cells), myeloid precursors (MP; Lin⁻c-kit^{hi}sca-1⁻CD115⁻; 10⁵ cells) or granulocytes (CD4⁻CD8a⁻CD19⁻CD11b⁺Ly6-C^{lo}Ly6-G⁺CD115⁻; 10⁶ cells) did not give rise to GFP⁺ donor-derived cells in the damaged CNS under these conditions (see Figs. 2b and 2c).

Collectively, these results demonstrate that MPCs rather than other BM-derived cell populations are the precursors of brain macrophages.

### Example 3: CNS recruitment of MPC-derived cells depends on CCR2 expression

Expression of the chemokine receptor CCR2 by bone marrow-derived cells is necessary for their engraftment in the CNS after myeloablative total body irradiation and bone marrow transplantation (Mildner, A. et al., Nat. Neurosci. 10, 1544-1553, 2007). To test whether MPCs exploit a CCR2-dependent mechanism for entry into the CNS, GFP⁺ MPCs obtained from CCR2^{-/-} mice were adoptively transferred into recipients that were conditioned by focal head irradiation and facial nerve axotomy. Up to 14d after adoptive transfer, no GFP⁺ cells were found in the lesioned facial nucleus (see Fig. 3b). Homing to the spleen was reduced, but not abolished for CCR2-deficient MPCs (see Fig. 3a).

In spleen, MPCs underwent differentiation into macrophages and DCs as early as one day after transfer (see Figs. 1e and 1f), suggesting that MPCs may need to differentiate in the spleen to CCR2⁺ macrophages before entering the CNS. However, splenectomy resulted in higher numbers of MPC-derived cells in the lesioned facial nucleus, thereby dismissing the concept of peripheral priming in the spleen (see Figs. 3d and 3e).

### Example 4: MPCs reduce amyloid load in a mouse model of Alzheimer's disease (AD)

In the next set of experiments, we wanted to assess whether the engraftment of MPC-derived cells at sites of brain damage would result in improved disease outcome in mouse models of chronic neurodegeneration such as Alzheimer's disease (AD; *APP*^{*swe*/}*^{PS1}, B6C3-Tg(APP695)3Dbo Tg(PSEN1)5Dbo*/*J*)*.* 5x10⁵ GFP⁺ MPCs were adoptively transferred into 9 month-old APP^{swe/PS1} mice two weeks after focal head irradiation (see Fig. 4a). Four weeks after transplantation, GFP⁺ cells were observed at perivascular sites (see Fig. 4b). Compared to non-transplanted APP^{swe/PS1} animals, a significant reduction of both soluble and insoluble Aβ₁₋₄₀ and Aβ₁₋₄₂ was observed in the brains of MPC-treated APP^{swe/PS1} mice (see Fig. 4c). Thus, the data suggest that MPC-derived perivascular macrophages are involved in the clearance of Aβ from the CNS.

### Example 5: Transplantation of MPCs after disease onset attenuates disease progression in a mouse model of amyotrophic lateral sclerosis (ALS)

Overexpression of the mutant SOD1 (G93A) gene in mice leads to progressive neurodegeneration and neuroinflammation (Gurney, M. E. et al., Science 264, 1772-1775, 1994; Boillée, S. et al. Neuron 52, 39-59, 2006), as well as degeneration of mesodermal tissues such as skeletal muscle and heart (Corti, S. et al. Brain 127, 2518-2532, 2004), thereby mimicking a familial form of amyotrophic lateral sclerosis (ALS). Transplantation of bone marrow cells expressing the wild type SOD1 gene into SOD1^{G93A} transgenic mice *(B6.Cg-Tg(SOD1-G93A)^{dl}1Gur*/*J*) after total body irradiation resulted in a delay of disease onset and increase of life span (Corti, S. et al. Brain 127, 2518-2532, 2004). These wild type BM cells also participated in muscle regeneration (Corti, S. et al. Brain 127, 2518-2532, 2004).

Since the pathological hallmarks of ALS are degeneration/inflammation of both the CNS (motor neurons in the cortex and spinal cord) and the periphery (sciatic nerve and muscle), we assessed potential therapeutic effects of wild type MPCs in SOD1^{G93A} transgenic mice. After disease onset (90-100 days of age), 5x10⁵ MPCs from *ATCβ-EGFP* transgenic mice were adoptively transferred into non-irradiated SOD1^{G93A} transgenic mice. Four weeks after transplantation, a significant reduction of inflammation (indicated by the activation of microglia/macrophages) was observed in the spinal cord and sciatic nerve (see Fig. 5a).

Up to 10 weeks after a single intravenous injection of GFP⁺ MPCs, recruitment of MPC-derived macrophages was stably detected in the sciatic nerve and hindlimb muscles of the recipients (see Figs. 5e and 5f). Although GFP⁺ cells were never detected in the brain or spinal cord, MPC administration resulted in improved motor function (see Figs. 5b and 5d), as well as prolongation of disease onset (∼ 29%) and progression (∼ 60%) compared to the non-transplanted SOD1^{G93A} transgenic mice (see Fig. 5c). Recruitment of MPC-derived macrophages to the sciatic nerve was associated with reduced inflammation and macrophage accumulation (see Fig. 5g), a hallmark of ALS (Dibaj, P. et al. PLoS ONE 6, e17910, 2011; Graber, D. J. et al. J Neuroinflammation 7, 8, 2010; Chiu, I. M. et al. Proc. Natl. Acad. Sci. U.S.A. 106, 20960-20965, 2009). Slight attenuation of microglial activation in the spinal cord was also observed in this long-term study but did not reach statistical significance (see Fig. 5g). However, MPC administration resulted in dramatically reduced expression of proinflammatory mediators like CCL2, TNF-α, TGF-β, IL-6 and IL-4 mRNAs in the spinal cords of SOD1^{G93A} transgenic mice (see Fig. 5h).

In the past, transplantation of SOD1 mutant bone marrow cells into total body irradiated SOD1^{G93A} transgenic mice did not result in an improvement of disease outcome whereas this was the case with wild type bone marrow cells (Corti, S. et al. Brain 127, 2518-2532, 2004). We confirmed these findings using MPCs; adoptive transfer of 5x10⁵ purified SOD1 mutant MPCs into SOD1^{G93A} transgenic mice failed to confer protection, while disease progression was attenuated in SOD1^{G93A} transgenic mice after adoptive transfer of wild type MPCs (see Figs. 6a-c).

### Example 6: Characterization of human bone marrow-derived myeloid progenitor cells (hMPCs) from mobilized peripheral blood

CD34-positive hematopoietic stem cells were isolated from mobilized peripheral blood (a frozen apheresis product) by means of magnetic-activated cell sorting (MACS). After sorting, the cells were characterized by flow cytometry. As indicated in Fig. 10a, no MPCs (SSC^{lo}FSC^{lo}CD45^{lo}CD34⁺CD115⁺) were detected in the sorted cell population.

Isolated stem cells (SSC^{lo}FSC^{lo}CD45^{lo}CD34⁺CD115⁻) were subsequently cultured in the presence of growth factors (IL-3, IL-6, SCF, Flt3ligand and GM-CSF) in serum-free medium. After three days of culture, the cells were harvested and analyzed by flow cytometry. The majority of living cells were identified as SSC^{lo}FSC^{lo}CD45^{lo}CD34⁺ (98.7 % of the entire cell population). 79.5 % of the SSC^{lo}FSC^{lo}CD45^{lo}CD34⁺ cell population expressed MPC markers, such as CD115 (CSF-1R), after differentiation in culture (see Fig. 10b).

### Example 7: Differentiation of sorted human bone marrow-derived myeloid progenitor cells (hMPCs)

Frozen mobilized human peripheral blood stem cells obtained by apheresis (G-CSF PBSC) were cultured in the presence of growth factors (IL-3, IL-6, SCF, Flt3ligand and GM-CSF) in serum-free medium for 3 days (see Fig. 11). Cells were then sorted using flow cytometry. hMPCs were isolated as SSC^{lo}FSC^{lo}D34⁺c-kit⁺CD115⁺CD135⁺ cells. SSC^{lo}FSC^{lo}CD34⁺c-kit⁻CD115⁻CD135⁻ cells served as a negative control population. The sorted cells (2x10³ cells) were then cultured on the murine hippocampal slices for 3 days. We observed that hMPCs expressing the human nuclear antigen differentiated into Iba-1-immunoreactive macrophages on hippocampal slices. This was not observed for CD34⁺ CD115⁻ control cells.

The features of the present invention disclosed in the specification, the claims, the sequence listing and/or the drawings may both separately and in any combination thereof be material for realizing the invention in various forms thereof.

## Claims

1. A bone marrow-derived myeloid progenitor cell (MPC), preferably an isolated bone marrow-derived myeloid progenitor cell (MPC), wherein the bone marrow-derived myeloid progenitor cell (MPC) is expressing CD34 and c-Kit (CD117).

2. The bone marrow-derived myeloid progenitor cell (MPC) of claim 1, wherein the bone marrow-derived myeloid progenitor cell (MPC) is expressing CD115 and/or CD135 and/or CX3CR1.

3. The bone marrow-derived myeloid probenitor cell (MPC) of any one of claims 1 to 2, wherein the bone marrow-derived myeloid progenitor cell (MPC) is SSC^{lo} and/or FSC^{lo}.

4. The bone marrow-derived myeloid progenitor cell (MPC) of any one of claims 1 to 3, wherein the bone marrow-derived myeloid progenitor cell (MPC) is IL-17Ra-negative.

5. The bone marrow-derived myeloid progenitor cell (MPC) of any one of claims 1 to 4, wherein the bone marrow-derived myeloid progenitor cell (MPC) is negative for at least one B-cell marker, preferably at least one membrane-bound B-cell marker, at least on T cell marker, at least one mature myeloid cell marker, at least one granulocyte / monocyte marker, at least one granulcyte marker and/or at least one erythrocyte marker.

6. The bone marrow-derived myeloid progenitor cell (MPC) of any one of claims 1 to 5, wherein the bone marrow-derived myeloid progenitor cell (MPC) is a recombinant bone marrow-derived myeloid progenitor cell (MPC), preferably the recombinant bone marrow-derived myeloid progenitor cell (MPC) comprises a heterologous nucleic acid.

7. The bone marrow-derived myeloid progenitor cell (MPC) of any one of claims 1 to 6, wherein the bone marrow-derived myeloid progenitor cell (MPC) is labelled with a therapeutically active isotope, preferably a therapeutically active radioisotope.

8. The bone marrow-derived myeloid progenitor cell (MPC) of any one of claims 1 to 6, wherein the bone marrow-derived myeloid progenitor cell (MPC) is labelled with a diagnostically active label, preferably the label is an isotope, more preferably a diagnostically active radioisotope.

9. The bone marrow-derived myeloid progenitor cell (MPC) of any one of claims 1 to 8, wherein the bone marrow-derived myeloid progenitor cell (MPC) is a human bone marrow-derived myeloid progenitor cell (hMPC).

10. The bone marrow-derived myeloid progenitor cell (MPC) of any one of claims 1 to 9, for use in a method for treating a subject suffering from a disease or being at risk of suffering from a disease.

11. The bone marrow-derived myeloid progenitor cell (MPC) of claim 10, wherein the method is adoptive transfer of the myeloid progenitor cell (MPC) derived from bone marrow or mobilized human peripheral blood stem cells, preferably adoptive transfer in the treatment of the disease.

12. The bone marrow-derived myeloid progenitor cell (MPC) of claim 10, wherein the disease is a neurodegenerative disease.

13. The bone marrow-derived myeloid progenitor cell (MPC) of any one of claims 1 to 9, for use in a method for treating a subject suffering from a disease or being at risk of suffering from a disease, wherein the bone marrow-derived myeloid progenitor cell (MPC) is a recombinant bone marrow-derived myeloid progenitor cell (MPC) delivering a therapeutically active agent into the subject.

14. The bone marrow-derived myeloid progenitor cell (MPC) of any one of claim 1 to 9, for use in a method for diagnosing a disease in a subject.

15. The bone marrow-derived myeloid progenitor cell (MPC) of claim 14, wherein the disease is a disease of the CNS, or an inflammatory disease.

16. A pharmaceutical composition comprising a bone marrow-derived myeloid progenitor cell (MPC) of any one of claims 1 to 9 and a pharmaceutically acceptable excipient.

17. A diagnostic composition comprising a bone marrow-derived myeloid progenitor cell (MPC) of any one of claims 1 to 9 and a diagnostically acceptable excipient.

18. A kit comprising a bone marrow-derived myeloid progenitor cell (MPC) according to any one of claims 1 to 9 and at least one constituent selected from the group comprising a container, a buffer, a diluent, an excipient and an instruction leaflet.
